# EUROPEAN PATENT SPECIFICATION

(11) **EP 3 351 525 B1**
(45) Date of publication and mention of the grant of the patent: **08.04.2020**
(21) Application number: 16846050.9
(22) Date of filing: 14.06.2016
(51) Int. Cl.: C07C 69/732, C09K 19/12, C09K 19/30, C09K 19/32, C09K 19/34, C09K 19/38, C09K 19/54, G02F 1/13, G02F 1/1337

(54) **POLYMERIZABLE POLAR COMPOUND, LIQUID CRYSTAL COMPOSITION, AND LIQUID CRYSTAL DISPLAY ELEMENT**
POLYMERISIERBARE POLARE VERBINDUNG, FLÜSSIGKRISTALLZUSAMMENSETZUNG UND FLÜSSIGKRISTALLANZEIGEELEMENT
COMPOSÉ POLAIRE POLYMÉRISABLE, COMPOSITION DE CRISTAUX LIQUIDES, ET ÉLÉMENT D'AFFICHAGE À CRISTAUX LIQUIDES

(30) Priority: 15.09.2015 JP 2015181370
(43) Date of publication of application: 25.07.2018
(73) Proprietor: JNC Corporation, Chiyoda-ku Tokyo 100-8105 (JP); JNC Petrochemical Corporation, Tokyo 100-0004 (JP)
(72) Inventor: YANO, Masakazu, Ichihara-shi Chiba 290-8551 (JP); TANAKA, Hiroyuki, Ichihara-shi Chiba 290-8551 (JP); OGITA, Kazuhiro, Ichihara-shi Chiba 290-8551 (JP); KONDOU, Fumitaka, Ichihara-shi Chiba 290-8551 (JP); YANO, Tomohiro, Ichihara-shi Chiba 290-8551 (JP)
(74) Representative: TBK
(86) International application number: PCT/JP2016/067664
(87) International publication number: WO 2017/047177

(56) References cited:
- CN-A- 104 710 990
- CN-A- 104 710 990
- JP-A- 2001 092 127
- JP-A- 2001 092 127
- JP-A- 2009 098 619
- JP-A- 2015 078 282
- KR-A- 20130 132 032
- KR-A- 20130 132 032
- US-A1- 2009 086 140
- US-A1- 2015 252 265
- US-B1- 6 184 339
- US-B1- 6 184 339

## Description

### Technical Field

The invention relates to a compound having a polymerizable group, a liquid crystal composition and a liquid crystal display device. More specifically, the invention relates to a compound having both a plurality of polymerizable groups such as methacryloyloxy and polar groups such as -OH groups, a liquid crystal composition containing the compound and having positive or negative dielectric anisotropy, and a liquid crystal display device including the composition.

### Background Art

In a liquid crystal display device, a classification based on an operating mode for liquid crystal molecules includes a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode and a field-induced photo-reactive alignment (FPA) mode. A classification based on a driving mode in the device includes a passive matrix (PM) and an active matrix (AM). The PM is classified into static, multiplex and so forth, and the AM is classified into a thin film transistor (TFT), a metal insulator metal (MIM) and so forth. The TFT is further classified into amorphous silicon and polycrystal silicon. The latter is classified into a high temperature type and a low temperature type based on a production process. A classification based on a light source includes a reflective type utilizing natural light, a transmissive type utilizing backlight and a transflective type utilizing both the natural light and the backlight.

The liquid crystal display device includes a liquid crystal composition having a nematic phase. The composition has suitable characteristics. An AM device having good characteristics can be obtained by improving characteristics of the composition. Table 1 below summarizes a relationship in two characteristics. The characteristics of the composition will be further described based on a commercially available AM device. A temperature range of the nematic phase relates to a temperature range in which the device can be used. A preferred maximum temperature of the nematic phase is about 70°C or higher, and a preferred minimum temperature of the nematic phase is about -10°C or lower. Viscosity of the composition relates to a response time in the device. A short response time is preferred for displaying moving images on the device. A shorter response time even by one millisecond is desirable. Accordingly, small viscosity in the composition is preferred. Small viscosity at low temperature is further preferred.

**Table 1. Characteristics of composition and AM device**

| No. | Characteristics of composition | Characteristics of AM device |
|---|---|---|
| 1 | Wide temperature range of a nematic phase | Wide usable temperature range |
| 2 | Small viscosity¹⁾ | Short response time |
| 3 | Suitable optical anisotropy | Large contrast ratio |
| 4 | Large positive or negative dielectric an isotropy | Low threshold voltage and small electric power consumption Large contrast ratio |
| 5 | Large specific resistance | Large voltage holding ratio and large contrast ratio |
| 6 | High stability to ultraviolet light and heat | Long service life |
| 7 | Large elastic constant | Large contrast ratio and short response time |

| | | |
|---|---|---|
| **1) A composition can be injected into a liquid crystal display device in a short time.** | | |

ratio in the device. According to a mode of the device, large optical anisotropy or small optical anisotropy, more specifically, suitable optical anisotropy is required. A product (Δn × d) of the optical anisotropy (Δn) of the composition and a cell gap (d) in the device is designed so as to maximize the contrast ratio. A suitable value of the product depends on a type of the operating mode. In a device having a mode such as a TN mode, the value is about 0.45 micrometer. In a device having the VA mode, the value is in the range of about 0.30 micrometer to about 0.40 micrometer, and in a device having the IPS mode or the FFS mode, the value is in the range of about 0.20 micrometer to about 0.30 micrometer. In the above case, a composition having large optical anisotropy is preferred for a device having a small cell gap. Large dielectric anisotropy in the composition contributes to low threshold voltage, small electric power consumption and a large contrast ratio in the device. Accordingly, large positive or negative dielectric anisotropy is preferred. Large specific resistance in the composition contributes to a large voltage holding ratio and the large contrast ratio in the device. Accordingly, a composition having large specific resistance at room temperature and also at a temperature close to the maximum temperature of the nematic phase in an initial stage is preferred. The composition having large specific resistance at room temperature and also at a temperature close to the maximum temperature of the nematic phase even after the device has been used for a long period of time is preferred. Stability of the composition to ultraviolet light and heat relates to a service life of the device. In the case where the stability is high, the device has a long service life. Such characteristics are preferred for an AM device use in a liquid crystal projector, a liquid crystal television and so forth.

In a polymer sustained alignment (PSA) mode liquid crystal display device, a liquid crystal composition containing a polymer is used. First, a composition to which a small amount of a polymerizable compound is added is injected into the device. Next, the composition is irradiated with ultraviolet light while voltage is applied between substrates of the device. The polymerizable compound is polymerized to form a network structure of the polymer in the composition. In the composition, alignment of liquid crystal molecules can be controlled by the polymer, and therefore the response time in the device is shortened and also image persistence is improved. Such an effect of the polymer can be expected for a device having the mode such as the TN mode, the ECB mode, the OCB mode, the IPS mode, the VA mode, the FFS mode and the FPA mode.

In a general-purpose liquid crystal display device, vertical alignment of liquid crystal molecules is achieved by a polyimide alignment film. On the other hand, as a liquid crystal display device having no alignment film, a mode in which a polar compound is added to a liquid crystal composition to align liquid crystal molecules has been proposed. First, a composition to which a small amount of the polar compound and a small amount of a polymerizable compound are added is injected into the device. Here, the liquid crystal molecules are aligned by action of the polar compound. Then, the composition is irradiated with ultraviolet light while voltage is applied between substrates of the device. Here, the polymerizable compound is polymerized to stabilize the alignment of liquid crystal molecules. In the composition, the alignment of liquid crystal molecules can be controlled by the polar compound and the polymer, and therefore the response time in the device is shortened and also image persistence is improved. Further, in a device having no alignment film, a process of forming the alignment film is unnecessary. The device has no alignment film, and therefore reduction in electric resistance of the device by interaction between the alignment film and the composition is not caused. Such an effect caused by a combination of the polar compound and the polymer can be expected for a device having the mode such as the TN mode, the ECB mode, the OCB mode, the IPS mode, the VA mode, the FFS mode and the FPA mode.

In the liquid crystal display device having no alignment film, various compounds each having a -OH group at a terminal have been so far prepared as a compound in which liquid crystal molecules can be vertically aligned. Patent literature No. 1 describes biphenyl compound (S-1) having a -OH group at a terminal.

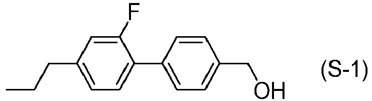

### Citation List

### Patent Literature

Patent literature No. 1: WO 2014/090362 A.
Patent literature No. 2: WO 2014/094959 A.
Patent literature No. 3: WO 2013/004372 A.
Patent literature No. 4: WO 2012/104008 A.
Patent literature No. 5: WO 2012/038026 A.
Patent literature No. 6: JP S50-35076 A.
Patent literature No. 7: US 2015/252265 A1.

### Summary of Invention

### Technical Problem

A first object of the invention is to provide a polar compound having high chemical stability, high capability of aligning liquid crystal molecules, high solubility in a liquid crystal composition, and a large voltage holding ratio when used in a liquid crystal display device. A second object is to provide a liquid crystal composition that contains the compound, and satisfies at least one of characteristics such as high maximum temperature of a nematic phase, low minimum temperature of the nematic phase, small viscosity, suitable optical anisotropy, large positive or negative dielectric anisotropy, large specific resistance, high stability to ultraviolet light, high stability to heat and a large elastic constant. A third object is to provide a liquid crystal display device that includes the composition, and has characteristics such as a wide temperature range in which the device can be used, a short response time, a high voltage holding ratio, low threshold voltage, a large contrast ratio, a long service life and good vertical alignment properties.

### Solution to Problem

The invention concerns a compound as defined in claim 1, a liquid crystal composition containing the compound, and a liquid crystal display device including the composition.

### Advantageous Effects of Invention

A first advantage of the invention is to provide a polar compound having high chemical stability, high capability of aligning liquid crystal molecules, high solubility in a liquid crystal composition, and a large voltage holding ratio when used in a liquid crystal display device. A second advantage is to provide a liquid crystal composition that contains the compound, and satisfies at least one of characteristics such as high maximum temperature of a nematic phase, low minimum temperature of the nematic phase, small viscosity, suitable optical anisotropy, large positive or negative dielectric anisotropy, large specific resistance, high stability to ultraviolet light, high stability to heat and a large elastic constant. A third advantage is to provide a liquid crystal display device that includes the composition, and has characteristics such as a wide temperature range in which the device can be used, a short response time, a large voltage holding ratio, low threshold voltage, a large contrast ratio, a long service life and good vertical alignment properties.

### Description of Embodiments

Usage of terms herein is as described below. Terms "liquid crystal composition" and "liquid crystal display device" may be occasionally abbreviated as "composition" and "device," respectively. "Liquid crystal display device" is a generic term for a liquid crystal display panel and a liquid crystal display module. "Liquid crystal compound" is a generic term for a compound having a liquid crystal phase such as a nematic phase and a smectic phase, and a compound having no liquid crystal phase but to be mixed with the composition for the purpose of adjusting characteristics such as a temperature range of the nematic phase, viscosity and dielectric anisotropy. The compound has a six-membered ring such as 1, 4-cyclohexylene or 1, 4-phenylene, and has rod-like molecular structure. "Polymerizable compound" is a compound to be added for the purpose of forming a polymer in the composition. "Polar compound" assists alignment of liquid crystal molecules by interaction of a polar group with substrate surface.

The liquid crystal composition is prepared by mixing a plurality of liquid crystal compounds. A proportion (content) of the liquid crystal compounds is expressed in terms of weight percent (% by weight) based on the weight of the liquid crystal composition. An additive such as an optically active compound, an antioxidant, an ultraviolet light absorber, a dye, an antifoaming agent, the polymerizable compound, a polymerization initiator, a polymerization inhibitor and the polar compound is added to the liquid crystal composition when necessary. A proportion (amount of addition) of the additive is expressed in terms of weight percent (% by weight) based on the weight of the liquid crystal composition in a manner similar to the proportion of the liquid crystal compound. Weight parts per million (ppm) may be occasionally used. A proportion of the polymerization initiator and the polymerization inhibitor is exceptionally expressed based on the weight of the polymerizable compound.

A compound in accordance with the invention may be occasionally abbreviated as "compound (1)." Compound (1) means one compound, a mixture of two compounds or a mixture of three or more compounds represented by any one of formula (1-7) to formula (1-34) of claim 1. Symbols such as A¹, B² and C¹ surrounded by a hexagonal shape correspond to rings such as ring A¹, ring B¹ and ring C¹, respectively. The hexagonal shape represents a six-membered ring such as a cyclohexane ring and a benzene ring, or a fused ring such as a naphthalene ring. An oblique line crossing one piece of the hexagonal shape represents that arbitrary hydrogen on the ring may be replaced by a group such as -Sp¹-P¹. A subscript such as c, d and e represents the number of groups to be replaced. When a subscript is 0, no such replacement exists. In an expression "ring A and ring C are independently X, Y or Z," a subject is plurality, and therefore a term "independently" is used. When the subject is "ring A," the subject is a singular, and therefore a term "independently" is not used.

A symbol of terminal group R¹¹ is used in a plurality of compounds in chemical formulas of component compounds. In the compounds, two groups represented by two pieces of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol such as R¹², R¹³ and Z¹¹. In compound (8), when i is 2, two pieces of ring D¹ exist. In the compound, two groups represented by two pieces of ring D¹ may be identical or different. When i is larger than 2, a same rule applies also to two pieces of arbitrary D¹. A same rule applies also to any other symbol.

An expression "at least one piece of 'A'" means that the number of 'A' is arbitrary. An expression "at least one piece of 'A' may be replaced by 'B'" means that, when the number of 'A' is 1, a position of 'A' is arbitrary, and also when the number of 'A' is 2 or more, positions thereof can be selected without restriction. A same rule applies also to an expression "at least one piece of 'A' is replaced by 'B'." An expression "at least one piece of A may be replaced by B, C or D" includes a case where at least one piece of A is replaced by B, a case where at least one piece of A is replaced by C, and a case where at least one piece of A is replaced by D, and also a case where a plurality of pieces of A are replaced by at least two of B, C and D. For example, "alkyl in which at least one piece of -CH₂- (or -CH₂CH₂-) may be replaced by -O- (or -CH=CH-)" includes alkyl, alkenyl, alkoxy, alkoxyalkyl, alkoxyalkenyl and alkenyloxyalkyl. In addition, a case where two pieces of consecutive -CH₂- are replaced by -O- to form -O-O- is not preferred. In alkyl or the like, a case where -CH₂- of a methyl part (-CH₂-H) is replaced by -O- to form -O-H is not preferred, either.

Halogen means fluorine, chlorine, bromine or iodine. Preferred halogen is fluorine or chlorine. Further preferred halogen is fluorine. In the liquid crystal compound, alkyl is straight-chain alkyl or branched-chain alkyl, but includes no cyclic alkyl. In general, straight-chain alkyl is preferred to branched-chain alkyl. A same rule applies also to a terminal group such as alkoxy and alkenyl. With regard to a configuration of 1,4-cyclohexylene, trans is preferred to cis for increasing the maximum temperature of the nematic phase. Then, 2-fluoro-1,4-phenylene means two divalent groups described below. In a chemical formula, fluorine may be leftward (L) or rightward (R). A same rule applies also to an asymmetrical divalent group formed by removing two hydrogens from a ring, such as tetrahydropyran-2,5-diyl.

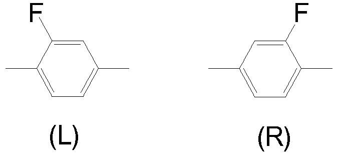

The invention includes items described below.
Item 1. A compound, represented by any one of formula (1-7) to formula (1-34) below.
Item 2. The compound according to item 1, represented by any one of formula (1-7) to formula (1-21) : 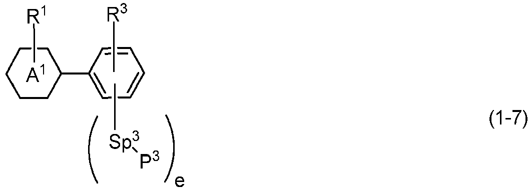 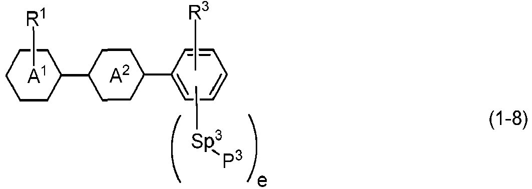 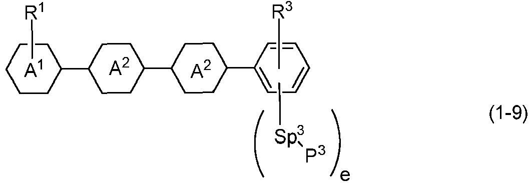 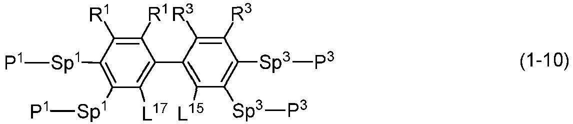 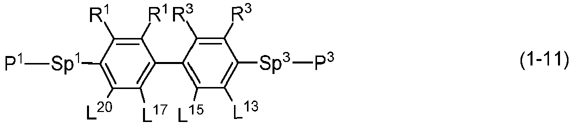 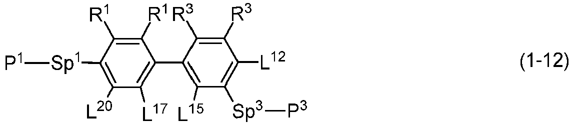 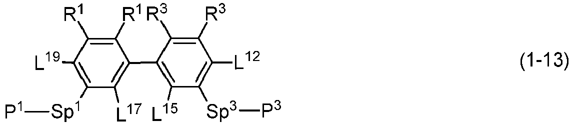 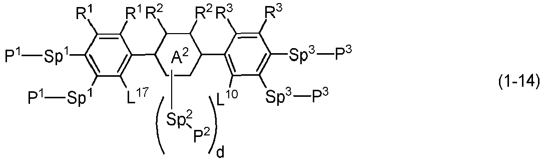 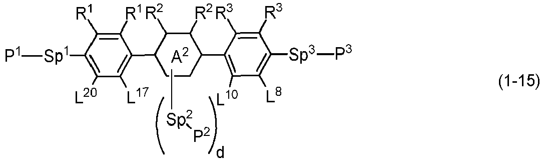 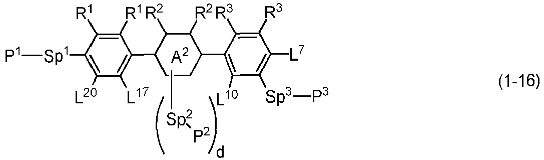 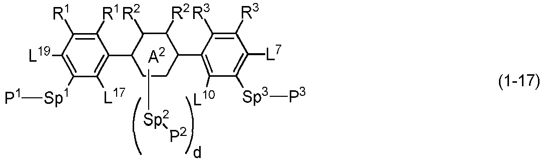 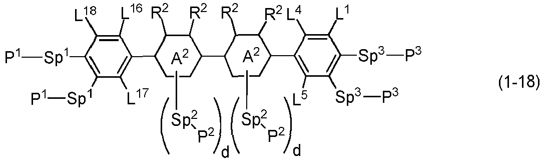 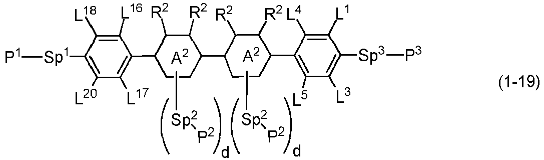 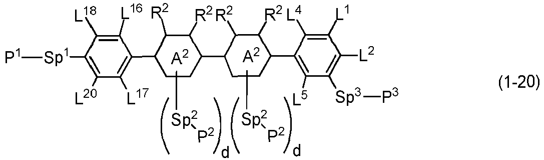 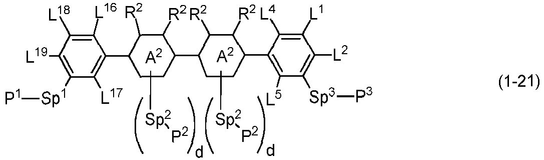 wherein, in formula (1-7) to formula (1-21),
   R¹, R² and R³ are independently hydrogen, alkyl having 1 to 8 carbons, alkenyl having 2 to 8 carbons, alkoxy having 1 to 7 carbons or alkenyloxy having 2 to 7 carbons;
   ring A¹ is cyclohexyl, cyclohexenyl or phenyl, and ring A² is 1,4-cyclohexylene, 1,4-cyclohexenylene or 1,4-phenylene, and in the rings, at least one hydrogen may be replaced by fluorine;
   L¹, L², L³, L⁴, L⁵, L⁷, L⁸, L¹⁰, L¹², L¹³, L¹⁵, L¹⁶, L¹⁷, L¹⁸, L¹⁹ and L²⁰ are independently hydrogen, fluorine, methyl or ethyl; Sp¹, Sp² and Sp³ are independently a single bond or alkylene having 1 to 5 carbons, and in the alkylene, at least one piece of -CH₂-may be replaced by -O-;
   c, d and e are independently, 0, 1 or 2, and a sum of c, d and e is 2, 3 or 4; and
   P¹, P² and P³ are independently a polymerizable group represented by formula (P-11) : 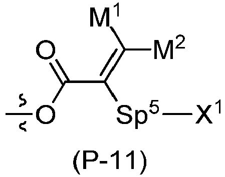 wherein, in formula (P-11), M¹ and M² are independently hydrogen, fluorine, methyl, ethyl or trifluoromethyl; Sp⁵ is a single bond or alkylene having 1 to 5 carbons, and in the alkylene, at least one piece of -CH₂- may be replaced by -O-; and X¹ is -OH or -NH₂.
Item 3. The compound according to item 2, wherein, in formula (1-7) to formula (1-21) described in item 6, R¹, R² and R³ are independently hydrogen, alkyl having 1 to 8 carbons, alkenyl having 2 to 8 carbons, alkoxy having 1 to 7 carbons or alkenyloxy having 2 to 7 carbons;
   ring A¹ is cyclohexyl, cyclohexenyl or phenyl, and ring A² is 1,4-cyclohexylene, 1,4-cyclohexenylene or 1,4-phenylene, and in the rings, at least one hydrogen may be replaced by fluorine;
   L¹, L², L³, L⁴, L⁵, L⁷, L⁸, L¹⁰, L¹², L¹³, L¹⁵, L¹⁶, L¹⁷, L¹⁸, L¹⁹ and L²⁰ are independently hydrogen, fluorine, methyl or ethyl; Sp¹, Sp² and Sp³ are independently a single bond or alkylene having 1 to 5 carbons, and in the alkylene, at least one piece of -CH₂-may be replaced by -O-;
   c, d and e are independently 0, 1 or 2, and a sum of c, d and e is 2, 3 or 4; and
   P¹, P² and P³ are independently a polymerizable group represented by formula (P-11) : 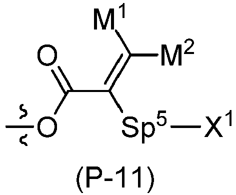 wherein, in formula (P-11), M¹ and M² are independently hydrogen, fluorine, methyl or ethyl; Sp⁵ is a single bond or alkylene having 1 to 5 carbons, and in the alkylene, at least one piece of -CH₂-may be replaced by -O-; and X¹ is -OH or -NH₂.
Item 4. The compound according to item 1, represented by any one of formula (1-22) to formula (1-34), 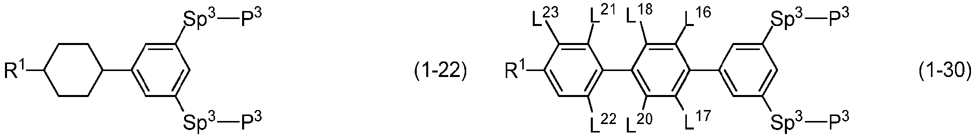 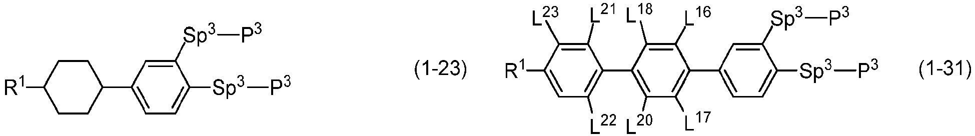 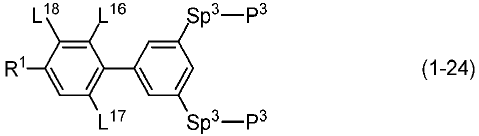 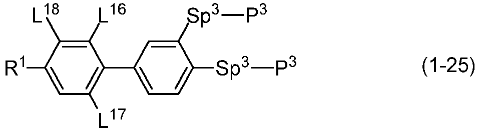 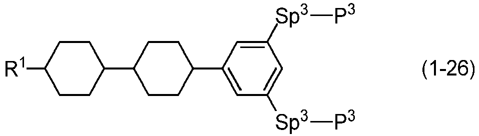 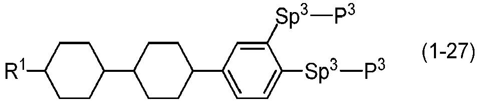 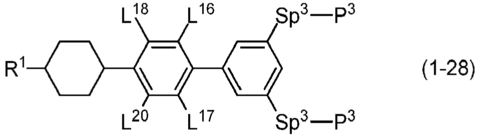 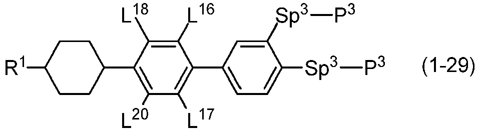 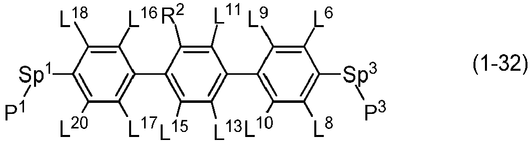 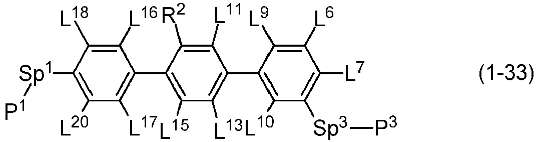 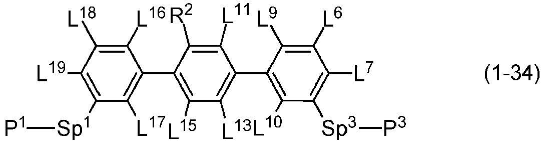 wherein, in formula (1-22) to formula (1-34),
   R¹ and R² are independently alkyl having 1 to 7 carbons, alkenyl having 2 to 7 carbons, alkoxy having 1 to 6 carbons or alkenyloxy having 2 to 6 carbons;
   L⁶, L⁷, L⁸, L⁹, L¹⁰, L¹¹, L¹³, L¹⁵, L¹⁶, L¹⁷, L¹⁸, L¹⁹, L²⁰, L²¹, L²² and L²³ are independently hydrogen, fluorine, methyl or ethyl;
   Sp¹ and Sp³ are independently a single bond or alkylene having 1 to 3 carbons, and in the alkylene, at least one piece of -CH₂-may be replaced by -O-; and
   P¹ and P³ are independently a polymerizable group represented by formula (P-111): 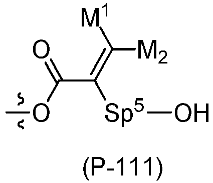 wherein, in formula (P-111), M¹ and M² are independently hydrogen, fluorine or methyl; and Sp⁵ is a single bond or alkylene having 1 to 3 carbons, and in the alkylene, at least one piece of -CH₂-may be replaced by -O-.
Item 5. A liquid crystal composition, containing at least one compound according to any one of items 1 to 4 as component A.
Item 6. The liquid crystal composition according to item 5, containing at least one compound selected from the group of compounds represented by formula (2) to formula (4) as component B: 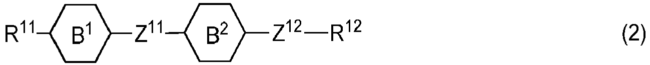 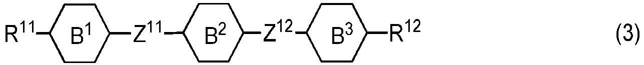 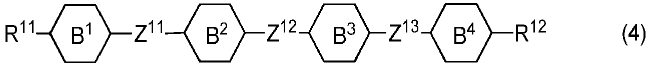 wherein, in formula (2) to formula (4),
   R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
   Z¹¹, Z¹² and Z¹³ are independently a single bond, -CH₂CH₂-, -CH=CH-, -C≡C- or -COO-.
Item 7. The liquid crystal composition according to item 5 or 6, further containing at least one compound selected from the group of compounds represented by formula (5) to formula (7) as component C: 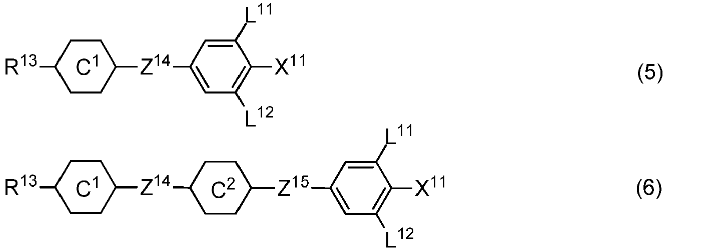 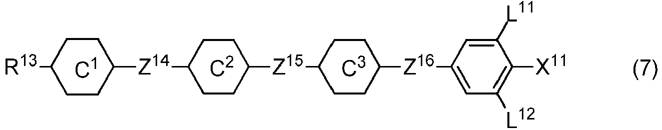 wherein, in formula (5) to formula (7),
   R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹¹ is fluorine, chlorine, -OCF₃, -OCHF₂, -CF₃, -CHF₂, -CH₂F, -OCF₂CHF₂ or -OCF₂CHFCF₃;
   ring C¹, ring C² and ring C³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁴, Z¹⁵ and Z¹⁶ are independently a single bond, -CH₂CH₂-, -CH=CH-, -C≡C-, -COO-, -CF₂O-, -OCF₂-, -CH₂O- or -(CH₂)₄-; and
   L¹¹ and L¹² are independently hydrogen or fluorine.
Item 8. The liquid crystal composition according to any one of items 5 to 7, further containing at least one compound selected from the group of compounds represented by formula (8) as component D: 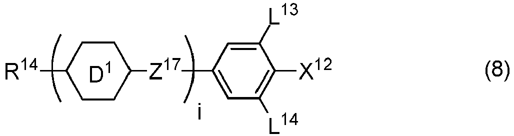 wherein, in formula (8),
   R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
   X¹² is -C≡N or -C≡C-C≡N;
   ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
   Z¹⁷ is a single bond, -CH₂CH₂-, -C≡C-, -COO-, -CF₂O-, -OCF₂- or -CH₂O-;
   L¹³ and L¹⁴ are independently hydrogen or fluorine; and
   i is 1, 2, 3 or 4.
Item 9. The liquid crystal composition according to any one of items 5 to 8, further containing at least one compound selected from the group of compounds represented by formula (9) to formula (15) as component E: 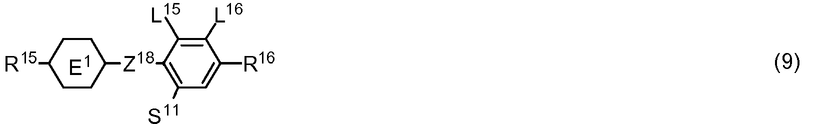 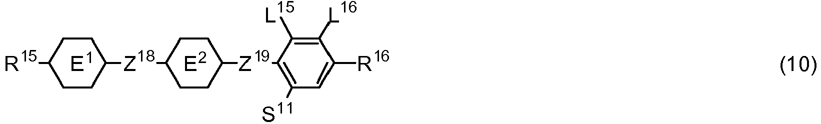 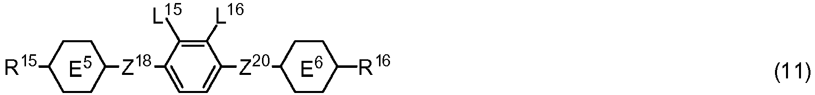 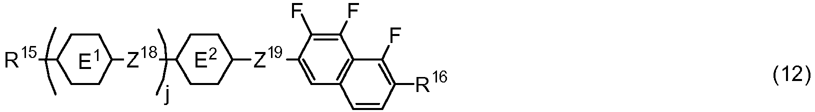 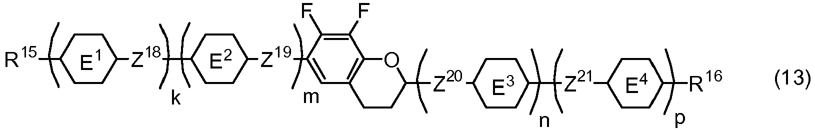 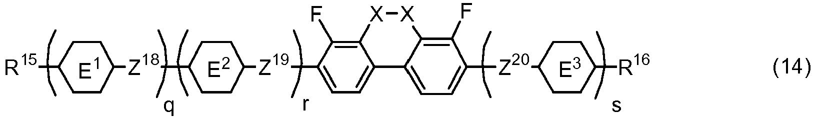 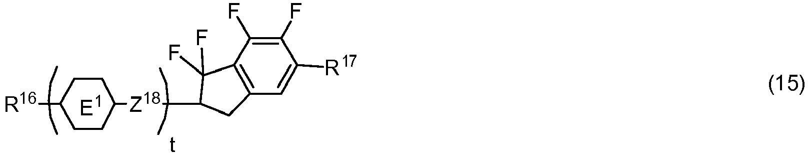 wherein, in formula (9) to formula (15),
   R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine;
   ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
   ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
   Z¹⁸, Z¹⁹, Z²⁰ and Z²¹ are independently a single bond, -CH₂CH₂-, -COO-, -CH₂O-, -OCF₂- or -OCF₂CH₂CH₂-;
   L¹⁵ and L¹⁶ are independently fluorine or chlorine;
   S¹¹ is hydrogen or methyl;
   X is -CHF- or -CF₂-; and
   j, k, m, n, p, q, r and s are independently 0 or 1, a sum of k, m, n and p is 1 or 2, a sum of q, r and s is 0, 1, 2 or 3, and t is 1, 2 or 3.
Item 10. The liquid crystal composition according to any one of items 5 to 9, further containing at least one compound selected from the group of polymerizable compounds represented by formula (16) as component F: 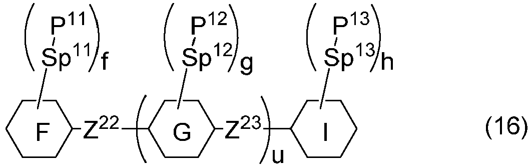 wherein, in formula (16),
   ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
   Z²² and Z²³ are independently a single bond or alkylene having 1 to 10 carbons, and in the alkylene, at least one piece of -CH₂-may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
   P¹¹, P¹² and P¹³ are independently a polymerizable group;
   Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
   u is 0, 1 or 2; and
   f, g and h are independently 0, 1, 2, 3 or 4, and a sum of f, g and h is 1 or more.
Item 11. The liquid crystal composition according to item 10, wherein, in formula (16) described in item 14, P¹¹, P¹² and P¹³ are independently a group selected from the group of polymerizable groups represented by formula (P-2) to formula (P-6) : 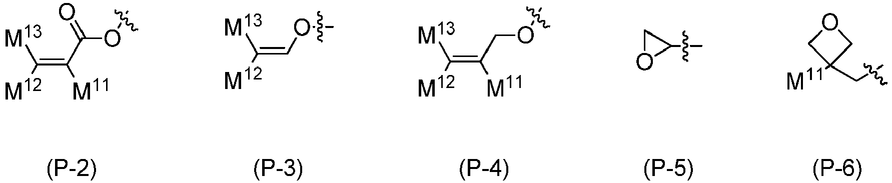 wherein, in formula (P-2) to formula (P-6), M¹¹, M¹² and M¹³ are independently hydrogen, fluorine, alkyl having 1 to 5 carbons, or alkyl having 1 to 5 carbons in which at least one hydrogen is replaced by halogen.
Item 12. The liquid crystal composition according to item 10 or 11, wherein component F is at least one compound selected from the group of polymerizable compounds represented by formula (16-1) to formula (16-7) : 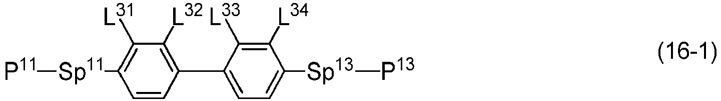 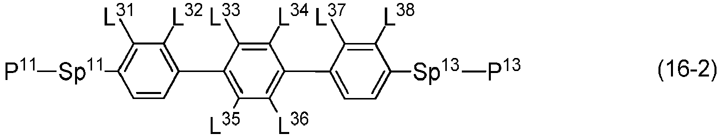 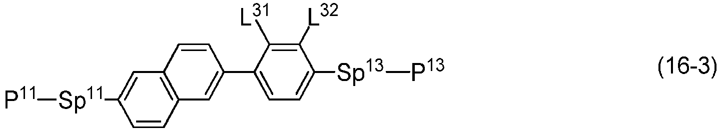 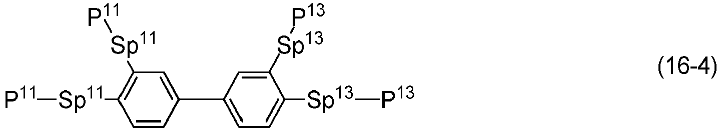 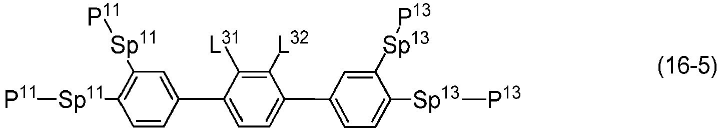 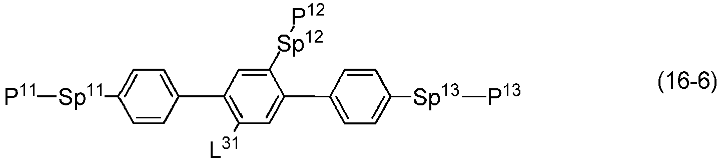 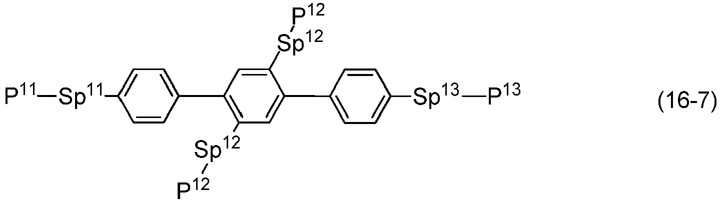 wherein, in formula (16-1) to formula (16-7), P¹¹, P¹² and P¹³ are independently a group selected from the group of polymerizable groups represented by formula (P-2) to formula (P-4), in which M¹¹, M¹² and M¹³ are independently hydrogen, fluorine, alkyl having 1 to 5 carbons, or alkyl having 1 to 5 carbons in which at least one hydrogen is replaced by halogen: 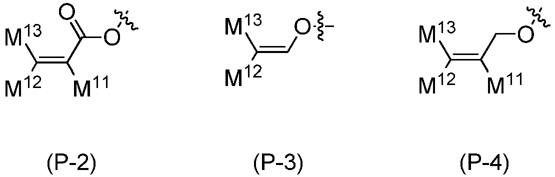 wherein, L³¹, L³², L³³, L³⁴, L³⁵, L³⁶, L³⁷ and L³⁸ are independently hydrogen, fluorine or methyl; and Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂-may be replaced by -CH=CH- or -C≡C-, and in the groups, at least one hydrogen may be replaced by fluorine or chlorine.
Item 13. The liquid crystal composition according to any one of items 5 to 12, further containing at least one of a polymerizable compound different from the compounds represented by formula (1) and formula (16), a polymerization initiator, a polymerization inhibitor, an optically active compound, an antioxidant, an ultraviolet light absorber, a light stabilizer, a heat stabilizer and an antifoaming agent.
Item 14. A liquid crystal display device, including at least one liquid crystal composition according to any one of items 5 to 13.

The invention further includes the following items: (a) the liquid crystal composition, further containing at least two of additives such as a polymerizable compound, a polymerization initiator, a polymerization inhibitor, an optically active compound, an antioxidant, an ultraviolet light absorber, a light stabilizer, a heat stabilizer and an antifoaming agent; (b) a polymerizable composition prepared by adding a polymerizable compound different from compound (1) or compound (16) to the liquid crystal composition; (c) a polymerizable composition prepared by adding compound (1) and compound (16) to the liquid crystal composition; (d) a liquid crystal composite prepared by polymerizing the polymerizable composition; (e) a polymer sustained alignment mode device including the liquid crystal composite; and (f) a polymer sustained alignment mode device, prepared by using a polymerizable composition prepared by adding compound (1), compound (16), and a polymerizable compound different from compound (1) or compound (16) to the liquid crystal composition.

An aspect of compound (1), synthesis of compound (1), the liquid crystal composition and the liquid crystal display device will be described in the order.

### 1. Aspect of compound (1)

Compound (1) of the invention has a future of having a mesogen moiety constituted of at least one ring, and a plurality of polar groups. The polar group noncovalently interacts with a substrate surface of glass (or metal oxide), and therefore compound (1) is useful. One of applications is an additive for the liquid crystal composition used in the liquid crystal display device. Compound (1) is added for the purpose of controlling alignment of liquid crystal molecules. Such an additive is preferably chemically stable under conditions that the additive is tighten sealed in the device, has high solubility in the liquid crystal composition, and a large voltage holding ratio when used in the liquid crystal display device. Compound (1) satisfies such characteristics to a significant extent.

Preferred examples of compound (1) will be described. Preferred examples of a symbol such as R¹, Z¹, A¹, Sp¹, P¹, n and a in compound (1) are applied also to a subordinate formula of formula (1-7) to formula (1-34) for compound (1). In compound (1), characteristics can be arbitrarily adjusted by suitably combining kinds of the groups. Compound (1) may contain a larger amount of isotope such as ²H (deuterium) and ¹³C than the amount of natural abundance because no significant difference exists in the characteristics of the compound.

Preferred R¹ to R³ are hydrogen, alkyl having 1 to 7 carbons, alkenyl having 2 to 7 carbons, alkoxy having 1 to 7 carbons or alkenyloxy having 2 to 7 carbons. Further preferred R¹ to R³ are hydrogen, alkyl having 1 to 3 carbons or alkoxy having 1 to 3 carbons. Particularly preferred R¹ to R³ are hydrogen, methyl, ethyl, methoxy or ethoxy.

Preferred ring A¹ is cyclohexyl, cyclohexenyl, phenyl or fluorophenyl. Particularly preferred ring A¹ is cyclohexyl or phenyl.

Preferred ring A² is 1,4-cyclohexylene, 1,4-cyclohexenylene, 1,4-phenylene, 2-fluoro-1,4-phenylene or 2,3-difluoro-1,4-phenylene. Particularly preferred ring A² is 1,4-cyclohexylene or 1,4-phenylene.

Preferred Sp¹ to Sp³ in formula (1-7) to (1-21) are a single bond, alkylene having 1 to 5 carbons, or alkylene having 1 to 5 carbons in which one piece of -CH₂- is replaced by -O-. Preferred Sp¹ to Sp³ in formula (1-22) to (1-34) are a single bond, alkylene having 1 to 3 carbons, or alkylene having 1 to 3 carbons in which one piece of -CH₂- is replaced by -O-. Further preferred Sp¹ to Sp³ in formula (1-7) to (1-34) are a single bond, -CH₂-, -(CH₂)₂-, -(CH₂)₃-, -(CH₂)₂O- or -O(CH₂)₂-. Particularly preferred Sp¹ to Sp³ in formula (1-7) to (1-34) are a single bond.

In formula (1-7) to formula (1-34), c, d and e are independently 0, 1 or 2, and a sum of c, d and e is 2, 3 or 4. A preferred sum thereof is 2 or 3. A further preferred sum thereof is 2.

In formula (1-7) to formula (1-21), P¹, P² and P³ are independently a polymerizable group represented by formula (P-1).

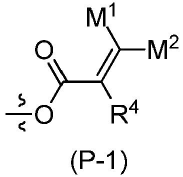

In formula (1-7) to formula (1-21), M¹ and M² of formula (P-1) are independently hydrogen, fluorine, methyl, ethyl or trifluoromethyl. In formula (1-22) to formula (1-34), M¹ and M² of formula (P-1) are independently hydrogen, fluorine or methyl. Preferred M¹ or M² of formula (P-1) is hydrogen or methyl for increasing a reactivity. Further preferred M¹ or M² is hydrogen.

R⁴ is a polar group represented by formula (1a).

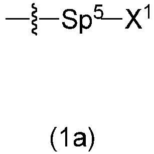

In formula (1-7) to formula (1-21), Sp⁵ of formula (1a) is a single bond or alkylene having 1 to 5 carbons, and in the alkylene, at least one piece of -CH₂- may be replaced by -O-. In formula (1-22) to formula (1-34), X¹ of formula (1a) is -OH and Sp⁵ is a single bond or alkylene having 1 to 3 carbons, and in the alkylene, at least one piece of -CH₂- may be replaced by -O-.

Preferred Sp⁵ is single bond, -CH₂-, -(CH₂)₂-, -(CH₂)₃-, -(CH₂)O₂- or -O(CH₂)₂-. Particularly preferred Sp⁵ is a single bond.

In formula (1-7) to formula (1-21), X¹ of formula (1a) is -OH or -NH₂. In formula (1-22) to formula (1-34), X¹ of formula (1a) is -OH.

In formula (1-7) to formula (1-21), particularly preferred X¹ of formula (1a) is -OH.

### 2. Synthesis of compound (1)

A method for synthesizing compound (1) will be described. Compound (1) can be prepared by suitably combining methods in synthetic organic chemistry. Any compounds whose synthetic methods are not described above are prepared according to methods described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course" (Shin Jikken Kagaku Koza in Japanese) (Maruzen Co., Ltd.).

### 2-1. Formation of a bonding group

An example of a method for forming a bonding group in compound (1) is as described in a scheme described below. In the scheme, MSG¹ (or MSG²) is a monovalent organic group having at least one ring. The monovalent organic groups represented by a plurality of MSG¹ (or MSG²) may be identical or different. Compounds (1A) to (1G) correspond to compound (1) or an intermediate of compound (1) .

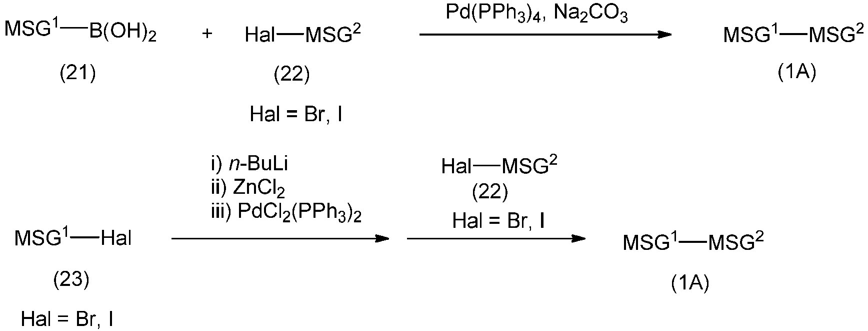

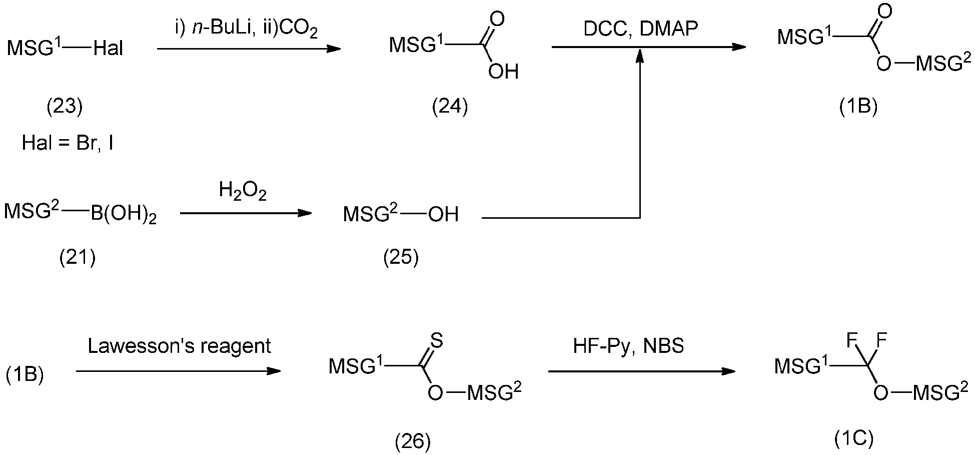

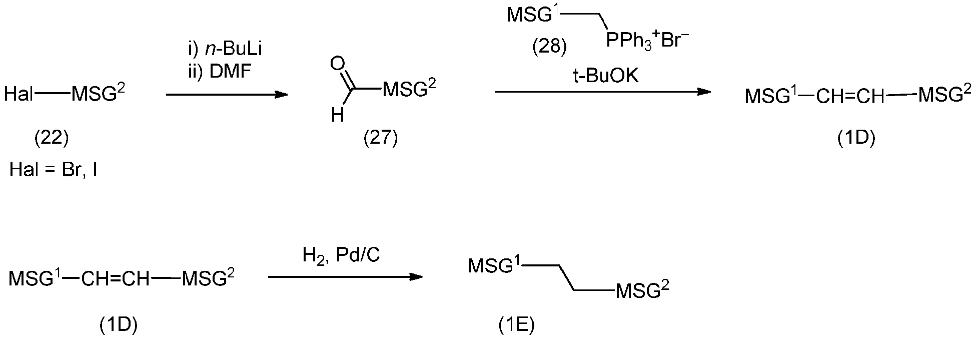

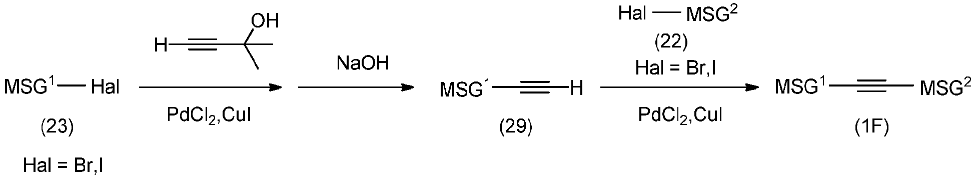

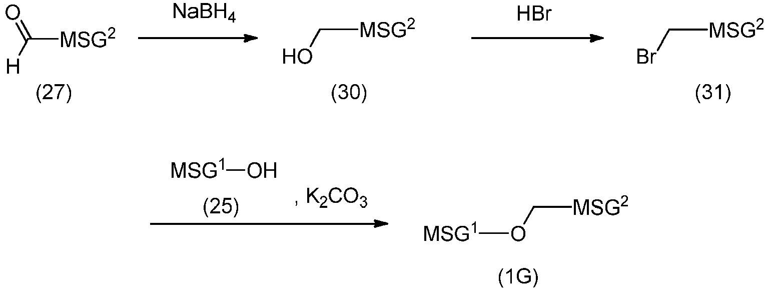

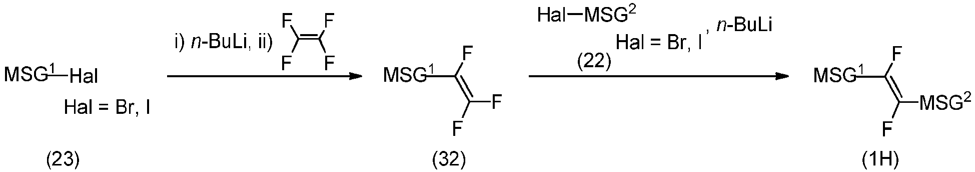

### (I) Formation of a single bond

Compound (1A) is prepared by allowing aryl boronic acid (21) to react with compound (22) in the presence of a carbonate and a tetrakis(triphenylphosphine)palladium catalyst. Compound (1A) is also prepared by allowing compound (23) to react with n-butyllithium and subsequently with zinc chloride, and further with compound (22) in the presence of a dichlorobis(triphenylphosphine)palladium catalyst.

### (II) Formation of -COO- and -OCO-

Carboxylic acid (24) is obtained by allowing compound (23) to react with n-butyllithium and subsequently with carbon dioxide. Compound (1B) having -COO- is prepared by dehydration of carboxylic acid (24) and phenol (25) derived from compound (21) in the presence of 1,3-dicyclohexylcarbodiimide (DCC) and 4-dimethylaminopyridine (DMAP). A compound having -OCO- is also prepared by the method.

### (III) Formation of -CF₂O- and -OCF₂-

Compound (26) is obtained by sulfurizing compound (1B) with Lawesson's reagent. Compound (1C) having -CF₂O- is prepared by fluorinating compound (26) with a hydrogen fluoride-pyridine complex and N-bromosuccinimide (NBS). Refer to M. Kuroboshi et al., Chem. Lett., 1992, 827. Compound (1C) is also prepared by fluorinating compound (26) with (diethylamino)sulfur trifluoride (DAST) . Refer to W. H. Bunnelle et al., J. Org. Chem. 1990, 55, 768. A compound having -OCF₂- is also prepared by the method.

### (IV) Formation of -CH=CH-

Aldehyde (27) is obtained by allowing compound (22) to react with n-butyllithium and subsequently with N,N-dimethylformamide (DMF). Compound (1D) is prepared by allowing phosphorus ylide generated by allowing phosphonium salt (28) to react with potassium t-butoxide to react with aldehyde (27). A cis isomer may be generated depending on reaction conditions, and therefore the cis isomer is isomerized into a trans isomer according to a publicly known method when necessary.

### (V) Formation of -CH₂CH₂-

Compound (1E) is prepared by hydrogenating compound (1D) in the presence of a palladium on carbon catalyst.

### (VI) Formation of -C≡C-

Compound (29) is obtained by allowing compound (23) to react with 2-methyl-3-butyn-2-ol in the presence of a catalyst of dichloropalladium and copper iodide, and then performing deprotection under basic conditions. Compound (1F) is prepared by allowing compound (29) to react with compound (22) in the presence of a catalyst of dichlorobis(triphenylphosphine)palladium and copper halide.

### (VII) Formation of -CH₂O- and -OCH₂-

Compound (30) is obtained by reducing compound (27) with sodium borohydride. Compound (31) is obtained by brominating the obtained compound with hydrobromic acid. Compound (1G) is prepared by allowing compound (25) to react with compound (31) in the presence of potassium carbonate. A compound having -OCH₂-is also prepared according to the method.

### (VIII) Formation of -CF=CF-

Compound (32) is obtained by treating compound (23) with n-butyllithium, and then allowing the treated material to react with tetrafluoroethylene. Compound (1H) is prepared by treating compound (22) with n-butyllithium, and then allowing the treated compound to react with compound (32).

### 2-2. Formation of ring A²

A starting material is commercially available or a synthesis method is well known with regard to a ring such as 1,4-cyclohexylene, 1,4-cyclohexenylene, 1,4-phenylene, 2-fluoro-1,4-phenylene, 2-methyl-1,4-phenylene, 2-ethyl-1,4-phenylene, naphthalene-2,6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl and pyridine-2,5-diyl.

### 2-3. Synthesis Example

An example of a method for preparing compound (1) is as described below. In the compounds, definitions of symbols such as R¹ and ring A¹ are as described in item 1.

Compound (1-71) can be prepared by the following methods. Compound (52) is obtained by allowing compound (51) to react with formaldehyde in the presence of 1,4-diazabicyclo[2.2.2]octane (DABCO). Next, compound (53) is obtained by allowing t-butyldimethylsilyl chloride and imidazole to act with the obtained material, and then compound (54) is obtained by hydrolyzing the obtained compound with a base such as lithium hydroxide. Compound (56) is obtained by allowing compound (55) to react with 1-bromo-3,5-dimethoxybenzene in the presence of a tetrakis(triphenylphosphine)palladium catalyst and a base. Meanwhile, compound (57) is obtained by allowing compound (56) to react with boron tribromide. Compound (58) is obtained by allowing compound (57) to react with compound (54) in the presence of DCC and DMAP, and then compound (1-71) is derived by performing deprotection using tetrabutylammonium fluoride (TBAF)

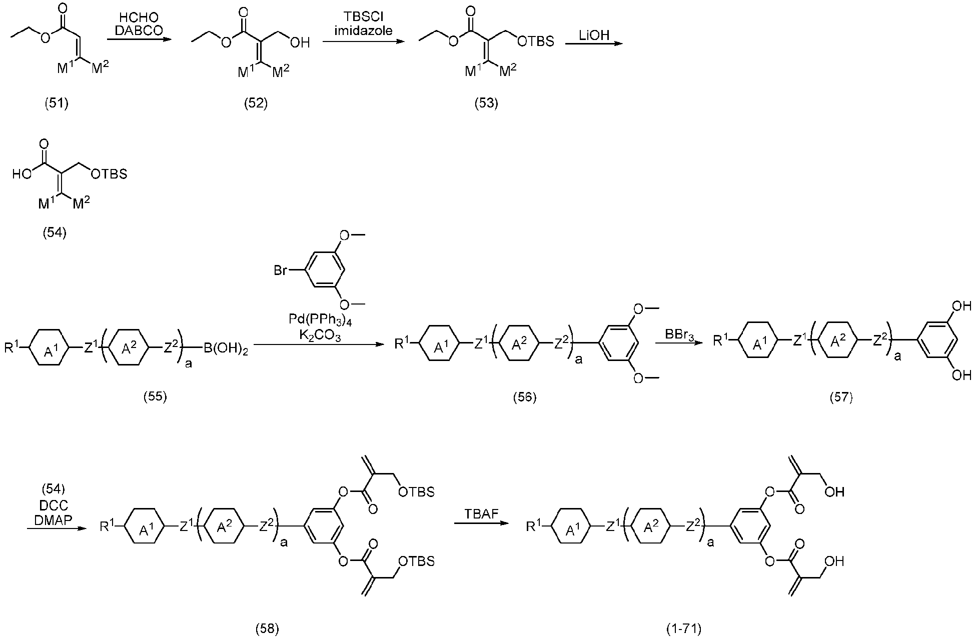

Compound (1-72) can be prepared by the following methods. Compound (59) is obtained by allowing compound (57) to react in the presence of ethylene carbonate and a base. Compound (60) is obtained by allowing compound (59) to react with compound (54) in the presence of DCC and DMAP, and then compound (1-72) is derived by performing deprotection using tetrabutylammonium fluoride (TBAF).

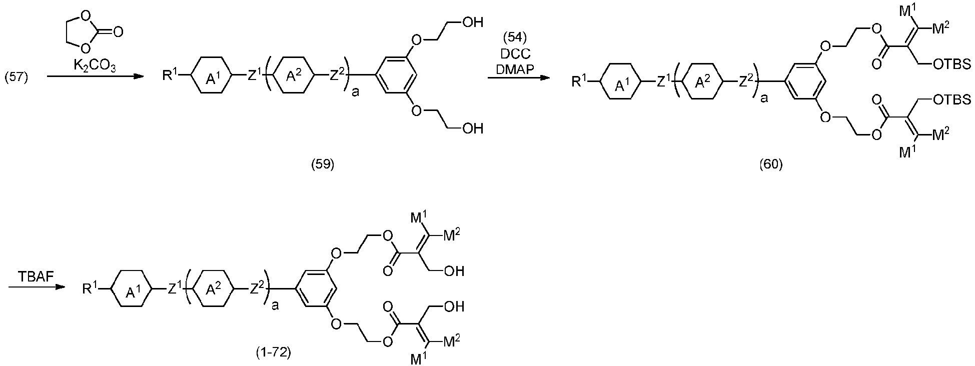

Compound (1-73) can be prepared by the following methods. Compound (61) is obtained by allowing compound (1-71) to react in the presence of phosphorustribromide. Indium is allowing to act with compound (61), and then compound (1-73) is derived by allowing the obtained material to react with formaldehyde.

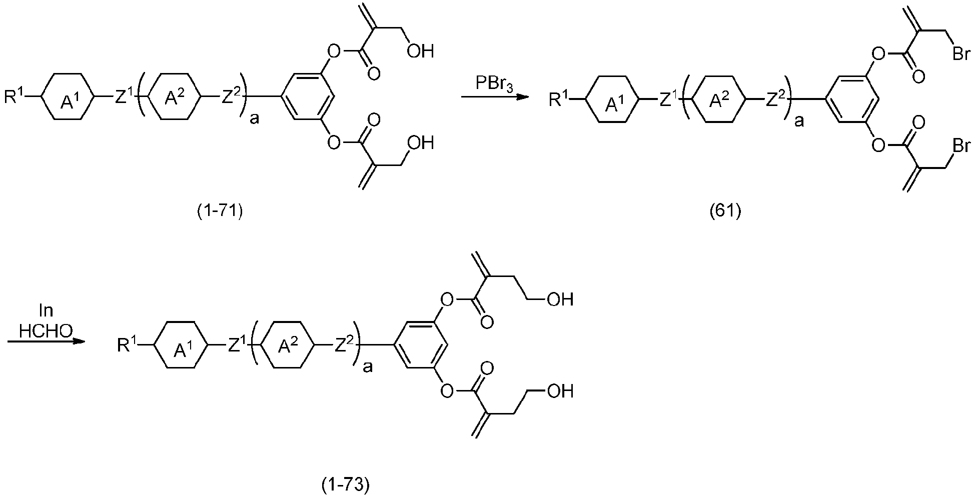

### 3. Liquid crystal composition

### 3-1. Component compound

A liquid crystal composition of the invention contains compound (1) as component A. Compound (1) noncovalently interacts with a substrate of a device, and thus can control alignment of liquid crystal molecules. The composition contains compound (1) as component A, and preferably further contains a liquid crystal compound selected from components B, C, D and E shown below. Component B includes compounds (2) to (4). Component C includes compounds (5) to (7). Component D includes compound (8). Component E includes compounds (9) to (15). The composition may contain any other liquid crystal compound different from compounds (2) to (15). When the composition is prepared, components B, C, D and E are preferably selected by taking into account magnitude of positive or negative dielectric anisotropy, or the like. A composition in which the components are suitably selected has high maximum temperature, low minimum temperature, small viscosity, suitable optical anisotropy (more specifically, large optical anisotropy or small optical anisotropy), large positive or negative dielectric anisotropy, large specific resistance, stability to heat or ultraviolet light and a suitable elastic constant (more specifically, a large elastic constant or a small elastic constant).

Compound (1) is added to the composition for the purpose of controlling alignment of liquid crystal molecules. A preferred proportion of compound (1) is 0.05% by weigh or more for aligning liquid crystal molecules, and 10% by weight or less for preventing poor display of a device. A further preferred proportion is in the range from 0.1% by weight to 7% by weight. A particularly preferred proportion is in the range from 0.5% by weight to 5% by weight. The above proportions are applied also to the total amount of compound (1) and compound (16).

Component B includes a compound in which two terminal groups are alkyl or the like. Preferred examples of component B include compounds (2-1) to (2-11), compounds (3-1) to (3-19) and compounds (4-1) to (4-7). In a compound of component B, R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine.

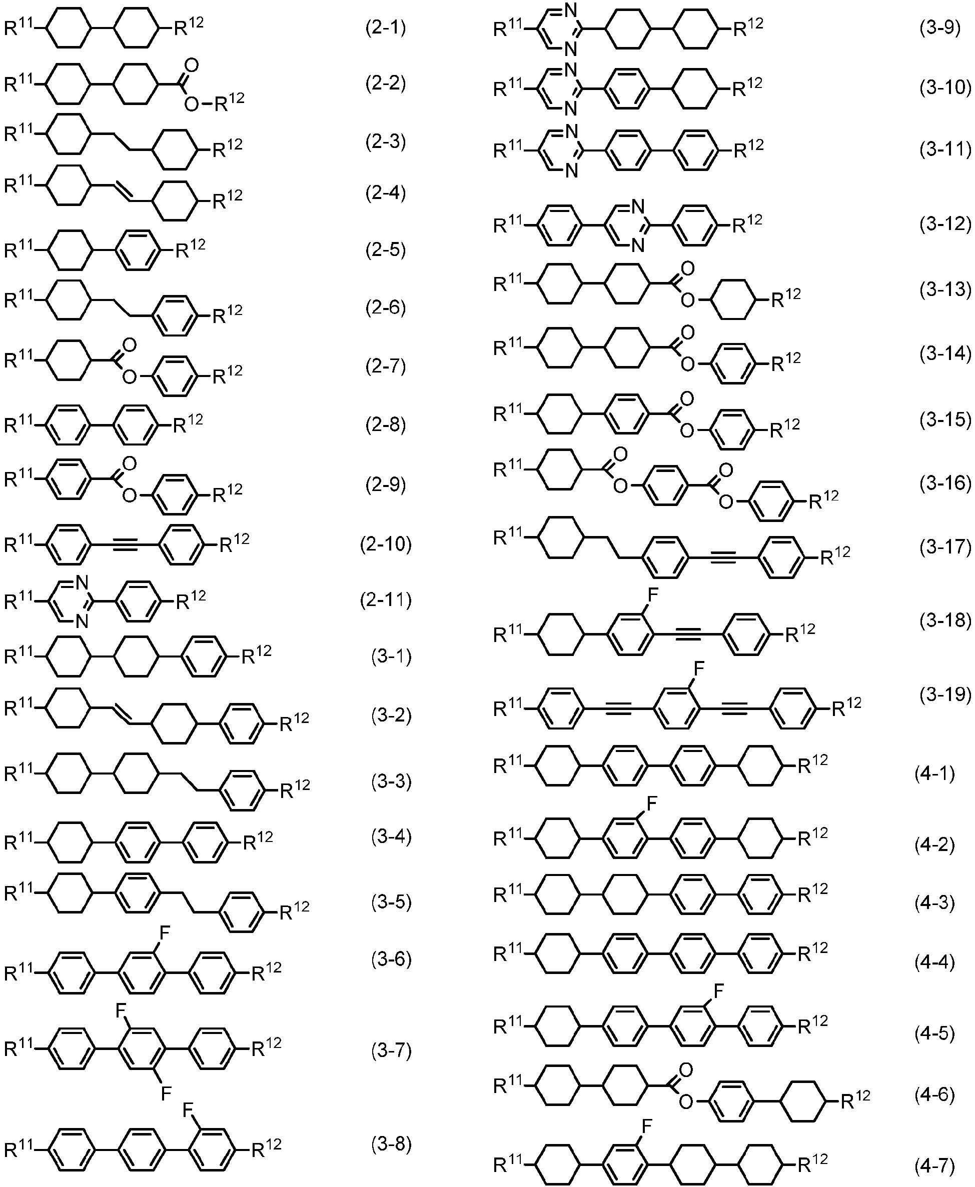

Component B has a small absolute value of dielectric anisotropy, and therefore is a compound close to neutrality. Compound (2) is mainly effective in decreasing the viscosity or adjusting the optical anisotropy. Compounds (3) and (4) are effective in extending the temperature range of a nematic phase by increasing the maximum temperature, or in adjustment of the optical anisotropy.

As a content of component B is increased, the dielectric anisotropy of the composition is decreased, but the viscosity is decreased. Thus, as long as a desired value of a threshold voltage of the device is met, the content is preferably as large as possible. When a composition for the IPS mode, the VA mode or the like is prepared, a content of component B is preferably 30% by weight or more, and further preferably 40% by weight or more, based on the weight of the liquid crystal composition.

Component C is a compound having a halogen-containing group or a fluorine-containing group at a right terminal. Preferred examples of component C include compounds (5-1) to (5-16), compounds (6-1) to (6-113) and compounds (7-1) to (7-57). In a compound of component C, R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹¹ is fluorine, chlorine, -OCF₃, -OCHF₂, -CF₃, -CHF₂, -CH₂F, -OCF₂CHF₂ or -OCF₂CHFCF₃.

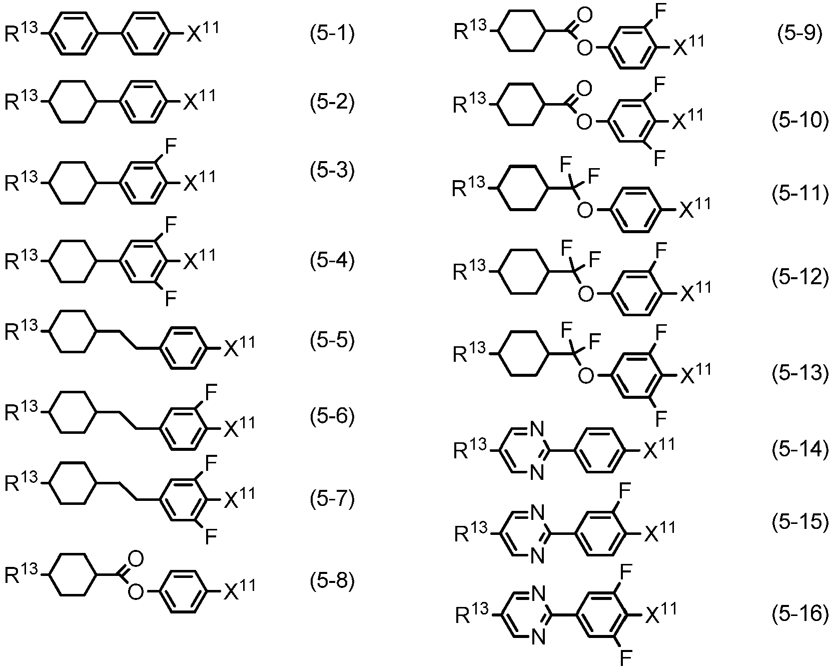

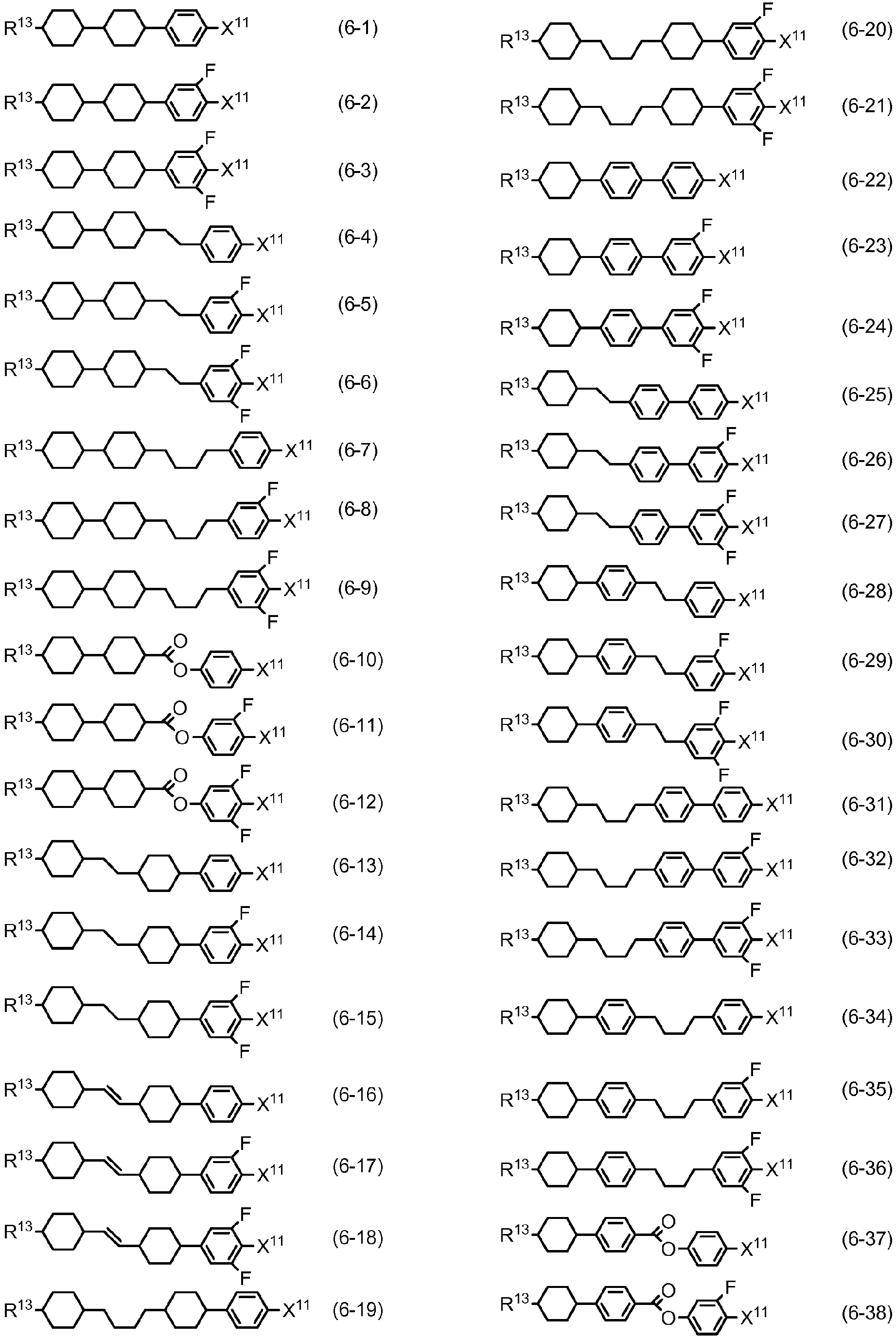

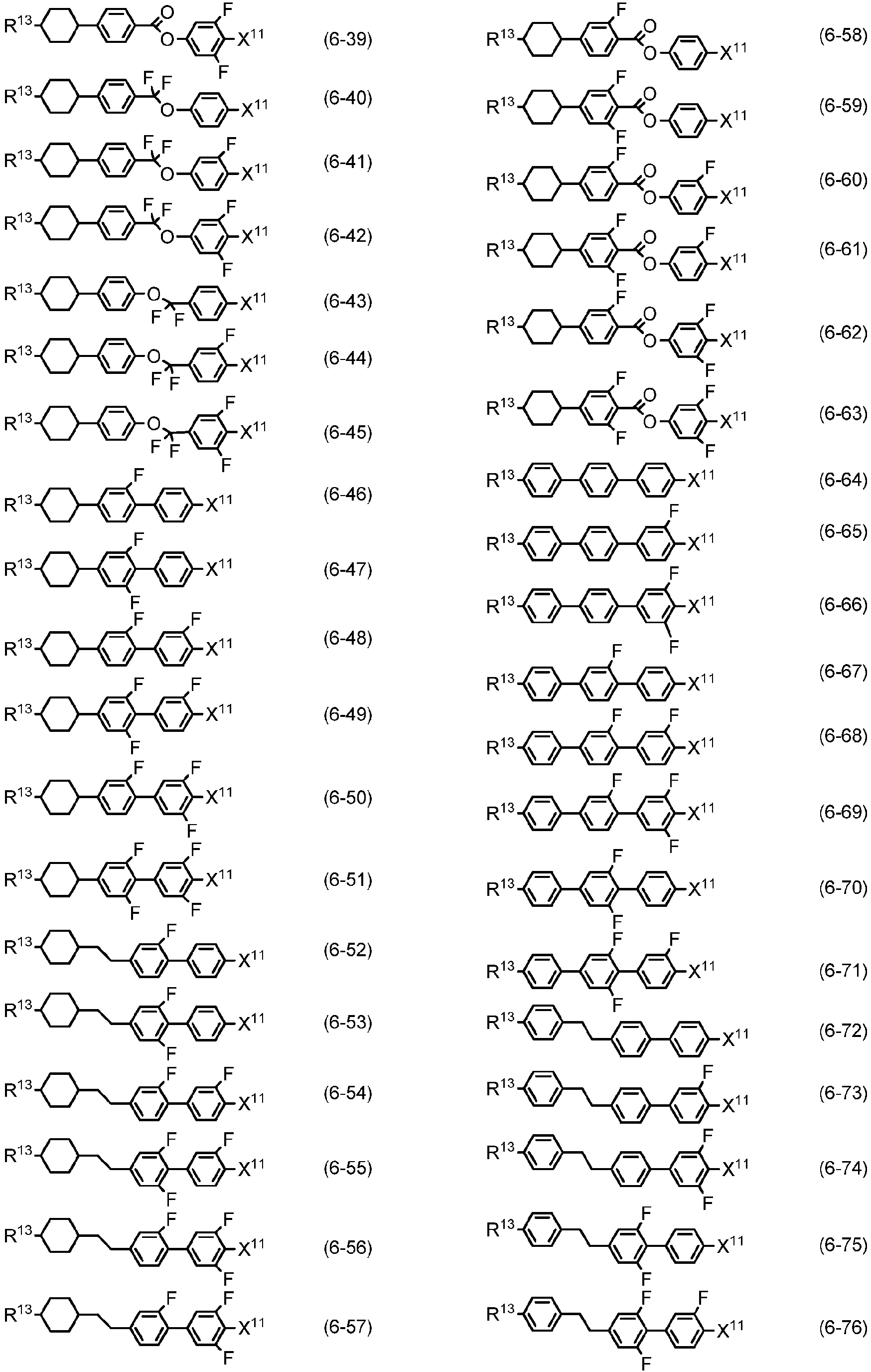

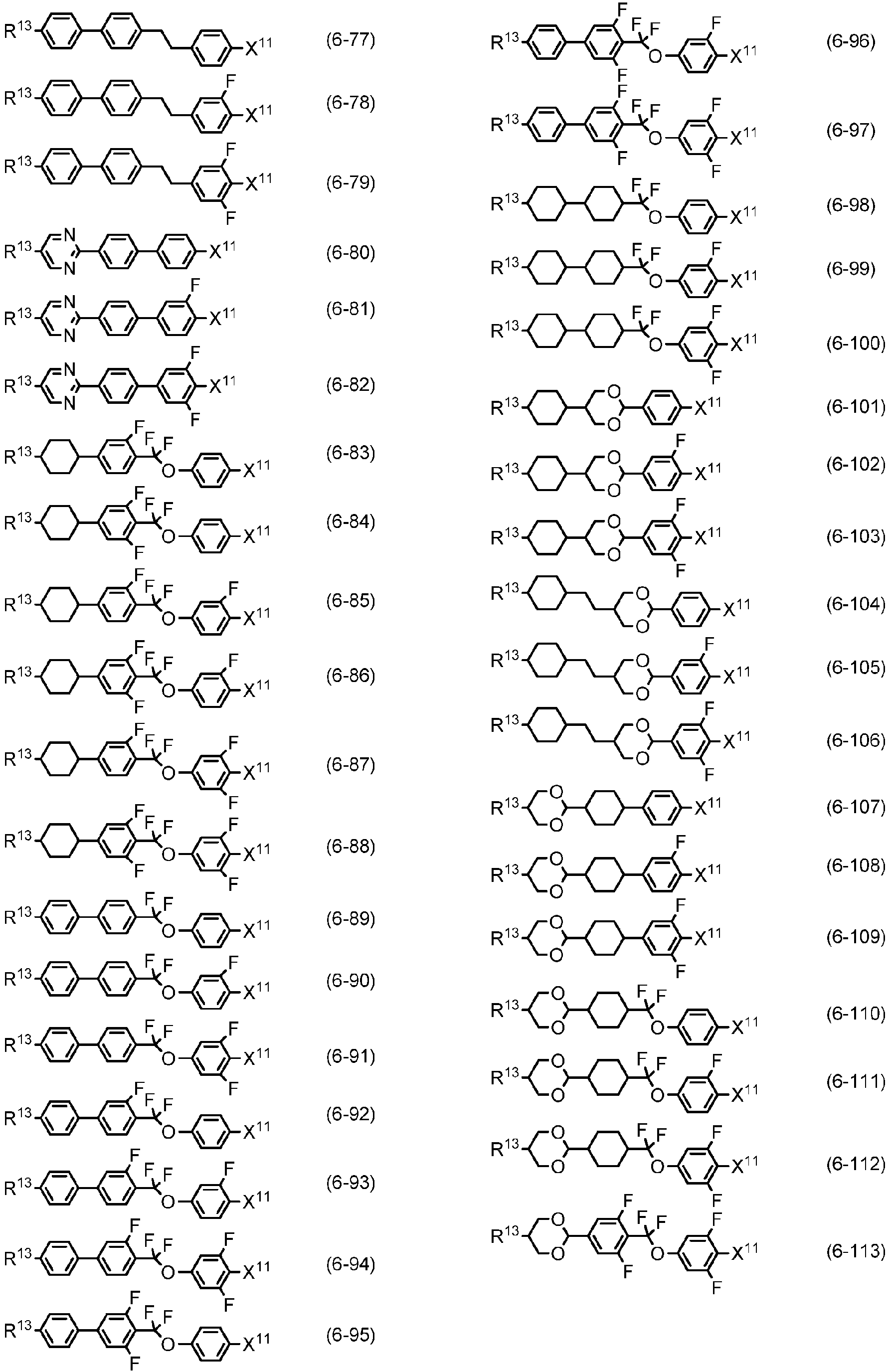

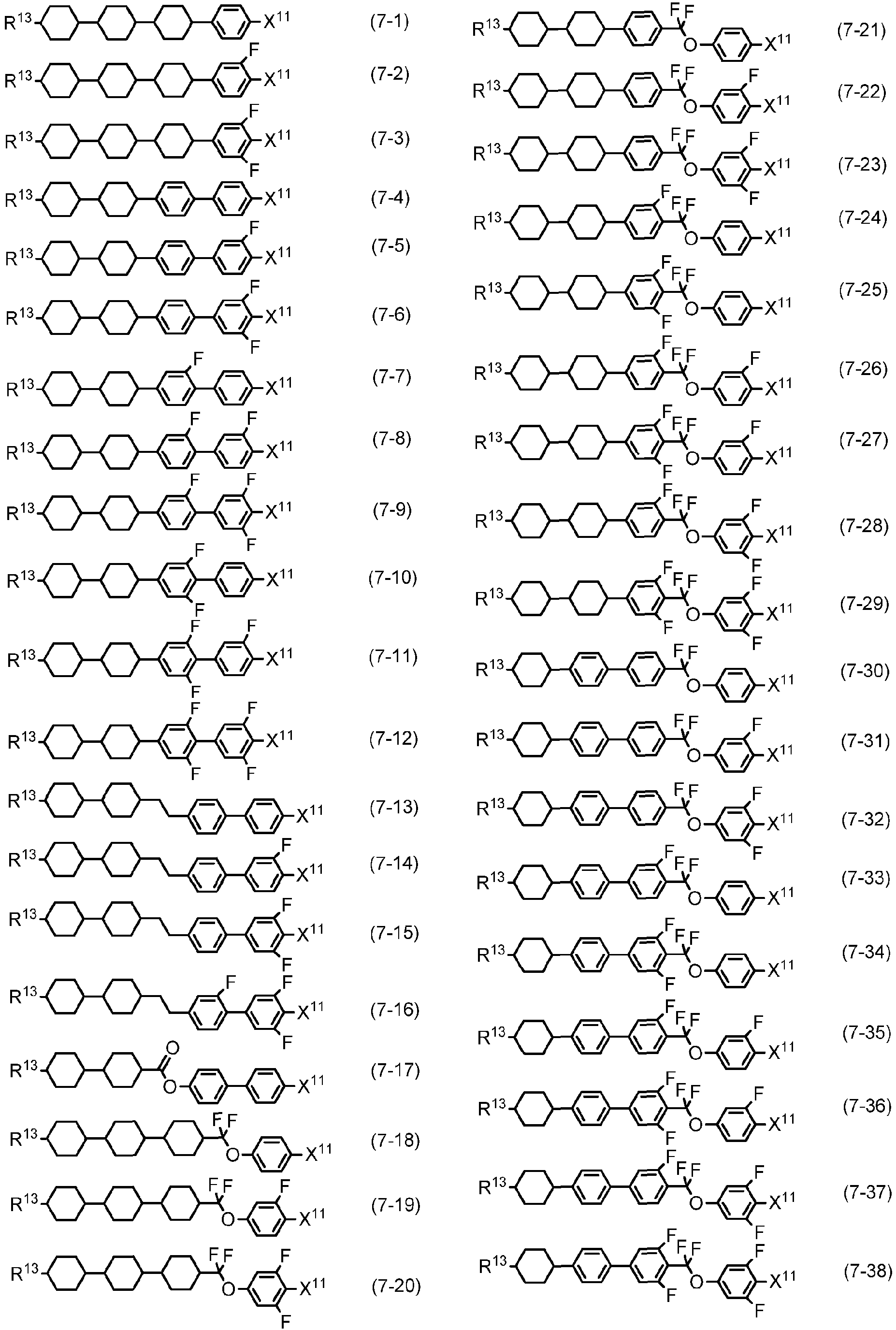

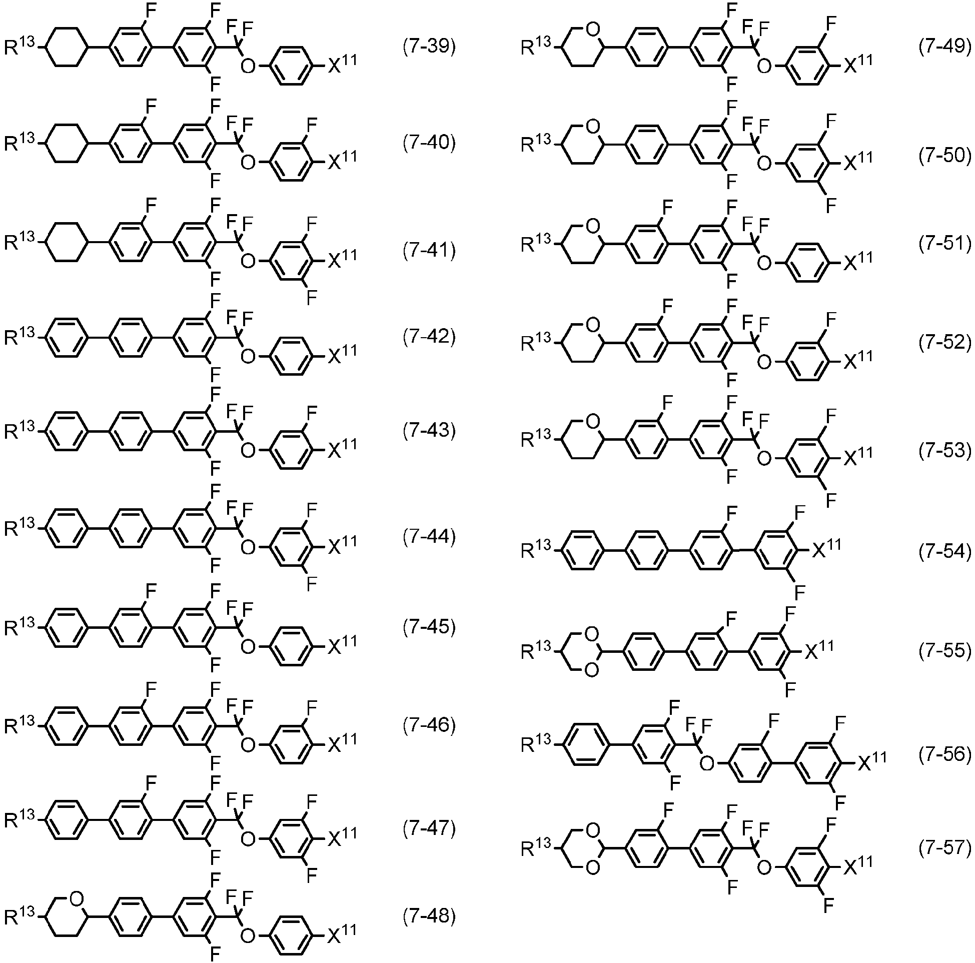

Component C has positive dielectric anisotropy, and superb stability to heat, light or the like, and therefore is used when a composition for the IPS mode, the FFS mode, the OCB mode or the like is prepared. A content of component C is suitably in the range from 1% by weight to 99% by weight, preferably in the range from 10% by weight to 97% by weight, and further preferably in the range from 40% by weight to 95% by weight, based on the weight of the liquid crystal composition. When component (C) is added to the composition having negative dielectric anisotropy, the content of component (C) is preferably 30% by weight or less based on the weight of the liquid crystal composition. Addition of component C allows adjustment of the elastic constant of the composition and adjustment of a voltage-transmittance curve of the device.

Component D is compound (8) in which a right terminal group is -C≡N or -C≡C-C≡N. Preferred examples of component D include compounds (8-1) to (8-64). In a compound of component D, R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹² is -C≡N or -C≡C-C≡N.

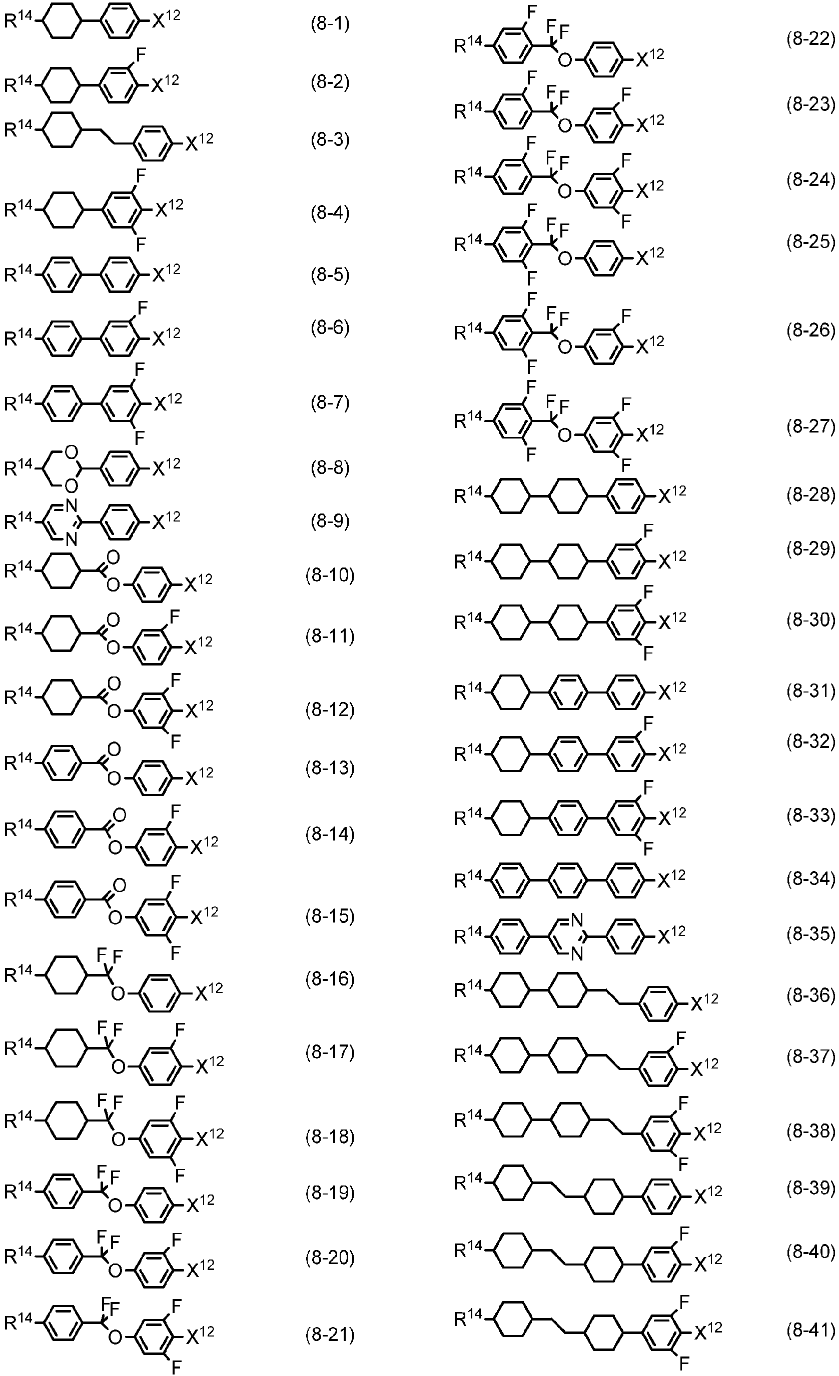

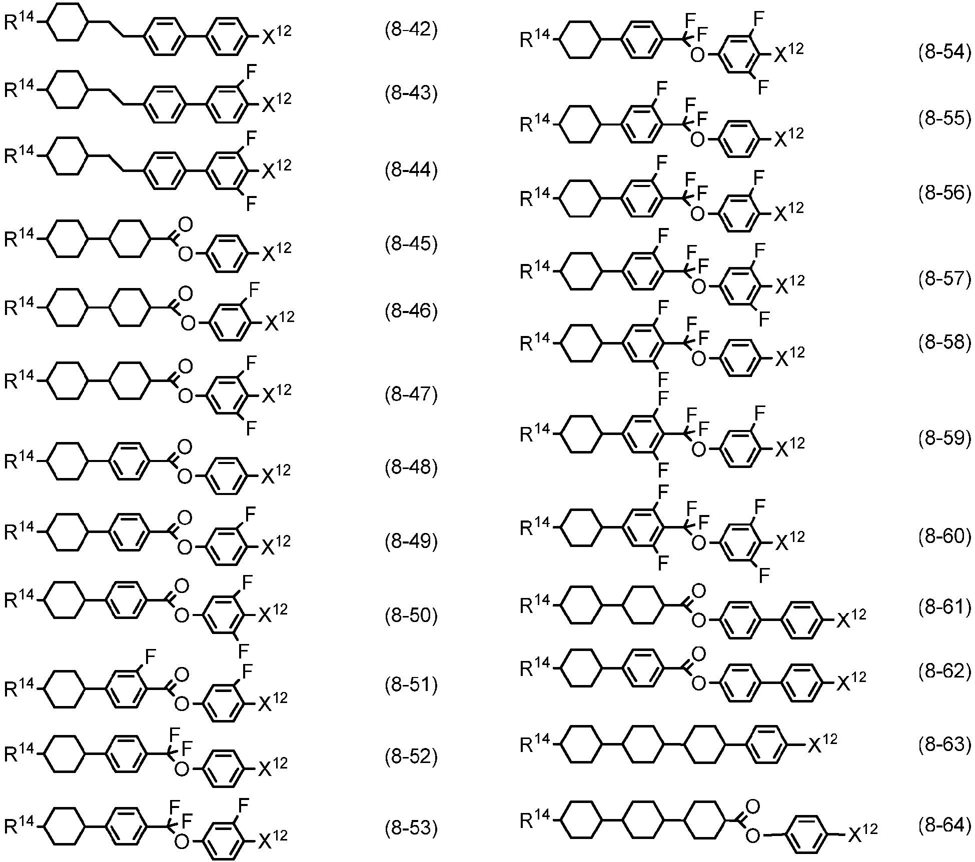

Component D has positive dielectric anisotropy and a value thereof is large, and therefore is mainly used when a composition for the TN mode or the like is prepared. Addition of component D can increase the dielectric anisotropy of the composition. Component D is effective in extending the temperature range of the liquid crystal phase, adjusting the viscosity or adjusting the optical anisotropy. Component D is also useful for adjustment of the voltage-transmittance curve of the device.

When the composition for the TN mode or the like is prepared, a content of component D is suitably in the range from 1% by weight to 99% by weight, preferably in the range from 10% by weight to 97% by weight, and further preferably in the range from 40% by weight to 95% by weight, based on the weight of the liquid crystal composition. When component (D) is added to the composition having negative dielectric anisotropy, the content of component (D) is preferably 30% by weight or less based on the weight of the liquid crystal composition. Addition of component D allows adjustment of the elastic constant of the composition and adjustment of the voltage-transmittance curve of the device.

Component E includes compounds (9) to (15). The compounds have phenylene in which hydrogen in lateral positions are replaced by two halogens, such as 2,3-difluoro-1,4-phenylene. Preferred examples of component E include compounds (9-1) to (9-8), compounds (10-1) to (10-17), compound (11-1), compounds (12-1) to (12-3), compounds (13-1) to (13-11), compounds (14-1) to (14-3) and compounds (15-1) to (15-3). In a compound of component E, R¹⁵ and R¹⁶ are independently alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and R¹⁷ is hydrogen, fluorine, alkyl having 1 to 10 carbons or alkenyl having 2 to 10 carbons, and in the alkyl and the alkenyl, at least one piece of -CH₂- may be replaced by -O-, and at least one hydrogen may be replaced by fluorine.

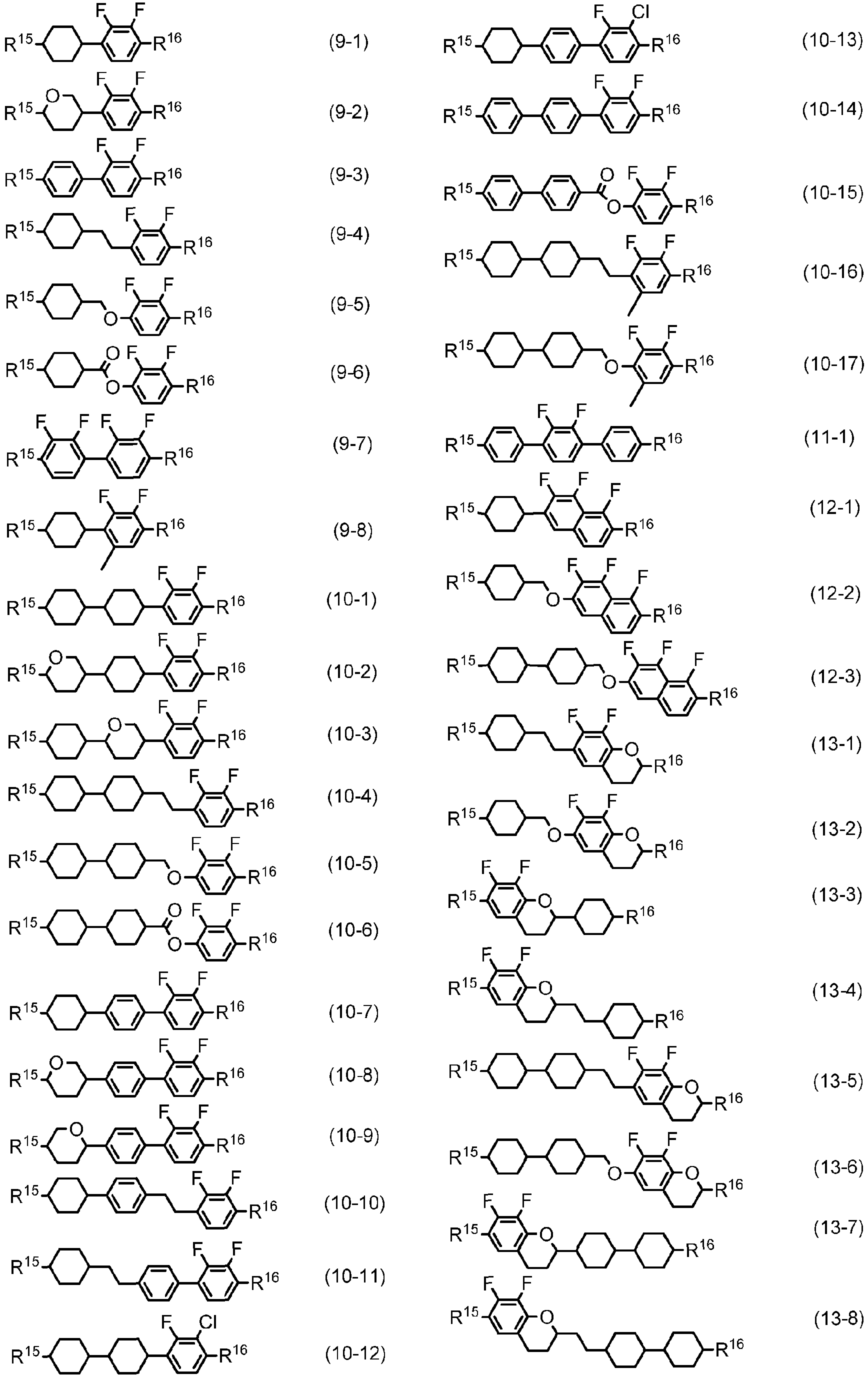

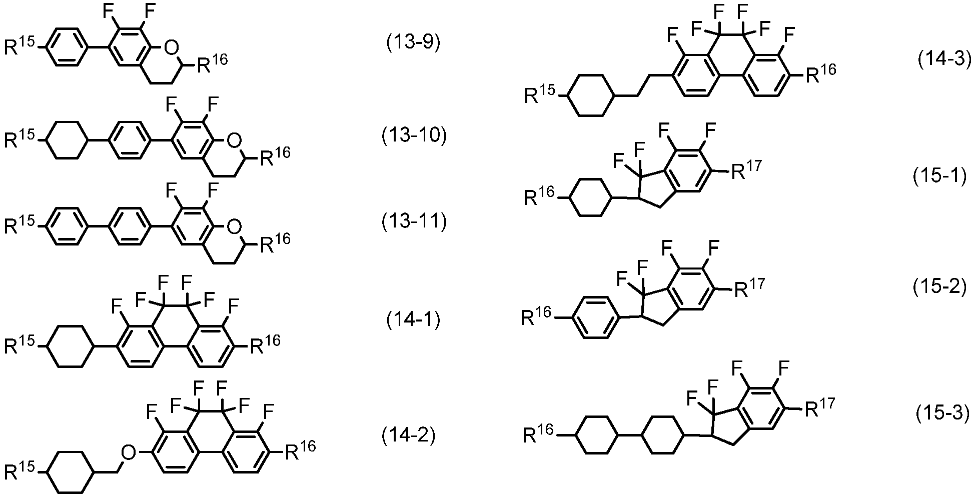

Component E has large negative dielectric anisotropy. Component E is used when a composition for the IPS mode, the VA mode, the PSA mode or the like is prepared. As a content of component E is increased, the dielectric anisotropy of the composition is negatively increased, but the viscosity is increased. Thus, as long as the desired value of threshold voltage of the device is met, the content is preferably as small as possible. When the dielectric anisotropy at a degree of -5 is taken into account, the content is preferably 40% by weight or more in order to allow sufficient voltage driving.

Among types of component E, compound (9) is a bicyclic compound, and therefore is mainly effective in decreasing the viscosity, adjusting the optical anisotropy or increasing the dielectric anisotropy. Compounds (10) and (11) are a tricyclic compound, and therefore are effective in increasing the maximum temperature, increasing the optical anisotropy or increasing the dielectric anisotropy. Compounds (12) to (15) are effective in increasing the dielectric anisotropy.

When a composition for the IPS mode, the VA mode, the PSA mode or the like is prepared, a content of component E is preferably 40% by weight or more, and further preferably in the range from 50% by weight to 95% by weight, based on the weight of the liquid crystal composition. When component E is added to a composition having positive dielectric anisotropy, the content of component E is preferably 30% by weight or less based on the weight of the liquid crystal composition. Addition of component E allows adjustment of the elastic constant of the composition and adjustment of the voltage-transmittance curve of the device.

A liquid crystal composition satisfying at least one of characteristics such as high maximum temperature, low minimum temperature, small viscosity, suitable optical anisotropy, large positive or negative dielectric anisotropy, large specific resistance, high stability to ultraviolet light, high stability to heat and a large elastic constant can be prepared by suitably combining components B, C, D and E as described above. A liquid crystal compound different from components B, C, D and E may be added, when necessary.

### 3-2. Additive

A liquid crystal composition is prepared according to a publicly known method. For example, the component compounds are mixed and dissolved in each other by heating. According to an application, an additive may be added to the composition. Examples of the additive include the polymerizable compound, the polymerization initiator, the polymerization inhibitor, the optically active compound, the antioxidant, the ultraviolet light absorber, the light stabilizer, the heat stabilizer and the antifoaming agent. Such an additive is well known to those skilled in the art, and described in literature.

The polymerizable compound is added for the purpose of forming a polymer in the liquid crystal composition. Compound (1) is polymerized by irradiation with ultraviolet light while voltage is applied between electrodes, and thus the polymer is formed in the liquid crystal composition. On the occasion, compound (1) is immobilized in a state in which the polar group noncovalently interacts with the substrate surface of glass (or metal oxide). Thus, capability of controlling alignment of liquid crystal molecules is further improved, and suitable pretilt can be obtained, and therefore a response time can be shortened.

Preferred examples of the polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy, and a compound having at least one methacryloyloxy. Still further preferred examples also include a compound having both acryloyloxy and methacryloyloxy.

Compound (1) has a polar group. On the other hand, compound (16) has no polar group. Preferred examples of compound (16) will be described.

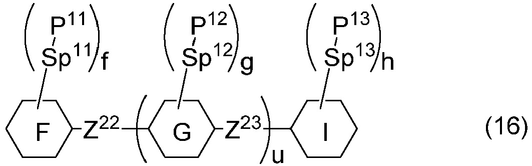

In formula (16), ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring F or ring I is cyclohexyl, cyclohexenyl, phenyl, fluorophenyl, difluorophenyl, 1-naphthyl or 2-naphthyl. Further preferred ring F or ring I is cyclohexyl, cyclohexenyl or phenyl. Particularly preferred ring F or ring I is phenyl.

Ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring G is 1,4-cyclohexylene, 1,4-cyclohexenylene, 1,4-phenylene, 2-fluoro-1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl. Further preferred ring G is 1,4-cyclohexylene, 1,4-cyclohexenylene, 1,4-phenylene or 2-fluoro-1,4-phenylene. Particularly preferred ring G is 1,4-phenylene or 2-fluoro-1,4-phenylene. Most preferred ring G is 1,4-phenylene.

In formula (16), Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine. Preferred Z⁷ or Z⁸ is a single bond, -CH₂CH₂-, -CH₂O-, -OCH₂-, -COO- or -OCO-. Further preferred Z²² or Z²³ is a single bond.

In compound (16), P¹¹, P¹² and P¹³ are independently a polymerizable group. Preferred P¹¹ to P¹³ are a group selected from the group of polymerizable groups represented by formula (P-2) to formula (P-6). Further preferred P¹¹ to P¹³ are a group represented by formula (P-2) or formula (P-6). Particularly preferred P¹¹ to P¹³ are a group represented by formula (P-2). A preferred group represented by formula (P-2) is acryloyloxy (-OCO-CH=CH₂) or methacryloyloxy (-OCO-C (CH₃) =CH₂). A wavy line in group (P-2) to group (P-6) represents a site to form a bonding.

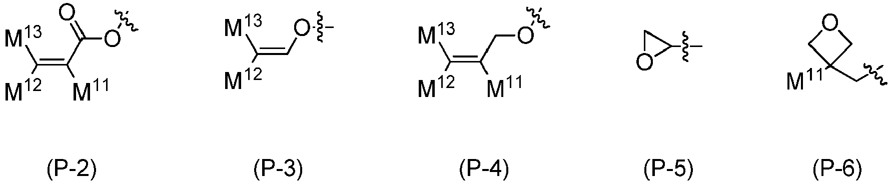

In group (P-2) to group (P-6), M¹¹, M¹² and M¹³ are independently hydrogen, fluorine, alkyl having 1 to 5 carbons, or alkyl having 1 to 5 carbons in which at least one hydrogen is replaced by halogen. Preferred M¹¹, M¹² or M¹³ is hydrogen or methyl for increasing reactivity. Further preferred M¹¹ is hydrogen or methyl, and further preferred M¹² or M¹³ is hydrogen.

In formula (16), Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine. Preferred Sp¹¹, Sp¹² or Sp¹³ is a single bond.

In formula (16), u is 0, 1 or 2. Preferred u is 0 or 1.

Then, f, g and h are independently 0, 1, 2, 3 or 4, and a sum of f, g and h is 1 or more. Preferred f, g or h is 1 or 2. A preferred sum thereof is 2, 3 or 4. A further preferred sum thereof is 2 or 3. A still further preferred sum thereof is 3 or 4.

A further preferred examples include compounds (16-1-1) to (16-16). In compounds (16-1-1) to (16-16), R²⁵ to R³¹ are independently hydrogen or methyl; v and x are independently 0 or 1; t and u are independently an integer from 1 to 10; and L³¹ to L³⁶ are independently hydrogen or fluorine, and L³⁷ and L³⁸ are independently hydrogen, fluorine or methyl.

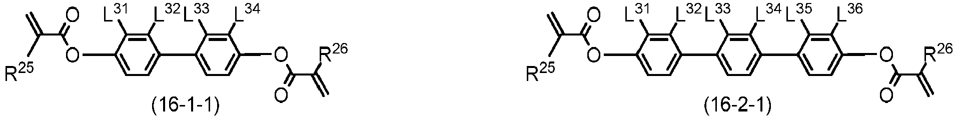

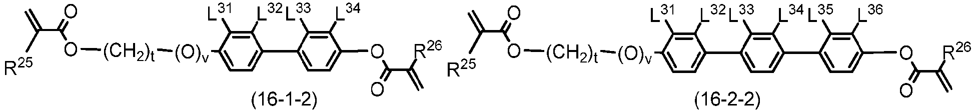

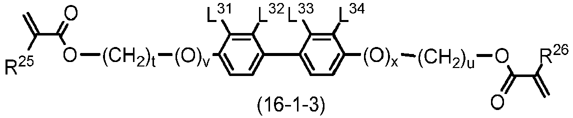

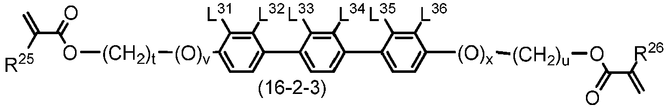

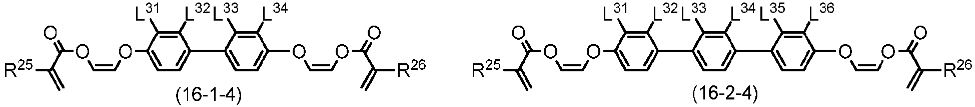

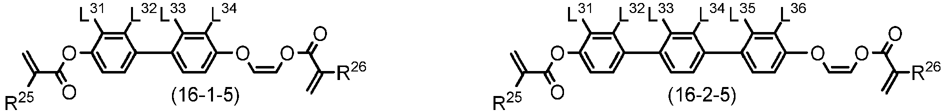

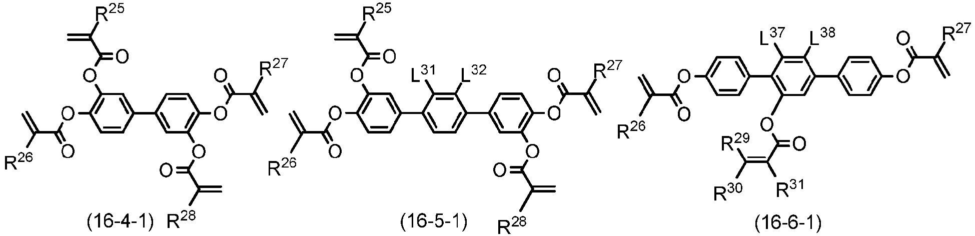

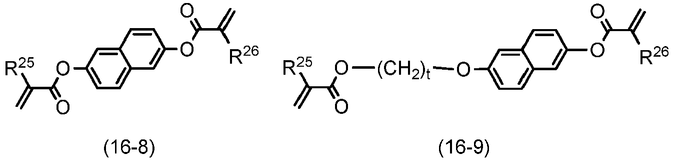

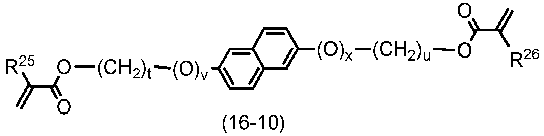

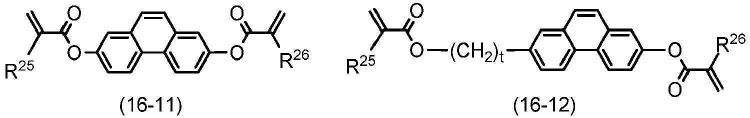

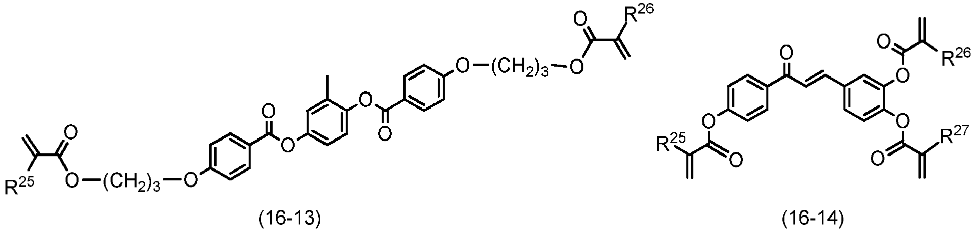

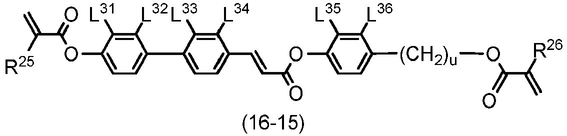

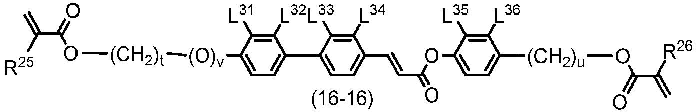

The polymerizable compound can be rapidly polymerized by adding the polymerization initiator. An amount of a remaining polymerizable compound can be reduced by optimizing a reaction temperature. Examples of a photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include
4-methoxyphenyl-2,4-bis(trichloromethyl)triazine,
2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture,
1-(4-isopropylphenyl)-2-hydroxy-2-methylpropane-1-one, benzyl dimethyl ketal,
2-methyl-1-[4-(methylthio)phenyl]-2-morpholinopropane-1-one,
a mixture of 2,4-diethylxanthone and methyl p-dimethylaminobenzoate and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause a poor display such as the image persistence in the device. In order to prevent such an event, photopolymerization may be performed with no addition of the polymerization initiator. A preferred wavelength of light to be irradiated is in the range from 150 nanometers to 500 nanometers. A further preferred wavelength is in the range from 250 nanometers to 450 nanometers, and a most preferred wavelength is in the range from 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Examples of the polymerization inhibitor include hydroquinone, a hydroquinone derivative such as methylhydroquinone, 4-t-butylcatechol, 4-methoxyphenol and phenothiazine.

The optically active compound is effective in inducing a helical structure in liquid crystal molecules to give a required twist angle to prevent a reverse twist. A helical pitch can be adjusted by adding the optically active compound thereto. Two or more optically active compounds may be added for the purpose of adjusting temperature dependence of the helical pitch. Preferred examples of the optically active compound include compounds (Op-1) to (Op-18) described below. In compound (Op-18), ring J is 1,4-cyclohexylene or 1,4-phenylene, and R²⁸ is alkyl having 1 to 10 carbons.

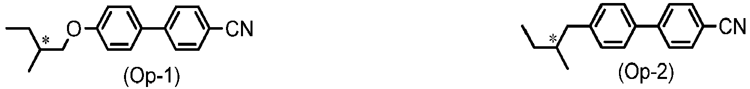

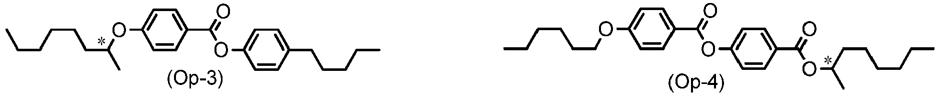

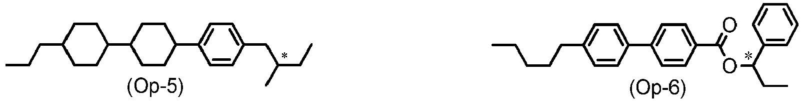

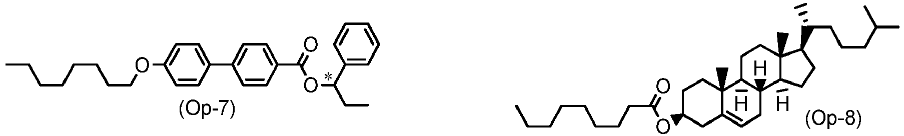

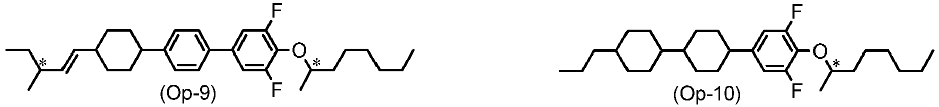

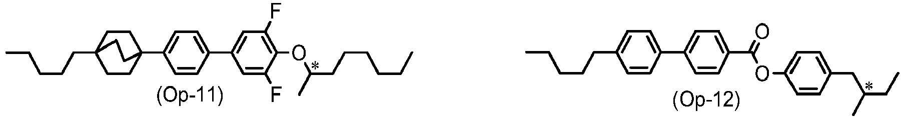

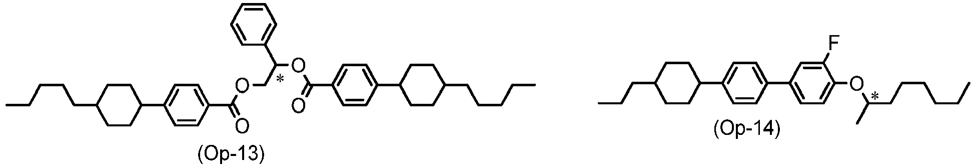

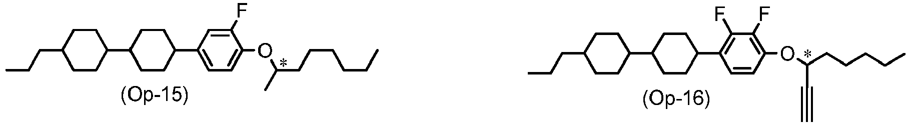

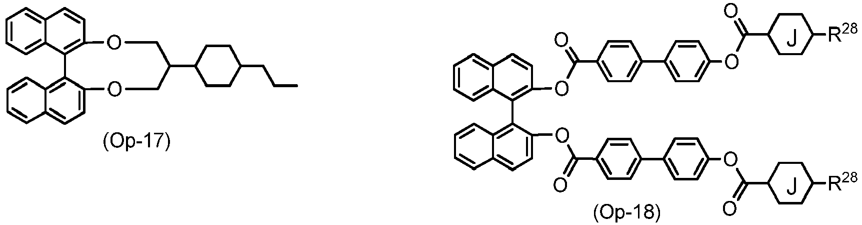

The antioxidant is effective for maintaining a large voltage holding ratio. Preferred examples of the antioxidant include compounds (AO-1) and (AO-2) described below; and Irganox 415, Irganox 565, Irganox 1010, Irganox 1035, Irganox 3114 and Irganox 1098 (trade names; BASF SE). The ultraviolet light absorber is effective for preventing reduction of the maximum temperature. Preferred examples of an ultraviolet light absorber are a benzophenone derivative, a benzoate derivative, a triazole derivative or the like. Specific examples include compounds (AO-3) and (AO-4) described below; Tinuvin 329, Tinuvin P, Tinuvin 326, Tinuvin 234, Tinuvin 213, Tinuvin 400, Tinuvin 328 and Tinuvin 99-2 (trade names; BASF SE); and 1,4-diazabicyclo[2.2.2]octane (DABCO).

The light stabilizer such as an amine having steric hindrance is preferred for maintaining the large voltage holding ratio. Preferred examples of the light stabilizer include compounds (AO-5) and (AO-6) described below; and Tinuvin 144, Tinuvin 765 and Tinuvin 770DF (trade names: BASF SE). The heat stabilizer is also effective for maintaining the large voltage holding ratio, and specific preferred examples thereof include Irgafos 168 (trade name; BASF SE). The antifoaming agent is effective for preventing foam formation. Preferred examples of the antifoaming agent include dimethyl silicone oil and methylphenyl silicone oil.

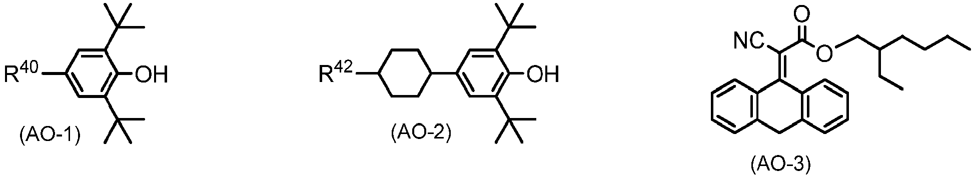

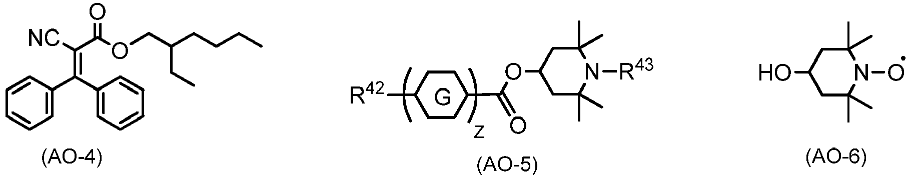

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O˙ (oxygen radical), ring G is 1,4-cyclohexylene or 1,4-phenylene, and z is 1, 2 or 3.

### 4. Liquid crystal display device

The liquid crystal composition can be used in a liquid crystal display device having an operating mode such as the PC mode, the TN mode, the STN mode, the OCB mode and the PSA mode, and driven by an active matrix. The composition can also be used in a liquid crystal display device having the operating mode such as the PC mode, the TN mode, the STN mode, the OCB mode, the VA mode and the IPS mode, and driven by a passive matrix mode. The devices can be applied to any of a reflective type, a transmissive type and a transflective type.

The composition can also be used in a nematic curvilinear aligned phase (NCAP) device prepared by microencapsulating a nematic liquid crystal, and a polymer dispersed liquid crystal display device (PDLCD) and a polymer network liquid crystal display device (PNLCD), in which a three-dimensional network-polymer is formed in the liquid crystal. When an amount of adding the polymerizable compound is about 10% by weight or less based on the weight of the liquid crystal composition, a liquid crystal display device having the PSA mode is prepared. A preferred proportion is in the range from about 0.1% by weight to about 2% by weight. A further preferred proportion is in the range from about 0.2% by weight to about 1.0% by weight. The device having the PSA mode can be driven by a driving mode such as the active matrix mode and the passive matrix mode. Such a device can be applied to any of the reflective type, the transmissive type and the transflective type. A device having a polymer dispersed mode can also be prepared by increasing the amount of adding the polymerizable compound.

In a polymer sustained alignment mode device, a polymer contained in a composition aligns liquid crystal molecules. The polar compound assists alignment of the liquid crystal molecules. More specifically, the polar compound can be used in place of an alignment film. One example of a method of producing such a device is as described below. A device having two substrates referred to as an array substrate and a color filter substrate is prepared. The substrate has no alignment film. At least one of the substrates has an electrode layer. The liquid crystal compound is mixed to prepare the liquid crystal composition. The polymerizable compound and the polar compound are added to the composition. The additive may be further added thereto when necessary. The composition is injected into the device. The device is irradiated with light in a state in which voltage is applied thereto. Ultraviolet light is preferred. The polymerizable compound is polymerized by irradiation with the light. The composition containing a polymer is formed by the polymerization, and thus a device having the PSA mode is prepared.

In the procedure, the polar compound is arranged on a substrate because the polar group interacts with a surface of the substrate. The polar compound aligns liquid crystal molecules. When voltage is applied thereto, alignment of liquid crystal molecules is further promoted by action of an electric field. The polymerizable compound is also aligned according to the alignment. The polymerizable compound is polymerized by ultraviolet light in the above state, and therefore the polymer maintaining the alignment is formed. The alignment of liquid crystal molecules is additionally stabilized by an effect of the polymer, and therefore the response time in the device is shortened. The image persistence is caused due to poor operation in the liquid crystal molecules, and therefore the image persistence is also simultaneously improved by the effect of the polymer. Compound (1) is polymerizable and therefore is consumed by polymerization thereof. Compound (1) is also consumed by copolymerizing with any other polymerizable compound. Accordingly, compound (1) has a polar group, but is consumed, and therefore a liquid crystal display device having a large voltage holding ratio can be obtained.

### Examples

The invention will be described in greater detail by way of Examples (including Synthesis Examples and Use Examples). However, the invention is not limited by the Examples. The invention includes a mixture of a composition in Use Example 1 and a composition in Use Example 2. The invention also includes a mixture prepared by mixing at least two of compositions in the Use Examples.

### 1. Example of compound (1)

Unless otherwise noted, a reaction was performed under a nitrogen atmosphere. Compound (1) was prepared according to procedures shown in Example 1 or the like. The thus prepared compound was identified by methods such as an NMR analysis. Characteristics of compound (1), a liquid crystal compound, a composition and a device were measured by methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sex and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber and a temperature of a detector (FID) part were set to 300°C and 300°C, respectively. A sample was dissolved in acetone and prepared to be a 1 wt% solution, and then 1 microliter of the solution obtained was injected into the sample vaporizing chamber. As a recorder, GC Solution System made by Shimadzu Corporation or the like was used.

HPLC analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set to 254 nanometers. A sample was dissolved in acetonitrile and prepared to be a 0.1 wt% solution, and then 1 microliter of the solution was introduced into a sample chamber. As a recorder, C-R7Aplus made by Shimadzu Corporation was used.

Ultraviolet-Visible spectrophotometry: For measurement, PharmaSpec UV-1700 made by Shimadzu Corporation was used. A detection wavelength was adjusted in the range of 190 nanometers to 700 nanometers. A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), the compound itself was used as a sample.

Measuring method: Characteristics were measured according to the methods described below. Most of the measuring methods are applied as described in a JEITA Standard (JEITA ED-2521B) discussed and established by Japan Electronics and Information Technology Industries Association (JEITA), or modified thereon. No thin film transistor (TFT) was attached to a TN device used for measurement.

### (1) Phase structure

A sample was placed on a hot plate in a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.

### (2) Transition temperature (°C)

For measurement, a differential scanning calorimeter, Diamond DSC System, made by PerkinElmer, Inc., or a high sensitivity differential scanning calorimeter, X-DSC7000, made by SSI NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to liquid may be occasionally abbreviated as "clearing point."

A crystal was expressed as C. When kinds of the crystals were distinguishable, each of the crystals was expressed as C₁ or C₂. The smectic phase or the nematic phase was expressed as S or N. When smectic A phase, smectic B phase, smectic C phase or smectic F phase was distinguishable among the smectic phases, the phases were expressed as S_{A}, S_{B}, S_{C} or S_{F}, respectively. A liquid (isotropic) was expressed as I. A transition temperature was expressed as "C 50.0 N 100.0 I," for example. The expression indicates that a transition temperature from the crystals to the nematic phase is 50.0°C, and a transition temperature from the nematic phase to the liquid is 100.0°C.

### (3) Maximum temperature of nematic phase (T_{NI} or NI; °C)

A sample was placed on a hot plate in a melting point apparatus equipped with a polarizing microscope, and heated at a rate of 1°C per minute. Temperature when part of the sample began to change from a nematic phase to an isotropic liquid was measured. A maximum temperature of the nematic phase may be occasionally abbreviated as "maximum temperature." When the sample was a mixture of compound (1) and a base liquid crystal, the maximum temperature was expressed in terms of a symbol T_{NI}. When the sample was a mixture of compound (1) and a compound such as components B, C and D, the maximum temperature was expressed in terms of a symbol NI.

### (4) Minimum temperature of nematic phase (T_{C}; °C)

Samples each having a nematic phase were kept in freezers at temperatures of 0°C, -10°C, -20°C, -30°C and -40°C for 10 days, and then liquid crystal phases were observed. For example, when the sample was maintained in the nematic phase at -20°C and changed to crystals or a smectic phase at -30°C, Tc was expressed as Tc ≤ -20°C. A minimum temperature of the nematic phase may be occasionally abbreviated as "minimum temperature."

### (5) Viscosity (bulk viscosity; η; measured at 20°C; mPa·s)

For measurement, an E type rotational viscometer made by Tokyo Keiki Inc. was used.

### (6) Optical anisotropy (refractive index anisotropy; measured at 25°C; Δn)

Measurement was carried out by an Abbe refractometer with a polarizing plate mounted on an ocular, using light at a wavelength of 589 nanometers . A surface of a main prism was rubbed in one direction, and then a sample was added dropwise onto the main prism. A refractive index (n∥) was measured when a direction of polarized light was parallel to a direction of rubbing. A refractive index (n⊥) was measured when the direction of polarized light was perpendicular to the direction of rubbing. A value of optical anisotropy (Δn) was calculated from an equation: Δn = n∥ - n⊥.

### (7) Specific resistance (ρ; measured at 25°C; Qcm)

Into a vessel equipped with electrodes, 1.0 milliliter of sample was injected. A direct current voltage (10 V) was applied to the vessel, and a direct current after 10 seconds was measured. Specific resistance was calculated from the following equation: $\left(specific resistance\right) = \left\{\left(voltage\right)\times \left(electric capacity of a vessel\right)\right\} / \left\{\left(direct current\right)\times \left(dielectric constant of vacuum\right)\right\} .$

The measuring method of the characteristics may be different between a sample having positive dielectric anisotropy and a sample having negative dielectric anisotropy. When the dielectric anisotropy was positive, the measuring methods were described in sections (8a) to (12a). When the dielectric anisotropy was negative, the measuring methods were described in sections (8b) to (12b).

### (8a) Viscosity (rotational viscosity; γ1; measured at 25°C; mPa·s)

Positive dielectric anisotropy: Measurement was carried out according to a method described in M. Imai et al., Molecular Crystals and Liquid Crystals, Vol. 259, p. 37 (1995) . A sample was put in a TN device in which a twist angle was 0 degrees, and a distance (cell gap) between two glass substrates was 5 micrometers. Voltage was applied stepwise to the device in the range of 16 V to 19.5 V at an increment of 0.5 V. After a period of 0.2 second with no voltage application, voltage was repeatedly applied under conditions of only one rectangular wave (rectangular pulse; 0.2 second) and no voltage application (2 seconds). A peak current and a peak time of transient current generated by the applied voltage were measured. A value of rotational viscosity was obtained from the measured values and calculation equation (8) on page 40 of the paper presented by M. Imai et al. A value of dielectric anisotropy required for the calculation was determined using the device by which the rotational viscosity was measured and by a method described below.

### (8b) Viscosity (rotational viscosity; γ1; measured at 25°C; mPa·s)

Negative dielectric anisotropy: Measurement was carried out according to the method described in M. Imai et al., Molecular Crystals and Liquid Crystals, Vol. 259, p. 37 (1995) . A sample was put in a VA device in which a distance (cell gap) between two glass substrates was 20 micrometers. Voltage was applied stepwise to the device in the range of 39 V to 50 V at an increment of 1 V. After a period of 0.2 second with no voltage application, voltage was repeatedly applied under conditions of only one rectangular wave (rectangular pulse; 0.2 second) and no voltage application (2 seconds). A peak current and a peak time of transient current generated by the applied voltage were measured. A value of rotational viscosity was obtained from the measured values and calculation equation (8) on page 40 of the paper presented by M. Imai et al. In dielectric anisotropy required for the calculation, a value measured according to items of dielectric anisotropy described below was used.

### (9a) Dielectric anisotropy (Δε; measured at 25°C)

Positive dielectric anisotropy: A sample was put in a TN device in which a distance (cell gap) between two glass substrates was 9 micrometers and a twist angle was 80 degrees. Sine waves (10 V, 1 kHz) were applied to the device, and after 2 seconds, a dielectric constant (ε∥) of liquid crystal molecules in a major axis direction was measured. Sine waves (0.5 V, 1 kHz) were applied to the device, and after 2 seconds, a dielectric constant (ε⊥) of liquid crystal molecules in a minor axis direction was measured. A value of dielectric anisotropy was calculated from an equation: Δε = ε∥ - ε⊥.

### (9b) Dielectric anisotropy (Δε; measured at 25°C)

Negative dielectric anisotropy: A value of dielectric anisotropy was calculated from an equation: Δε = ε∥ - ε⊥. A dielectric constant (ε∥ and ε⊥) was measured as described below.
(1) Measurement of dielectric constant (ε∥): An ethanol (20 mL) solution of octadecyltriethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive . Sine waves (0.5 V, 1 kHz) were applied to the device, and after 2 seconds, a dielectric constant (ε∥) of liquid crystal molecules in a major axis direction was measured.
(2) Measurement of dielectric constant (ε⊥): A polyimide solution was applied to a well-cleaned glass substrate. After calcining the glass substrate, rubbing treatment was applied to the alignment film obtained. A sample was put in a TN device in which a distance (cell gap) between two glass substrates was 9 micrometers and a twist angle was 80 degrees. Sine waves (0.5 V, 1 kHz) were applied to the device, and after 2 seconds, a dielectric constant (ε⊥) of liquid crystal molecules in a minor axis direction was measured.

### (10a) Elastic constant (K; measured at 25°C; pN)

Positive dielectric anisotropy: For measurement, HP4284A LCR Meter made by Yokogawa-Hewlett-Packard Co. was used. A sample was put in a horizontal alignment device in which a distance (cell gap) between two glass substrates was 20 micrometers. An electric charge of 0 V to 20 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook (Ekisho Debaisu Handobukku in Japanese; Nikkan Kogyo Shimbun, Ltd.)," and values of K₁₁ and K₃₃ were obtained from equation (2.99) . Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.

### (10b) Elastic constant (K₁₁ and K₃₃; measured at 25°C; pN)

Negative dielectric anisotropy: For measurement, Elastic Constant Measurement System Model EC-1 made by TOYO Corporation was used. A sample was put in a vertical alignment device in which a distance (cell gap) between two glass substrates was 20 micrometers. An electric charge of 20 V to 0 V was applied to the device, and electrostatic capacity and applied voltage were measured. Values of electrostatic capacity (C) and applied voltage (V) were fitted to equation (2.98) and equation (2.101) on page 75 of "Liquid Crystal Device Handbook (Ekisho Debaisu Handobukku in Japanese; Nikkan Kogyo Shimbun, Ltd.)," and a value of elastic constant was obtained from equation (2.100).

### (11a) Threshold voltage (Vth; measured at 25°C; V)

Positive dielectric anisotropy: For measurement, an LCD-5100 luminance meter made by Otsuka Electronics Co., Ltd. was used. A light source was a halogen lamp. A sample was put in a normally white mode TN device in which a distance (cell gap) between two glass substrates was 0.45/Δn (µm) and a twist angle was 80 degrees. A voltage (32 Hz, rectangular waves) to be applied to the device was stepwise increased from 0 V to 10 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of voltage at 90% transmittance.

### (11b) Threshold voltage (Vth; measured at 25°C; V)

Negative dielectric anisotropy: For measurement, an LCD-5100 luminance meter made by Otsuka Electronics Co., Ltd. was used. A light source was a halogen lamp. A sample was put in a normally black mode VA device in which a distance (cell gap) between two glass substrates was 4 micrometers and a rubbing direction was anti-parallel, and the device was sealed with an ultraviolet-curable adhesive. A voltage (60 Hz, rectangular waves) to be applied to the device was stepwise increased from 0 V to 20 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of voltage at 10% transmittance.

### (12a) Response time (τ; measured at 25°C; ms)

Positive dielectric anisotropy: For measurement, an LCD-5100 luminance meter made by Otsuka Electronics Co., Ltd. was used. A light source was a halogen lamp. A low-pass filter was set to 5 kHz. A sample was put in a normally white mode TN device in which a distance (cell gap) between two glass substrates was 5.0 micrometers and a twist angle was 80 degrees. A voltage (rectangular waves; 60 Hz, 5 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. Arise time (τr; millisecond) was expressed in terms of time required for a change from 90% transmittance to 10% transmittance. A fall time (τf; millisecond) was expressed in terms of time required for a change from 10% transmittance to 90% transmittance. A response time was expressed by a sum of the rise time and the fall time thus determined.

### (12b) Response time (τ; measured at 25°C; ms)

Negative dielectric anisotropy: For measurement, an LCD-5100 luminance meter made by Otsuka Electronics Co., Ltd. was used. A light source was a halogen lamp. A low-pass filter was set to 5 kHz. A sample was put in a normally black mode PVA device in which a distance (cell gap) between two glass substrates was 3.2 micrometers, and a rubbing direction was anti-parallel. The device was sealed with an ultraviolet-curable adhesive. The device was applied with a voltage of a little exceeding a threshold voltage for 1 minute, and then was irradiated with ultraviolet light of 23.5 mW/cm² for 8 minutes, while applying a voltage of 5.6 V. A voltage (rectangular waves; 60 Hz, 10 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A response time was expressed in terms of time required for a change from 90% transmittance to 10% transmittance (fall time; millisecond).

### (13) Voltage holding ratio

A polymerizable compound was polymerized by irradiating a device with ultraviolet light using Black Light F40T10/BL (a peak wavelength of 369 nm) made by Eye graphics Co. Ltd. A pulse voltage (60 microseconds at 1 V) was applied to the device at 60°C and the device was charged. A decaying voltage was measured for 1.67 seconds by a high-speed voltmeter, and area A between a voltage curve and a horizontal axis in a unit cycle was determined. Area B is an area without decay. A voltage holding ratio is expressed in terms of a percentage of area A to area B.

### Raw material

Solmix (registered trademark) A-11 is a mixture of ethanol (85.5%), methanol (13.4%) and isopropanol (1.1%), and was purchased from Japan Alcohol Trading Co., Ltd.

### Synthesis Example 1

### Synthesis of compound (T-4)

The compound was used in the seventh step in Synthesis Example 1 and in the sixth step in Synthesis Example 2.

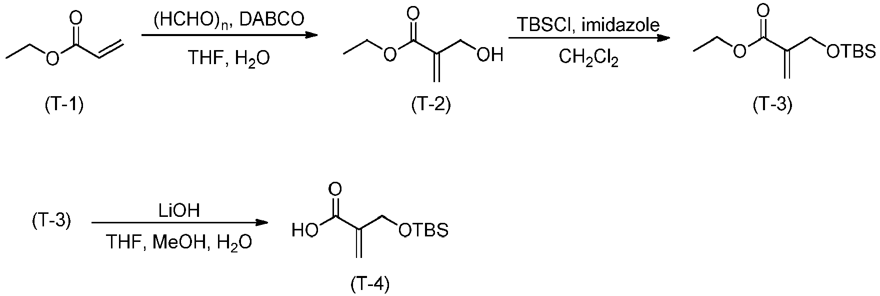

### First step

Paraformaldehyde (60.0 g), DABCO (56.0 g) and water (200 mL) were put in a reaction vessel, and the resulting mixture was stirred at room temperature for 15 minutes. A THF solution (400 mL) of compound (T-1) (50.0 g) was added dropwise thereto, and the resulting mixture was stirred at room temperature for 72 hours . A reaction mixture was poured into water, and an aqueous layer thereof was subjected to extraction with ethyl acetate. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 2 : 1) to obtain compound (T-2) (44.1 g; 68%).

### Second step

Compound (T-2) (44.1 g), imidazole (25.0 g) and dichloromethane (400 mL) were put in a reaction vessel, and the resulting mixture was cooled to 0°C. A dichloromethane solution (200 mL) of t-buthyldimethyl chlorosilane (TBSCl; 56.1 g) was added dropwise thereto, and the resulting mixture was stirred for 4 hours while warming to room temperature. A reaction mixture was poured into water, and an aqueous layer thereof was subjected to extraction with dichloromethane. A combined organic layer was washed with water, and dried over anhydrous magnesium sulfate. The obtained solution was concentrated under reduced pressure, and the resulting residue was purified by silica gel chromatography (a volume ratio, heptane : ethyl acetate = 9 : 1) to obtain compound (T-3) (69.6 g; 84%).

### Third step

Compound (T-3) (69.6 g), THF (600 mL), methanol (150 mL) and water (100 mL) were put in a reaction vessel, and the resulting mixture was cooled to 0°C. Lithium hydroxide monohydrate (23.9 g) was added thereto, and the resulting mixture was stirred for 12 hours while returning to room temperature. A reaction mixture was poured into water, and 6 N hydrochloric acid (20 mL) was slowly added thereto to becoming acidic atmosphere, and then an aqueous layer thereof was subjected to extraction with ethyl acetate. A combined organic layer was washed with water, and dried over anhydrous magnesium sulfate. The obtained solution was concentrated under reduced pressure to obtain compound (T-4) (21.6 g; 35%).

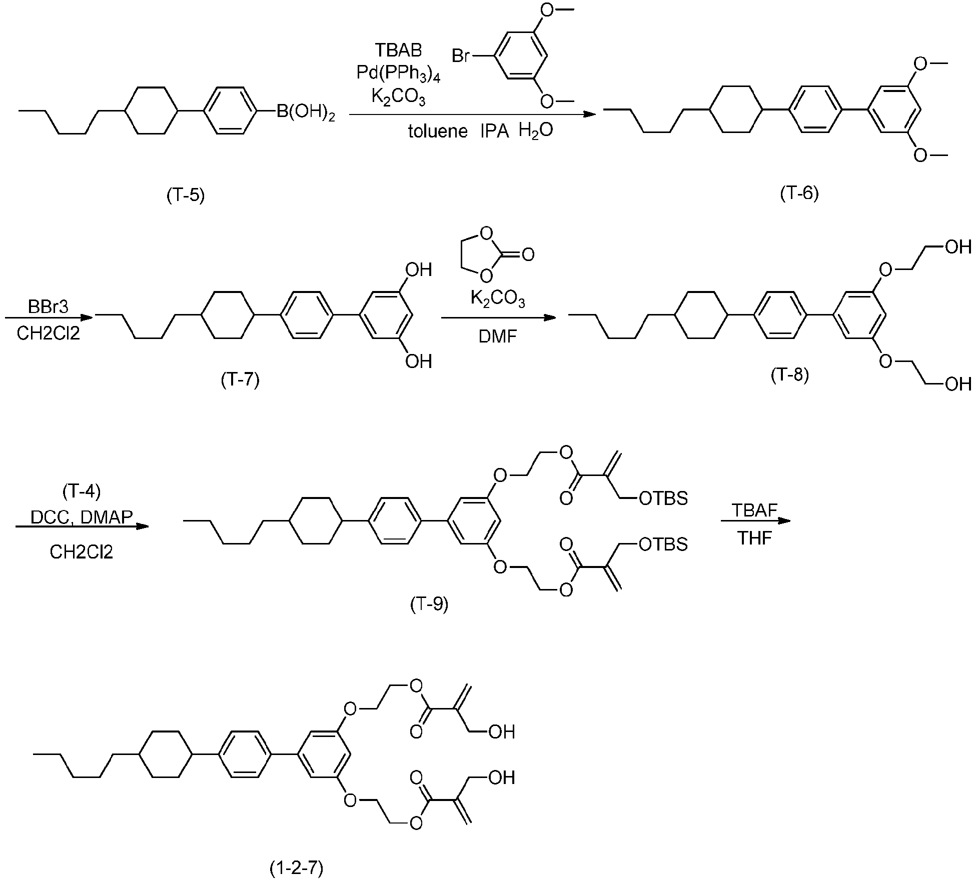

### Fourth step

Compound (T-5) was prepared in accordance with a conventional method. Compound (T-5) (7.5 g), tetrakis(triphenylphosphine)palladium (1.3 g), tetrabutylammonium bromide (TBAB) (1.5 g), potassium carbonate (6.4 g), 1-bromo-3,5-dimethoxybenzene (5 g), toluene (200 mL), 2-propanol (IPA) (80 mL) and pure water (20 mL) were put in a reaction vessel, and the resulting mixture was stirred at 90°C. A reaction mixture was poured into water, and an aqueous layer thereof was subjected to extraction with toluene. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 9 : 1), and further purified by recrystallization from a mixed solvent of heptane and toluene (a volume ratio, 1 : 1) to obtain compound (T-6) (7.18 g; 85%).

### Fifth step

Compound (T-6) (7.18 g) and dichloromethane (200 mL) were put in a reaction vessel, and the resulting mixture was cooled to -50°C while stirring thereof. Then, boron tribromide (2.1 mL) was added dropwise thereto. The resulting mixture was stirred for 5 hours while warming to room temperature. A reaction mixture was poured into water, and an aqueous layer thereof was subjected to extraction with dichloromethane. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 1 : 1) to obtain compound (T-7) (5.3 g; 80%).

### Sixth step

Compound (T-7) (5.3 g), ethylene carbonate (3.0 g), potassium carbonate (6.5 g) and DMF (200 mL) were put in a reaction vessel, and the resulting mixture was stirred at 100°C. A reaction mixture was poured into water, and an aqueous layer thereof was subjected to extraction with ethyl acetate. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 1 : 1) to obtain compound (T-8) (5.5 g; 83%).

### Seventh step

Compound (T-8) (5.3 g), compound (T-4) (5.9 g), DMAP (1.52 g) and dichloromethane (150 mL) were put in a reaction vessel, and the resulting mixture was cooled to 0°C while stirring thereof. A dichloromethane solution (50 mL) of DCC (7.7 g) was added dropwise thereto. The resulting mixture was stirred for 5 hours while warming to room temperature. A reaction mixture was poured into water, and an aqueous layer thereof was subjected to extraction with dichloromethane. A combined organic layer was washed with water, and dried over anhydrous magnesium sulfate. The obtained solution was concentrated under reduced pressure, and the resulting residue was purified by silica gel chromatography (a volume ratio, toluene : heptane = 1 : 1) to obtain compound (T-9) (8.3 g; 81%).

### Eighth step

Compound (T-9) (8.3 g) and THF (100 mL) were put in a reaction vessel, and the resulting mixture was cooled to 0°C while stirring thereof. TBAF (2.9 g) was added dropwise thereto, and the resulting mixture was stirred for 3 hours while waring to room temperature. A reaction mixture was poured into water, and an aqueous layer thereof was subjected to extraction with ethyl acetate. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 1 : 1), and further purified by recrystallization from heptane to obtain compound (1-2-7) (4.5 g; 75%).

NMR analytical values of the obtained compound (1-2-7) were as described below.
¹H-NMR : Chemical shift δ (ppm; CDCl₃): 7.48 - 7.46 (m, 2H), 7.27 - 7.26 (m, 2H), 6.75 (d, J 2.3 Hz, 2H), 6.47 - 6.46 (m, 1H), 6.30 (s, 2H), 5.86 (d, J = 1.1 Hz, 2H), 4.54 (t, J = 4.4 Hz, 4H), 4.33 (s, 4H), 4.27 - 4.25 (m, 4H), 2.52 - 2.47 (m, 1H), 2.34 (s, 2H), 1.90 (t, J = 14 Hz, 4H), 1.51 - 1.44 (m, 2H), 1.35 - 1.20 (m, 9H), 1.09 - 1.02 (m, 2H), 0.90 (t, J = 6.9 Hz, 3H).

Characteristics of compound (No. 1-2-7) were as described below.
Transition temperature: C 58.8 I.

### Synthesis example 2

### Synthesis of compound (No. 1-5-2)

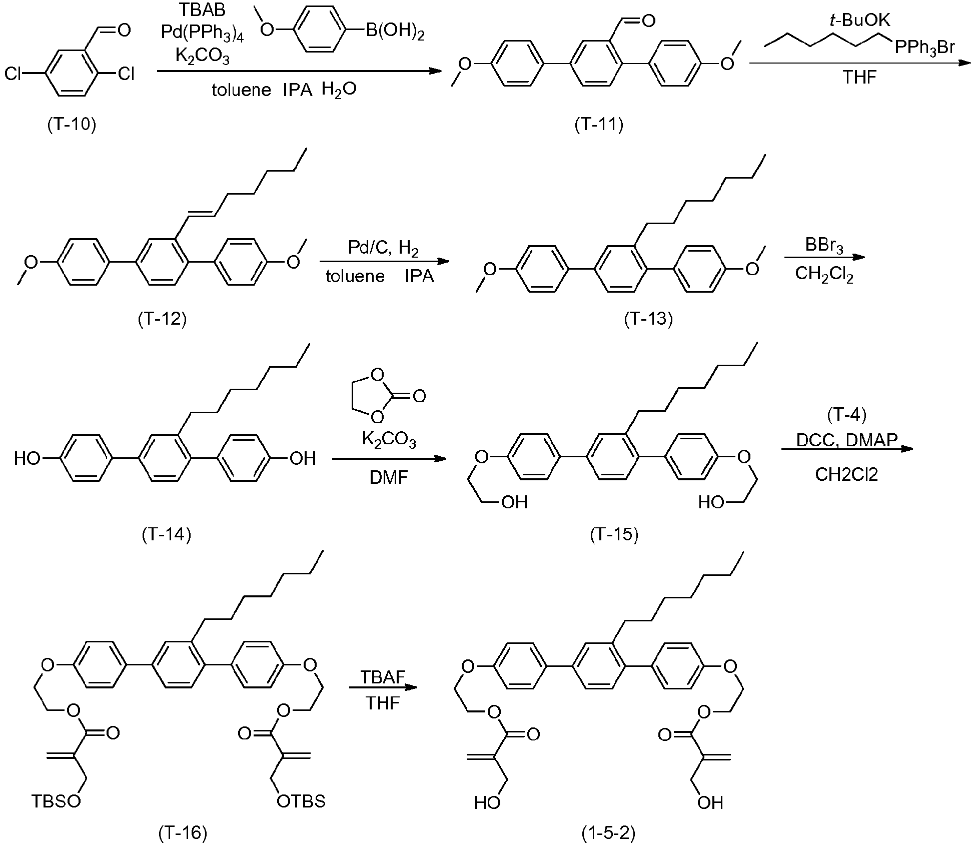

### First step

Compound (T-10) (10.0 g), 4-methoxyphenylboronic acid (19.1 g), tetrakis(triphenylphosphine)palladium (1.9 g), potassium carbonate (15.8 g), TBAB (3.7 g), toluene (200 mL), IPA (80 mL) and pure water (20 mL) were put in a reaction vessel, and the resulting mixture was stirred at 90°C. A reaction mixture was poured into water, and an aqueous layer thereof was subjected to extraction with toluene. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 9 : 1), and further purified by recrystallization from a mixed solvent of heptane and toluene (a volume ratio, 1 : 1) to obtain compound (T-11) (14.9 g; 82%) .

### Second step

Hexyltriphenylphosphonium bromide (22.0 g) and THF (100 mL) were put in a reaction vessel, and the resulting mixture was stirred while cooling to -30°C. Then, t-butoxide potassium (5.7 g) was put in thereto, and the resulting mixture was stirred at -30°C for 1 hour. A THF solution (100 mL) of compound (T-11) (14.9 g) was added dropwise thereto, and the resulting mixture was stirred for 4 hours while waring to room temperature. A reaction mixture was poured into water, and an aqueous layer thereof was subjected to extraction with toluene. A combined organic layer was washed with water, and dried over anhydrous magnesium sulfate. The obtained solution was concentrated under reduced pressure, and the resulting residue was purified by silica gel chromatography (toluene) to obtain compound (T-12) (16.2 g; 90%).

### Third step

Compound (T-12) (16.2 g), Pd/C (0.2 g), toluene (100 mL) and IPA (100 mL) were put in a reaction vessel, and the resulting mixture was stirred for 10 hours under hydrogen atmosphere. A reaction mixture was poured into water, and an aqueous layer thereof was subjected to extraction with toluene. A combined organic layer was washed with water, and dried over anhydrous magnesium sulfate. The obtained solution was concentrated under reduced pressure, and the resulting residue was purified by silica gel chromatography (toluene) to obtain compound (T-13) (15.5 g; 95%) .

### Fourth step

Compound (T-13) (15.5 g) and dichloromethane (200 mL) were put in a reaction vessel, and the resulting mixture was stirred while cooling to -50°C. Boron tribromide (22.0 g) was added dropwise thereto, and the resulting mixture was stirred for 5 hours while waring to room temperature. A reaction mixture was poured into water, and an aqueous layer thereof was subjected to extraction with toluene. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 8 : 2) to obtain compound (T-14) (13.0 g; 90%)

### Fifth step

Compound (T-14) (13. 0g), ethylene carbonate (9.5 g), potassium carbonate (15.0 g) and DMF (200 mL) were put in a reaction vessel, and the resulting mixture was stirred at 100°C. A reaction mixture was poured into water, and an aqueous layer thereof was subjected to extraction with ethyl acetate. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 8 : 2) to obtain compound (T-15) (13.6 g; 84%).

### Sixth step

Compound (T-15) (13.6g), compound (T-4) (14.4 g), DMAP (1.85 g) and dichloromethane (350 mL) were put in a reaction vessel, and the resulting mixture was stirred while cooling to 0°C. A dichloromethane solution (150 mL) of DCC (18.8 g) was added dropwise thereto. The resulting mixture was stirred for 5 hours while warming to room temperature. A reaction mixture was poured into water, and an aqueous layer thereof was subjected to extraction with dichloromethane. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 9 : 1) to obtain compound (T-9) (19.2 g; 75%)

### Seventh step

Compound (T-9) (19.2 g) and THF (200 mL) were put in a reaction vessel, and the resulting mixture was stirred while cooling to 0°C. TBAF (6.5 g) was added dropwise thereto, and the resulting mixture was stirred for 3 hours while warming to room temperature. A reaction mixture was poured into water, and an aqueous layer thereof was subjected to extraction with ethyl acetate. A combined organic layer was washed with water, and dried over anhydrous magnesium sulfate. The obtained solution was concentrated under reduced pressure, and the resulting residue was purified by silica gel chromatography (a volume ratio, toluene : ethyl acetate = 1 : 1), and further purified by recrystallization from heptane to obtain compound (No. 1-5-2) (9.8; 70%)

NMR analytical values of the obtained compound (1-5-2) were as described below.
¹H-NMR : Chemical shift δ (ppm; CDCl₃): 7.65 - 7.55 (m, 2H), 7.45 (d, J = 1.6 Hz, 1H), 7.39 (dd, J = 7.8 Hz, J = 1.8 Hz, 1H), 7.25 - 7.22 (m, 3H), 7.13 (d, J = 8.6 Hz, 2H), 6.98 - 6.93 (m, 4H), 6.84 (d, J = 8.7 Hz, 2H), 6.29 (s, 1H), 5.85 (d, J = 1.2 Hz, 1H), 4.52 (t, J = 4.8 Hz, 2H), 4.33 (d, J = 6.7 Hz, 2H), 4.21 (t, J = 7.8 Hz, J = 1.8 Hz, 1H), 6.27 (d, J = 3.5 Hz, 2H), 5.85 (s, 1H), 4.35 - 4.28 (m, 8H), 4.06 - 4.04 (m, 4H), 2.62 (t, J = 7.8 Hz, 2H), 2.30 (s, 2H), 1.93 - 1.92 (m, 8H), 1.58 - 1.48 (m, 2H), 1.26 - 1.17 (m, 8H), 0.84 (t, 6.9 Hz, 3H).

Characteristics of compound (No. 1-5-2) were as described below.
Transition temperature: C 44.0 I.

### Synthesis example 3

### Synthesis of compound (No. 1-1-15)

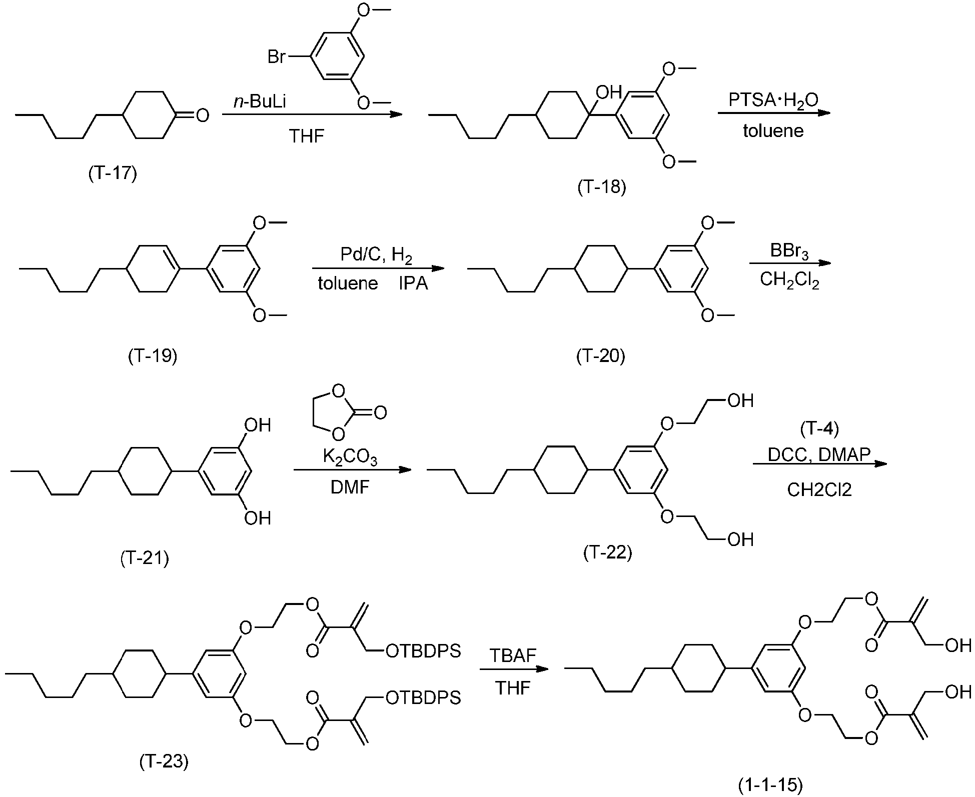

### First step

Then, 1-bromo-3,5-dimethoxybenzene (30.0 g) and THF (300 mL) were put in a reaction vessel, and the resulting mixture was cooled to -70°C. Then, n-butyllithium (BuLi) (1.60 M; in a hexane solution; 103 mL) was slowly added dropwise thereto, and the resulting mixture was stirred at -70°C for 1 hour. A THF (300 mL) solution of compound (T-17) (27.9 g) was slowly added thereto, and the resulting mixture was stirred for 4 hours while returning to room temperature. A reaction mixture was poured into water, and an aqueous layer thereof was subjected to extraction with ethyl acetate. A combined organic layer was washed with brine, and dried over anhydrous magnesium sulfate. The obtained solution was concentrated under reduced pressure, and the resulting residue was purified by silica gel chromatography (a volume ratio, toluene : ethyl acetate = 4 : 1) to obtain compound (T-18) (36.0 g; 85%).

### Second step

Compound (T-18) (36.0 g), p-toluenesulfonic acid monohydrate (2.2 g) and toluene (400 mL) were put in a reaction vessel, and the resulting mixture was stirred at 110 °C for 5 hours. A reaction mixture was poured into water, and an aqueous layer thereof was subjected to extraction with toluene. A combined organic layer was washed with brine, and dried over anhydrous magnesium sulfate. The obtained solution was concentrated under reduced pressure, and the resulting residue was purified by silica gel chromatography (a volume ratio, toluene : heptane = :) to obtain compound (T-19) (32.1 g; 95%).

### Third step

Compound (T-20) (9.7 g; 30%) was obtained by using compound (T-19) (32.1 g) as a raw material in a manner similar to the fourth step in Synthesis Example 2.

### Fourth step

Compound (T-21) (7.01 g; 80%) was obtained by using compound (T-20) (9.70 g) as a raw material in a manner similar to the sixth step in Synthesis Example 2.

### Fifth step

Compound (T-21) (7.01 g), ethylene carbonate (5.17 g), potassium carbonate (11.0 g) and DMF (300 mL) were put in a reaction vessel, and the resulting mixture was stirred at 110°C for 5 hours. A reaction mixture was poured into water, and an aqueous layer thereof was subjected to extraction with toluene. A combined organic layer was washed with brine, and dried over anhydrous magnesium sulfate. The obtained solution was concentrated under reduced pressure, and the resulting residue was purified by silica gel chromatography (a volume ratio, toluene : heptane = :) to obtain compound (T-22) (6.09 g; 65%).

### Sixth step

Compound (T-23) (9.86 g; 57%) was obtained by using compound (T-22) (6.09 g) as a raw material in a manner similar to the sixth step in Synthesis Example 2.

### Seventh step

Compound (1-1-15) (2.5 g; 48%) was obtained by using compound (T-23) (9.86 g) as a raw material in a manner similar to the seventh step in Synthesis Example 2.

NMR analytical values of the obtained compound (1-1-15) were as described below.
¹H-NMR : Chemical shift δ (ppm; CDCl₃): 6.43 (d, J = 2.0 Hz, 1H), 6.40 (d, J = 2.0 Hz, 1H), 6.32 - 6.30 (m, 3H), 5.86 (d, J = 1.2 Hz, 2H), 4.52 - 4.50 (m, 4H), 4.33 (s, 4H), 4.21 - 4.19 (m, 4H), 1.86 - 1.84 (m, 2H), 1.67 - 1.18 (m, 14H), 1.02 - 0.85 (m, 7H).
Transition temperature: SA 47.6 I.

### Comparative Example 1

Compound (S-1) was prepared as a comparative compound, and characteristics thereof were measured. The compound was described in WO 2014/090362 A, and was selected because the compound is similar to the compounds of the invention.

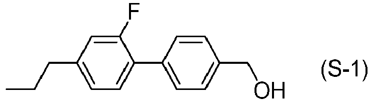

NMR analytical values of comparative compound (S-1) were as described below.
¹H-NMR : Chemical shift δ (ppm; CDCl₃): 7.57 - 7.52 (m, 2H), 7.45 - 7.42 (m, 2H), 7.36 - 7.30 (m, 1H), 7.04 - 6.95 (m, 2H), 4.75 (d, 6.0 Hz, 2H), 2.62 (t, J = 7.8 Hz, 2H), 1.75 - 1.64 (m, 3H), 0.98 (t, J = 7.4 Hz, 3H).

Vertical alignment properties of compound (No. 1-7-2) and comparative compound (S-1) were compared. In addition, composition (i) and polymerizable compound (RM-1) were used for evaluation.

A proportion of composition (i) was expressed in terms of % by weight.

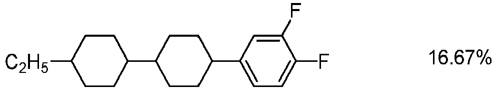

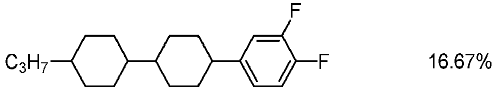

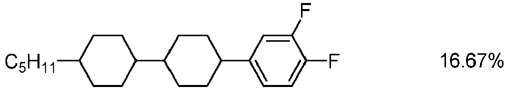

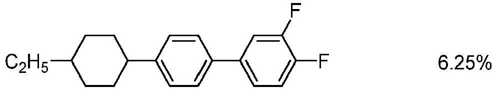

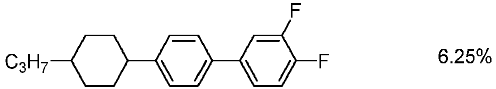

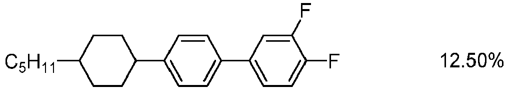

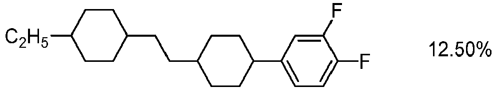

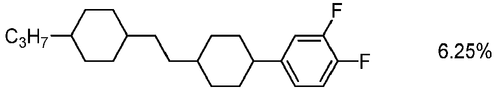

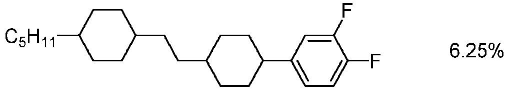

Polymerizable compound (RM-1) is as described below.

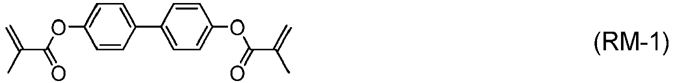

### Vertical alignment properties

To composition (i), polymerizable compound (RM-1) was added in a proportion of 0.4% by weight. Then, compound (1-1-10) or comparative compound (S-1) was added thereto in a proportion from 0.5% to 3.0%. The resulting mixture was injected into a device having no alignment film in which a distance (cell gap) between two glass substrates was 3.5 micrometers. The polymerizable compound was polymerized by irradiating the device with ultraviolet light (30 J) using Black Light F40T10/BL (a peak wavelength of 369 nm) made by Eye graphics Co. Ltd. The device was set to a polarizing microscope, and the device was irradiated with light from below and presence or absence of light leakage was observed. When liquid crystal molecules were sufficiently aligned to prevent light from passing through the device, the vertical alignment properties were judged as "good." When light passed through the device was observed, the vertical alignment properties were expressed as "poor."

**Table 2. Alignment properties of compound (1-7-2) and comparative compound (S-1)**

| Addition amount (% by weight) | 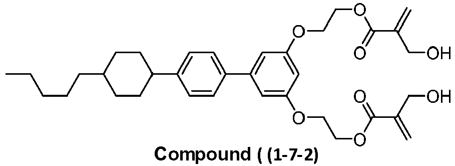 | 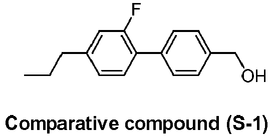 |
|---|---|---|
| 0.5 | Good | Poor |
| 1.0 | Good | Poor |
| 2.0 | Good | Poor |
| 3.0 | Good | Good |

The vertical alignment properties of compound (No. 1-7-2) in Example 1 and comparative compound (S-1) are summarized in Table 2. In the case of comparative compound (S-1), the vertical alignment properties were confirmed by addition of 3.0%. On the other hand, in the case of using compound (No. 1-7-2), the vertical alignment properties were confirmed by addition of 0.5%, and good vertical alignment properties at a lower concentration were exhibited in comparison with comparative compound (S-1). The reason is that, in compound (No. 1-7-2), the vertical alignment properties are enhanced by having a plurality of -OH groups for inducing vertical alignment. Accordingly, compound (No. 1-7-2) was found to be a superior compound exhibiting good vertical alignment properties at a low concentration.

According to the synthesis method described in Example 1, compounds (1-1-1) to (1-1-80), compounds (1-2-1) to (1-2-225), compounds (1-3-1) to (1-3-100), compounds (1-4-1) to (1-4-70), compounds (1-5-1) to (1-5-75) and compounds (1-6-1) to (1-6-60) described below can be prepared.

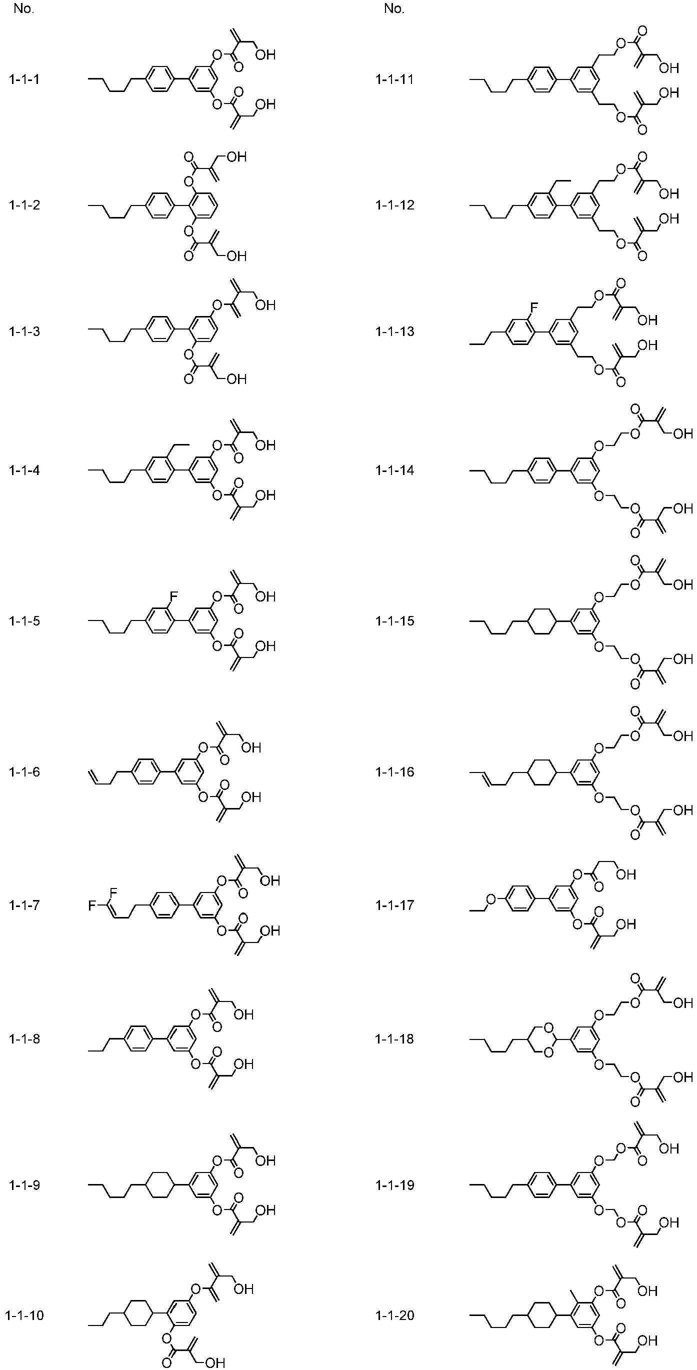

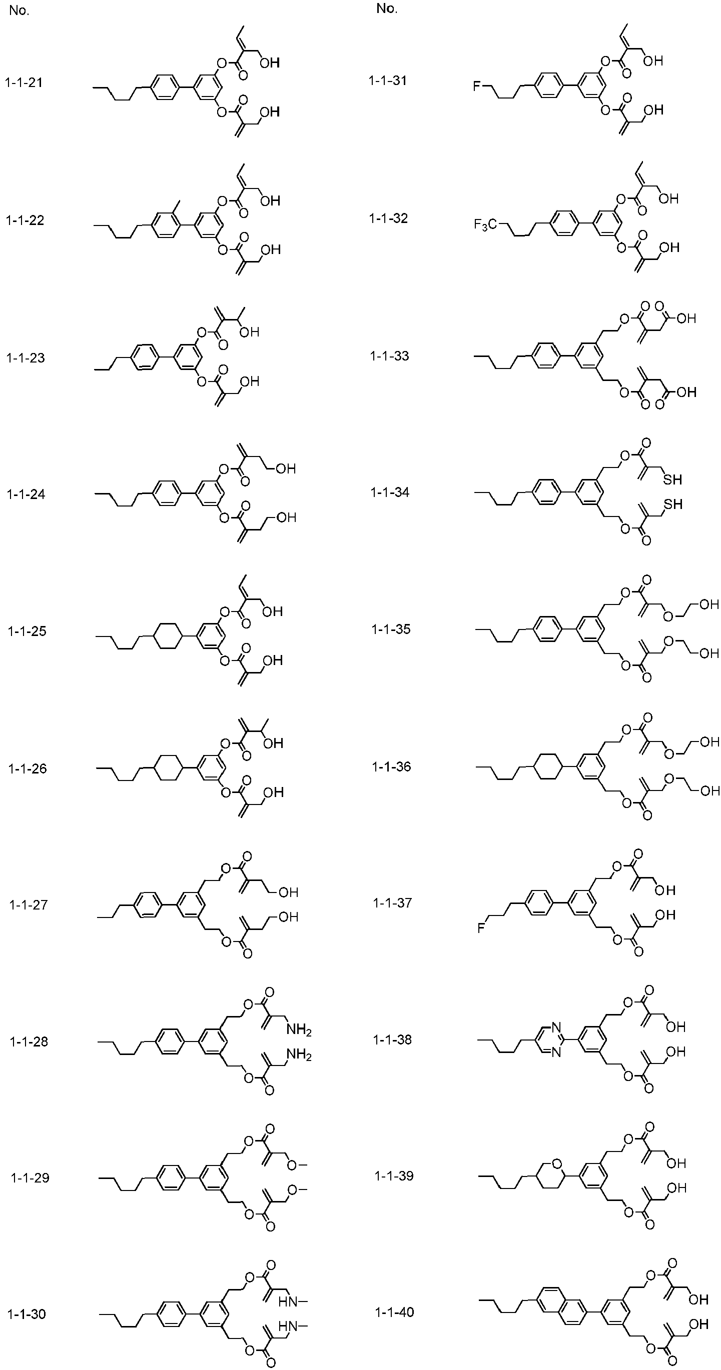

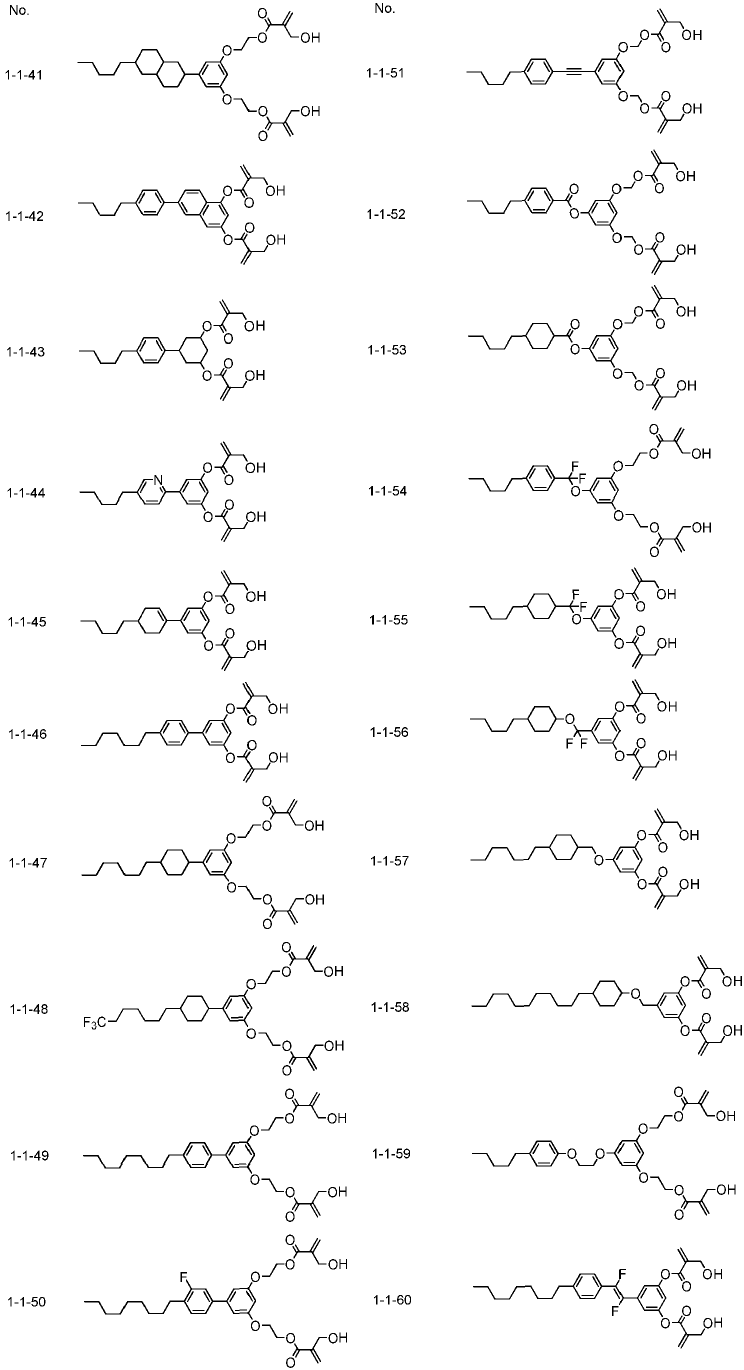

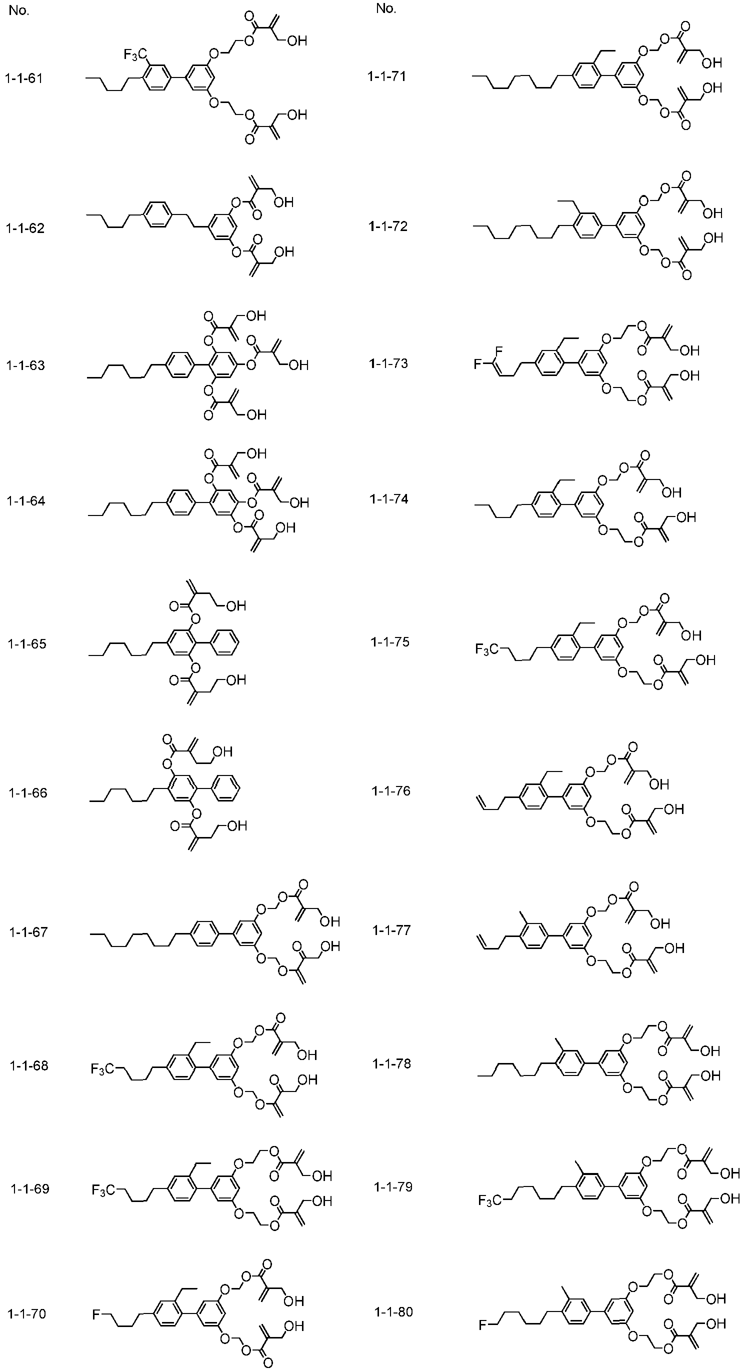

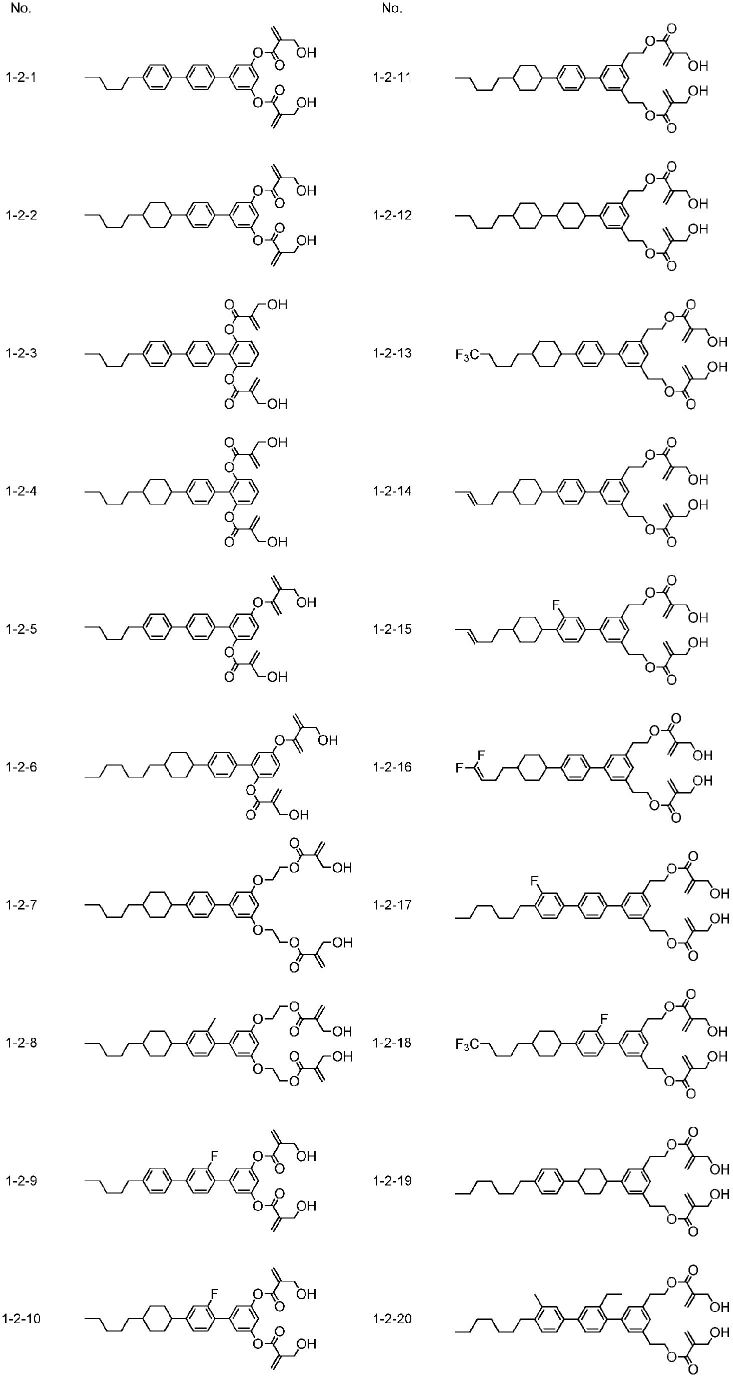

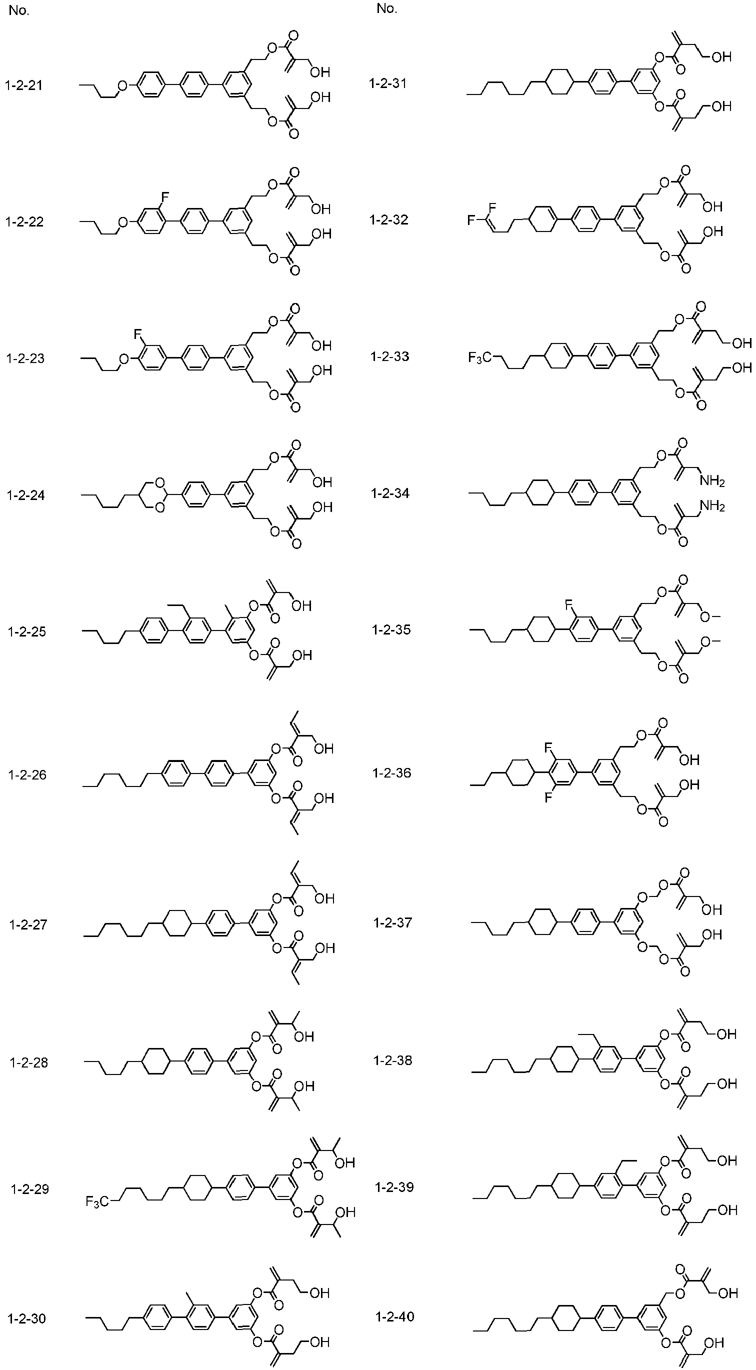

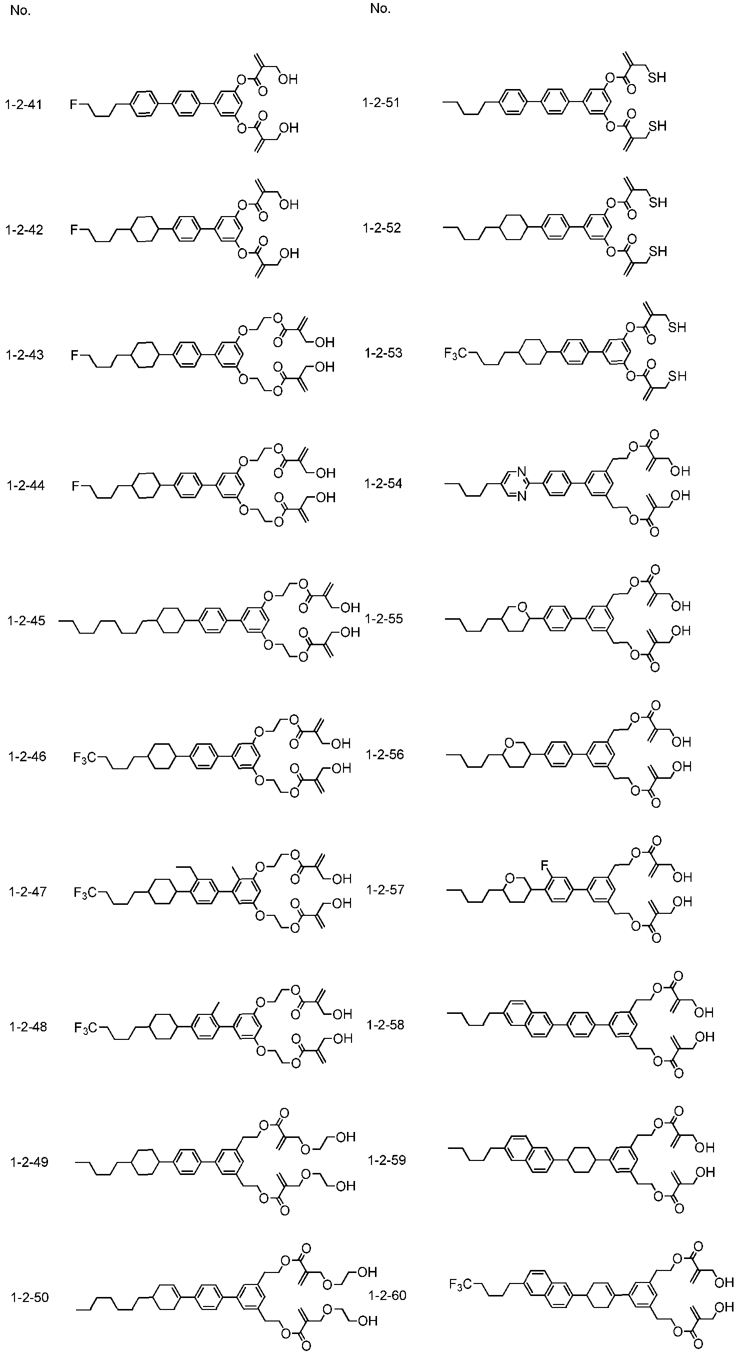

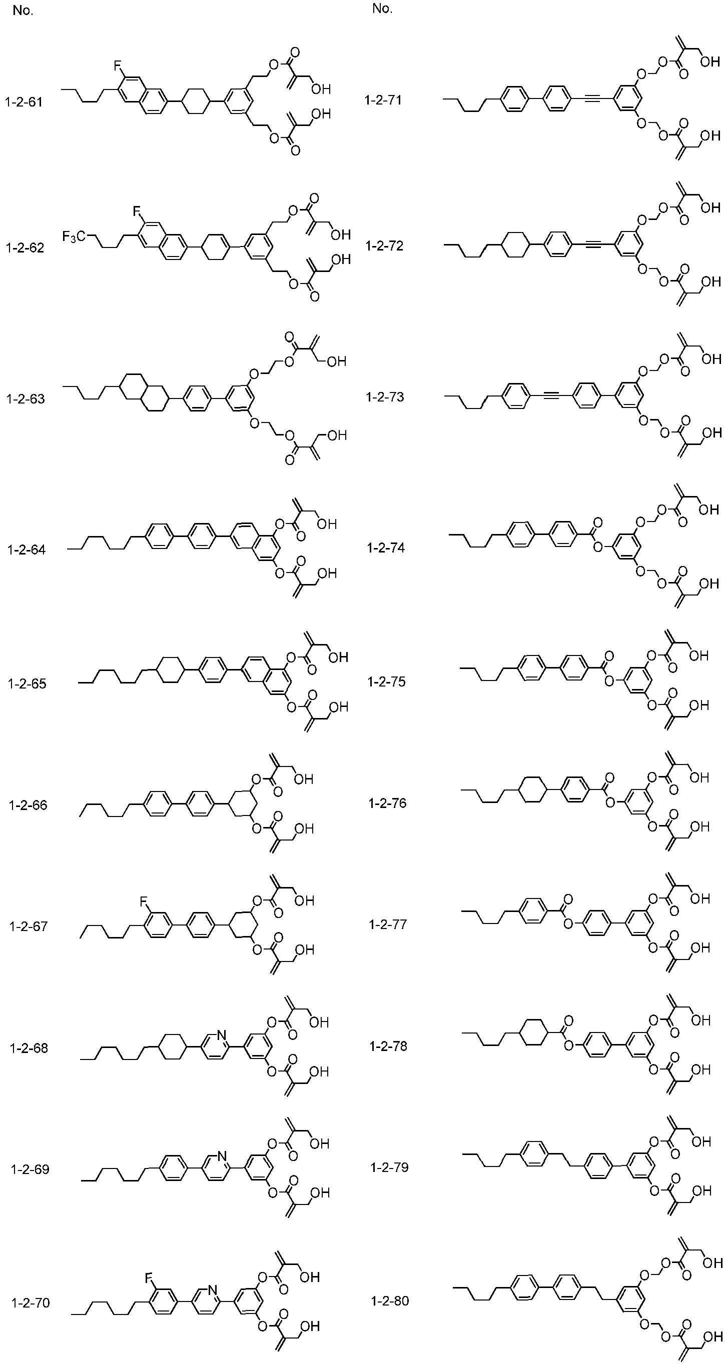

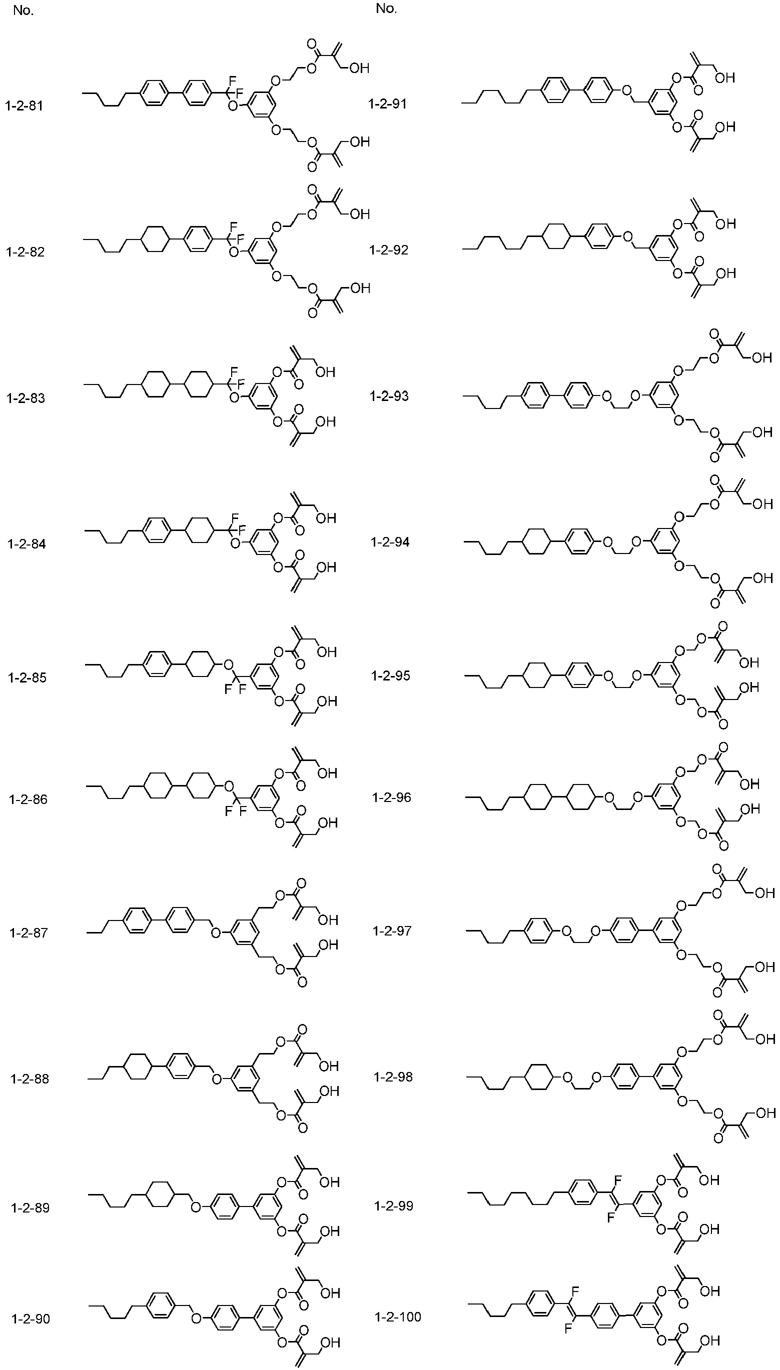

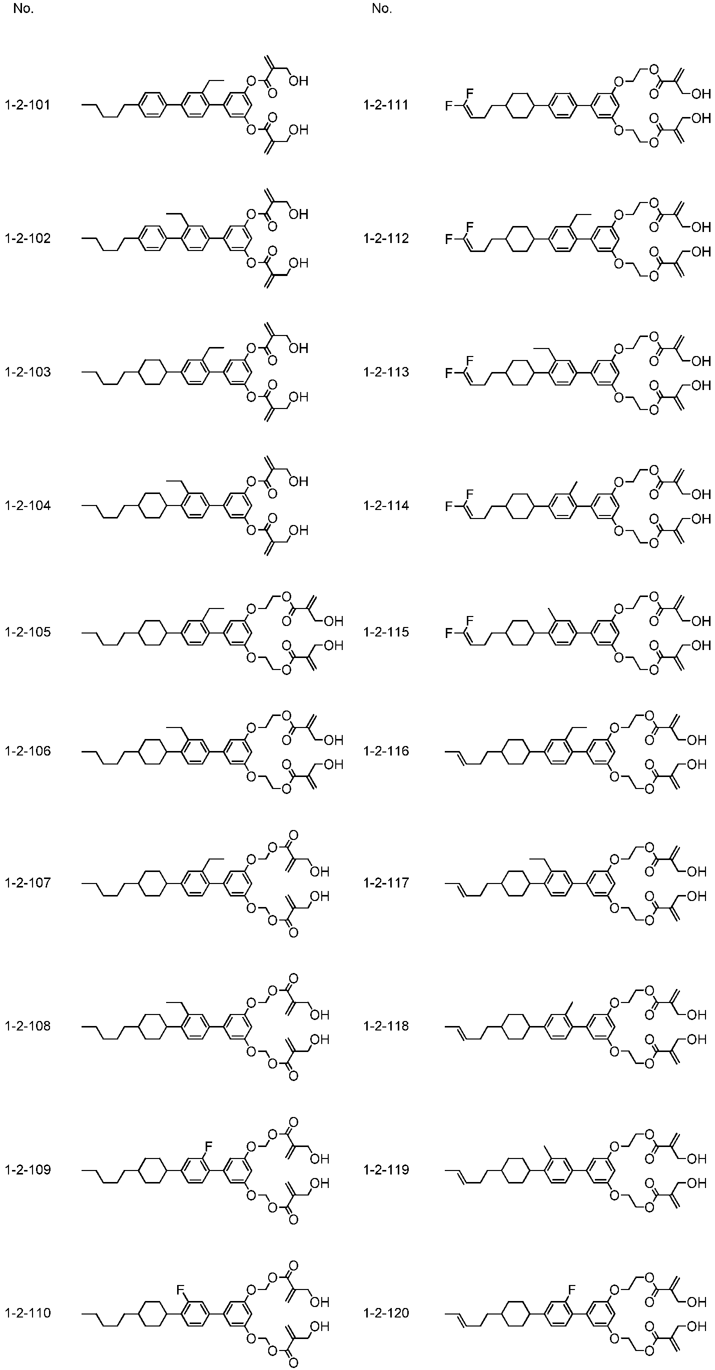

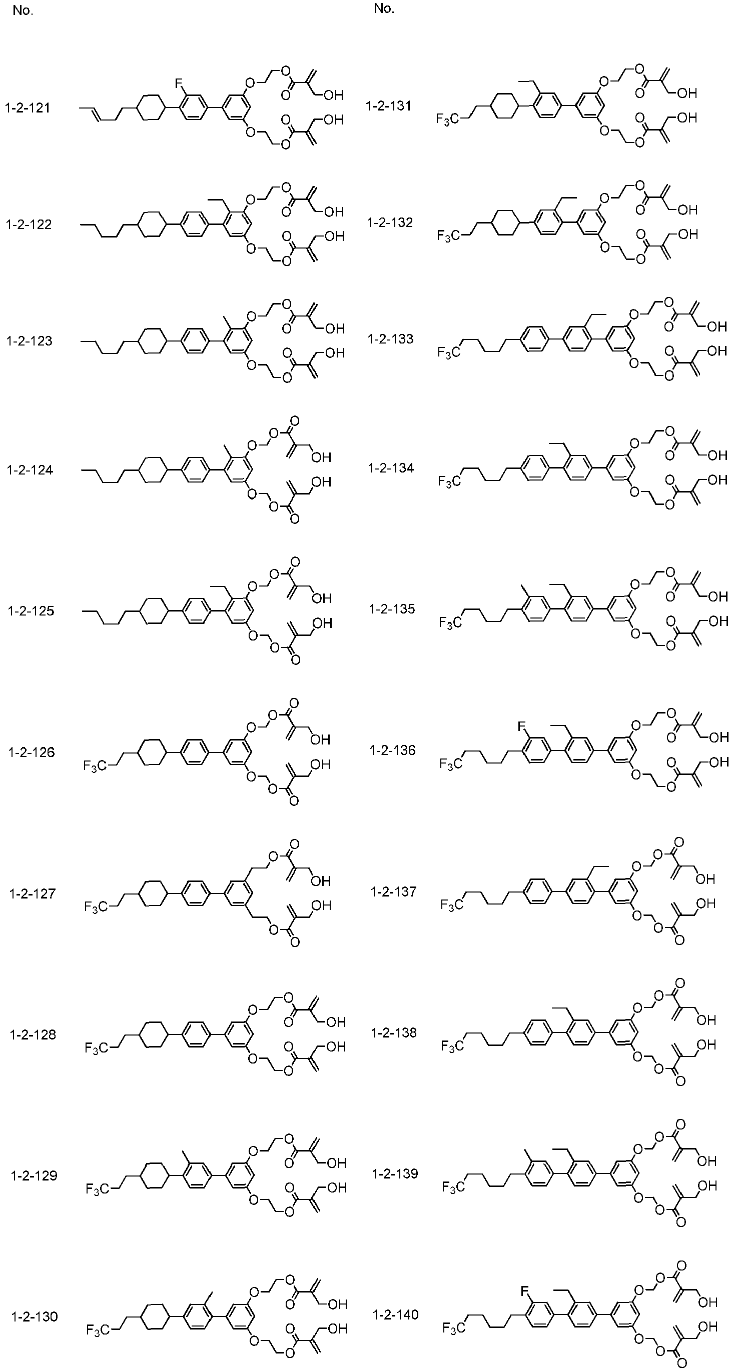

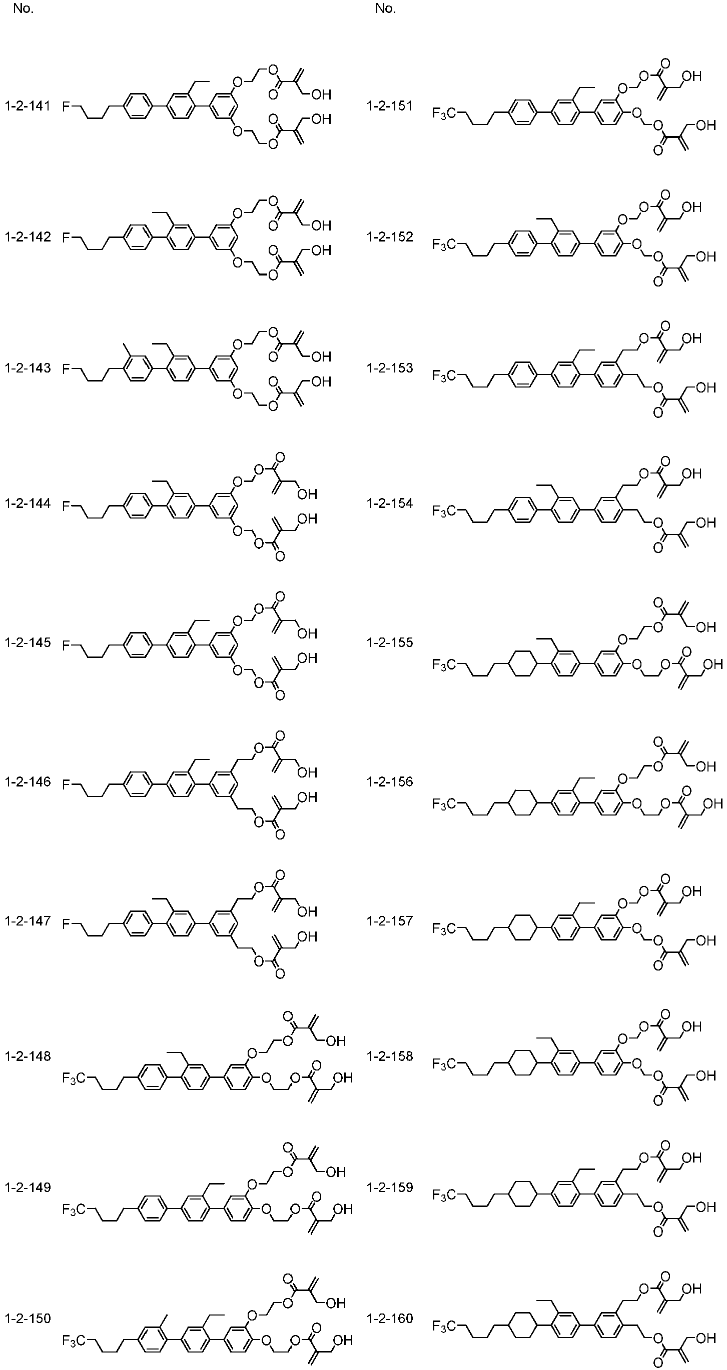

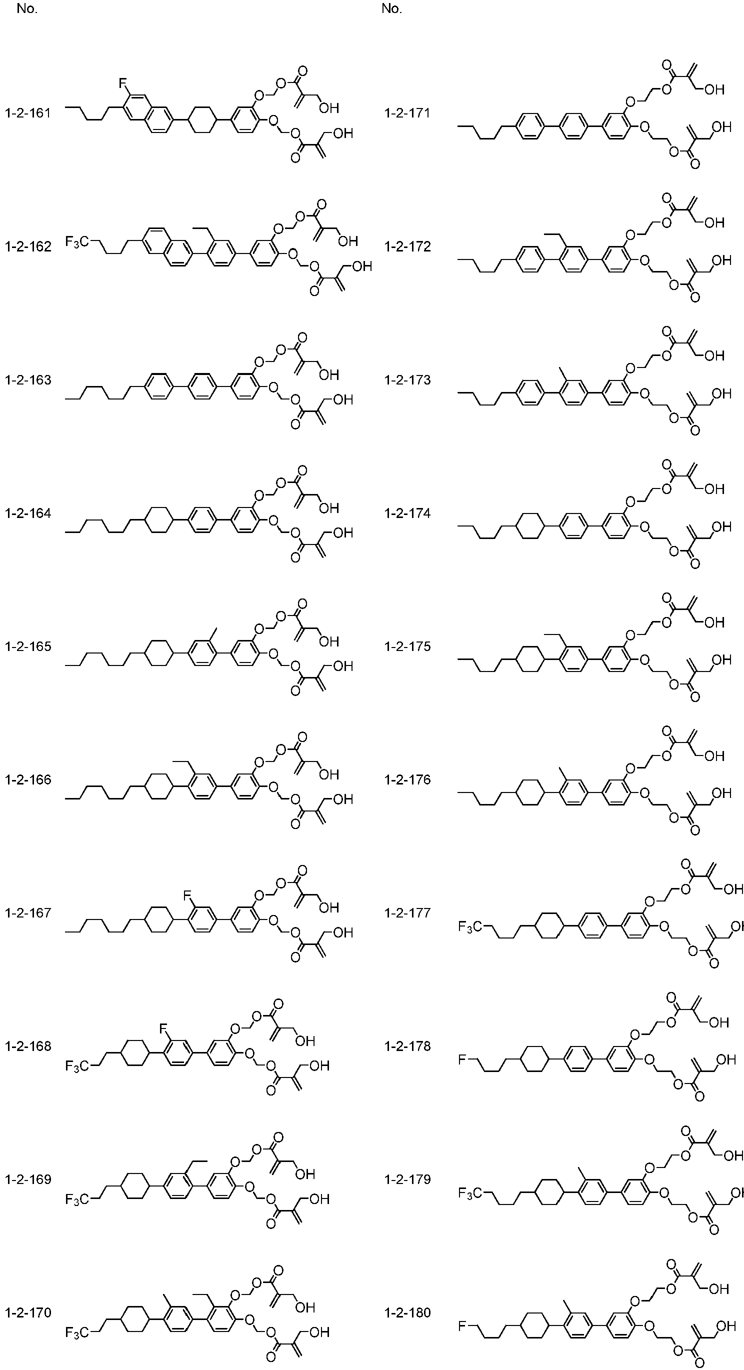

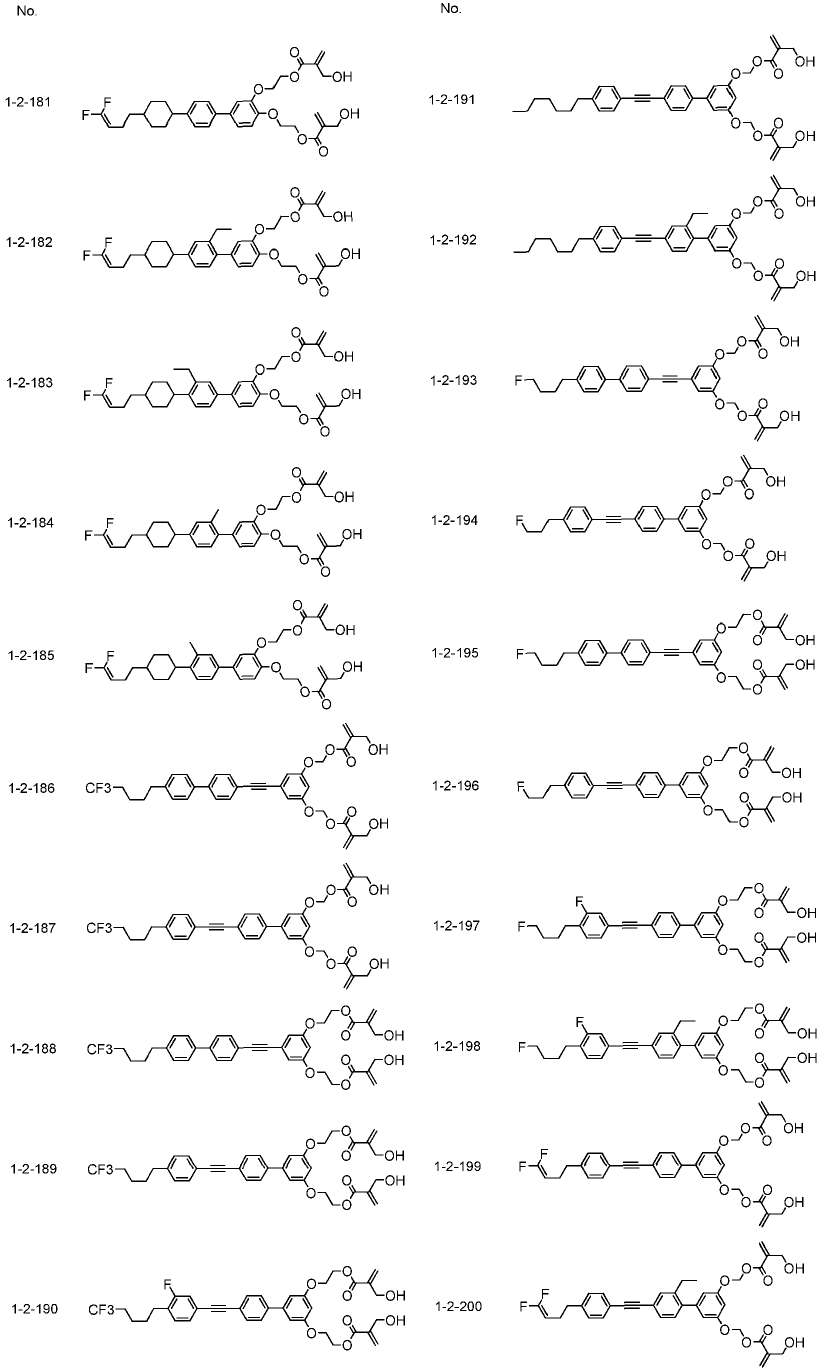

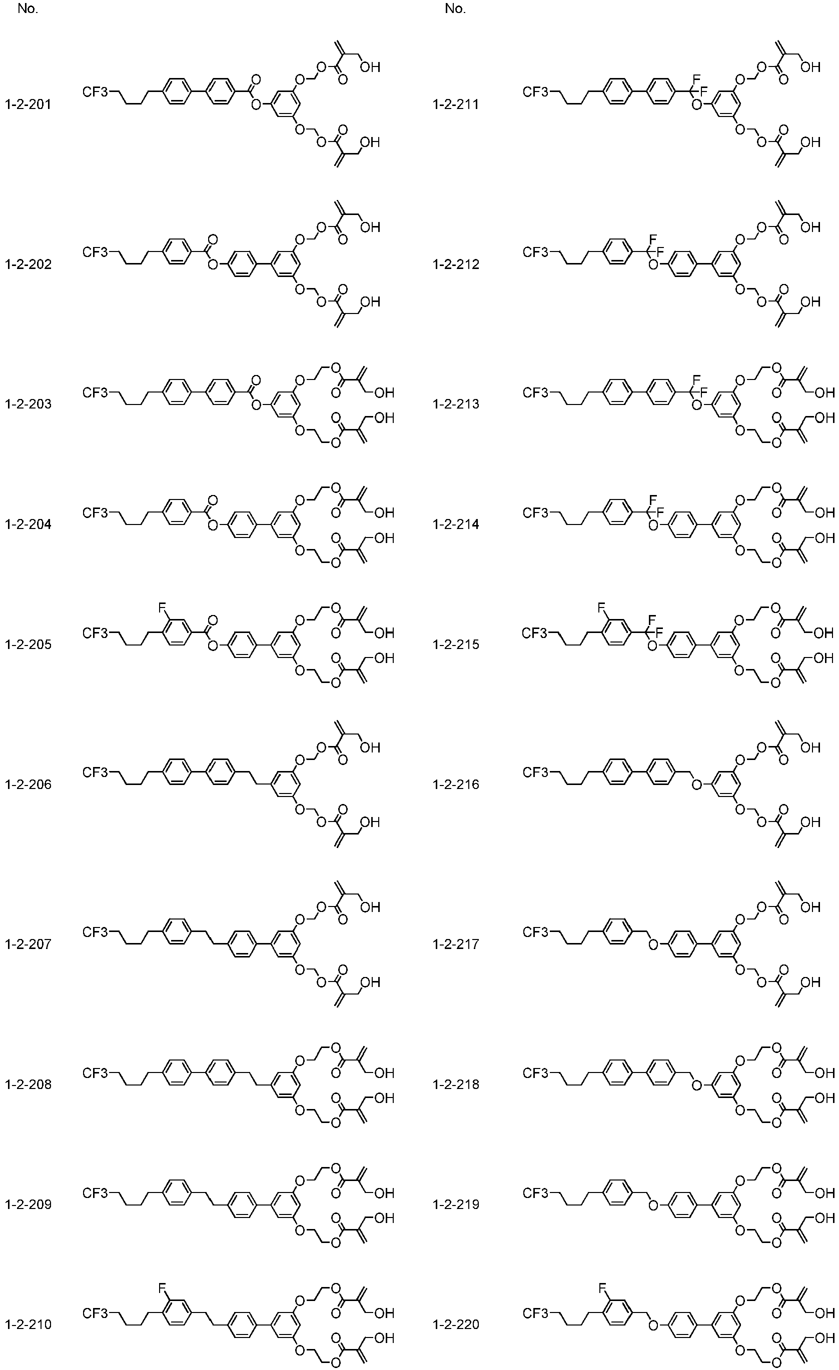

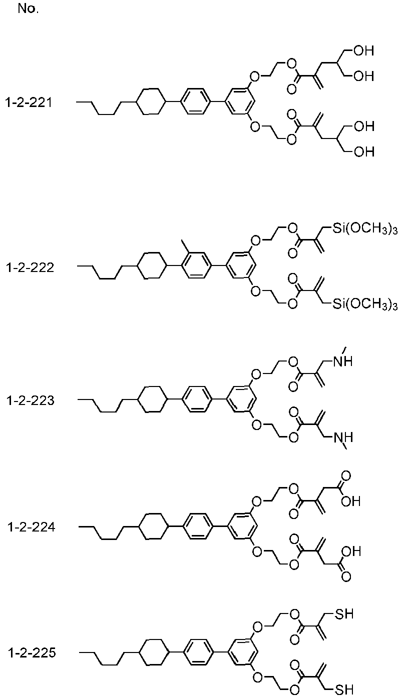

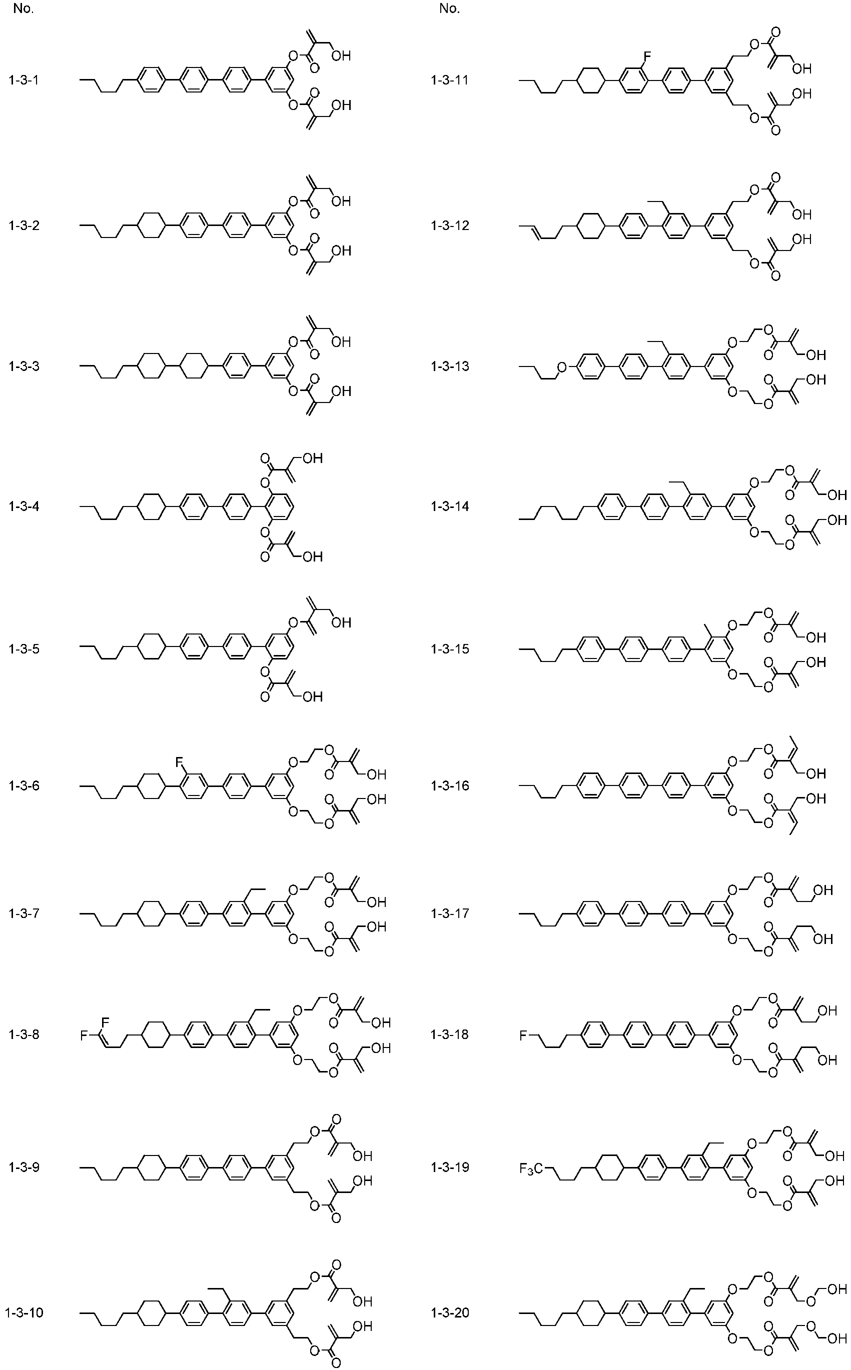

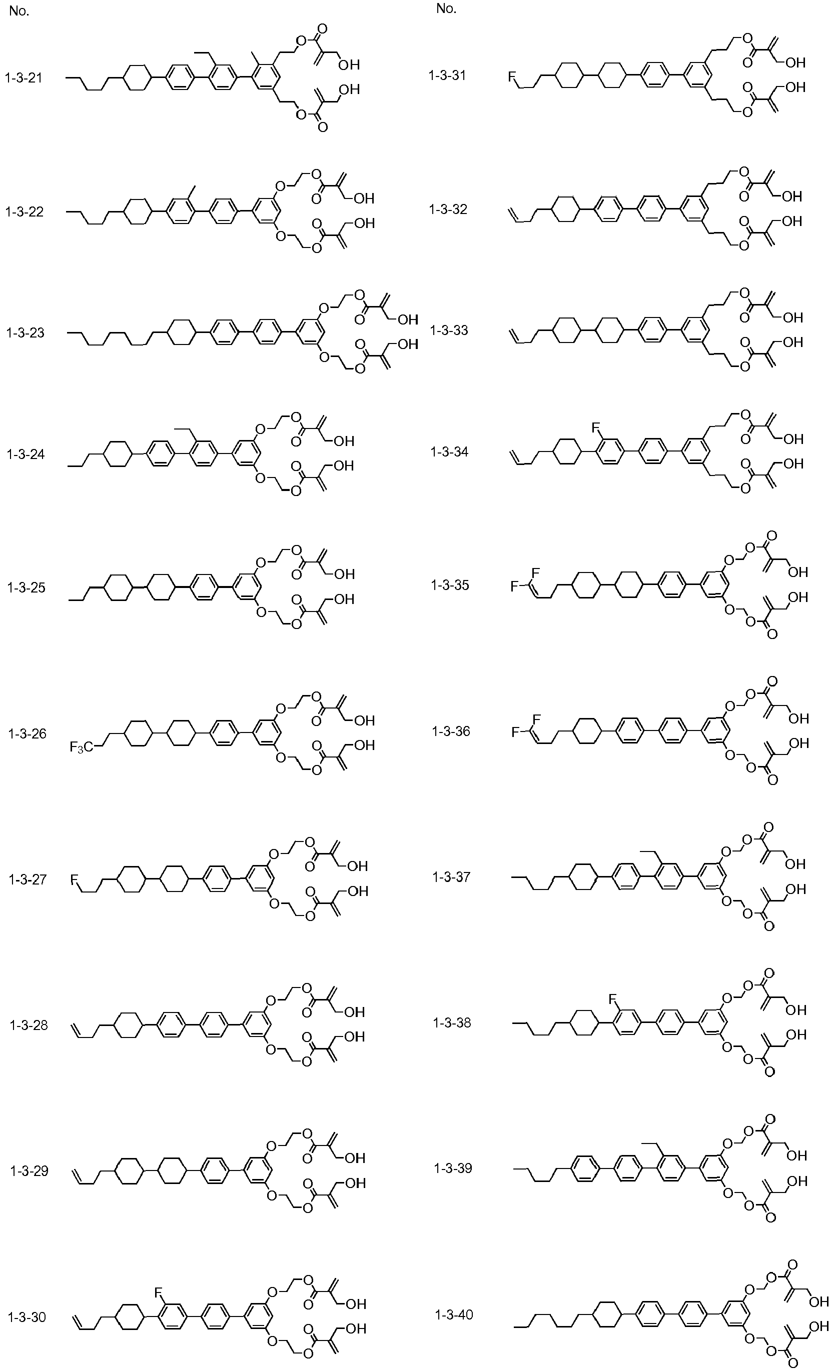

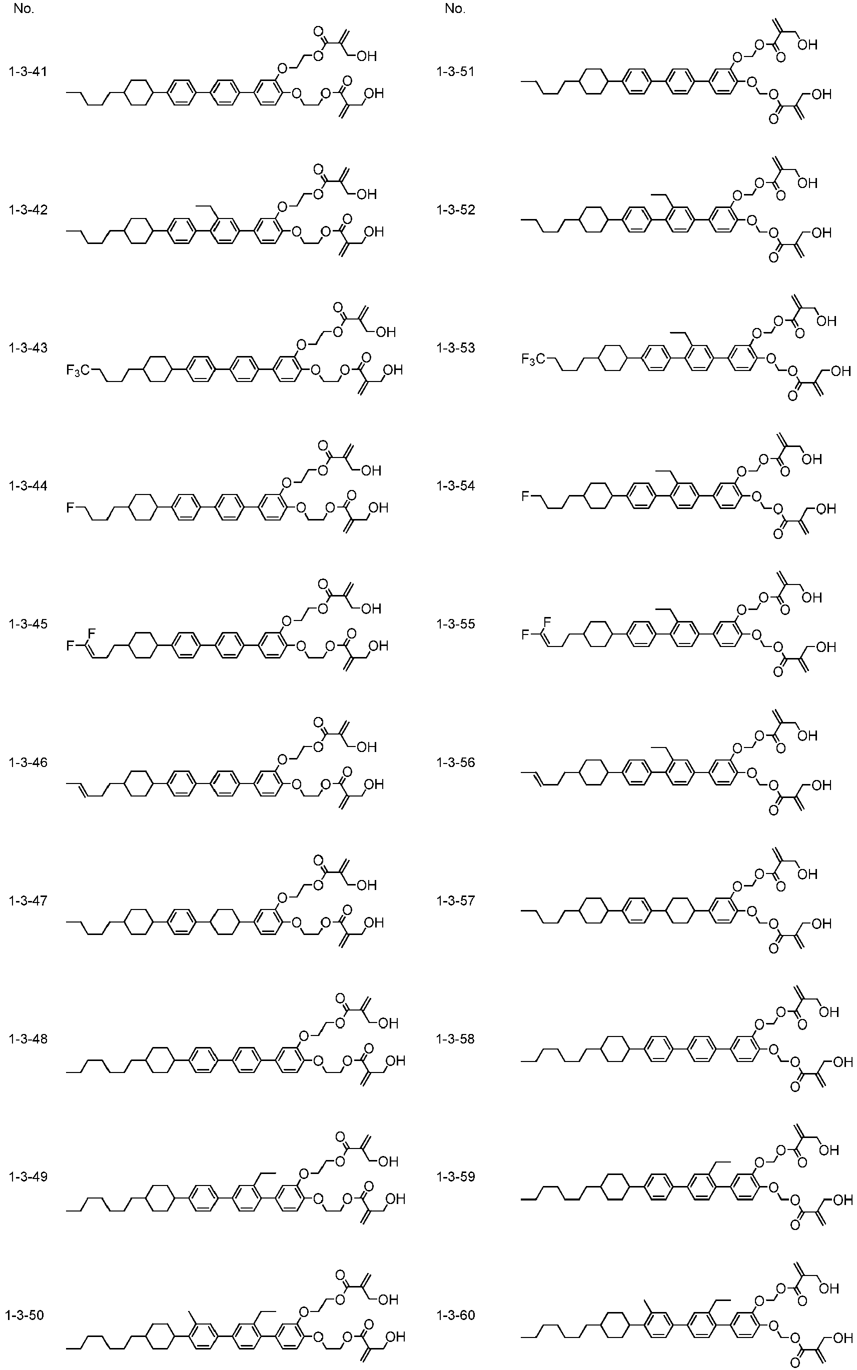

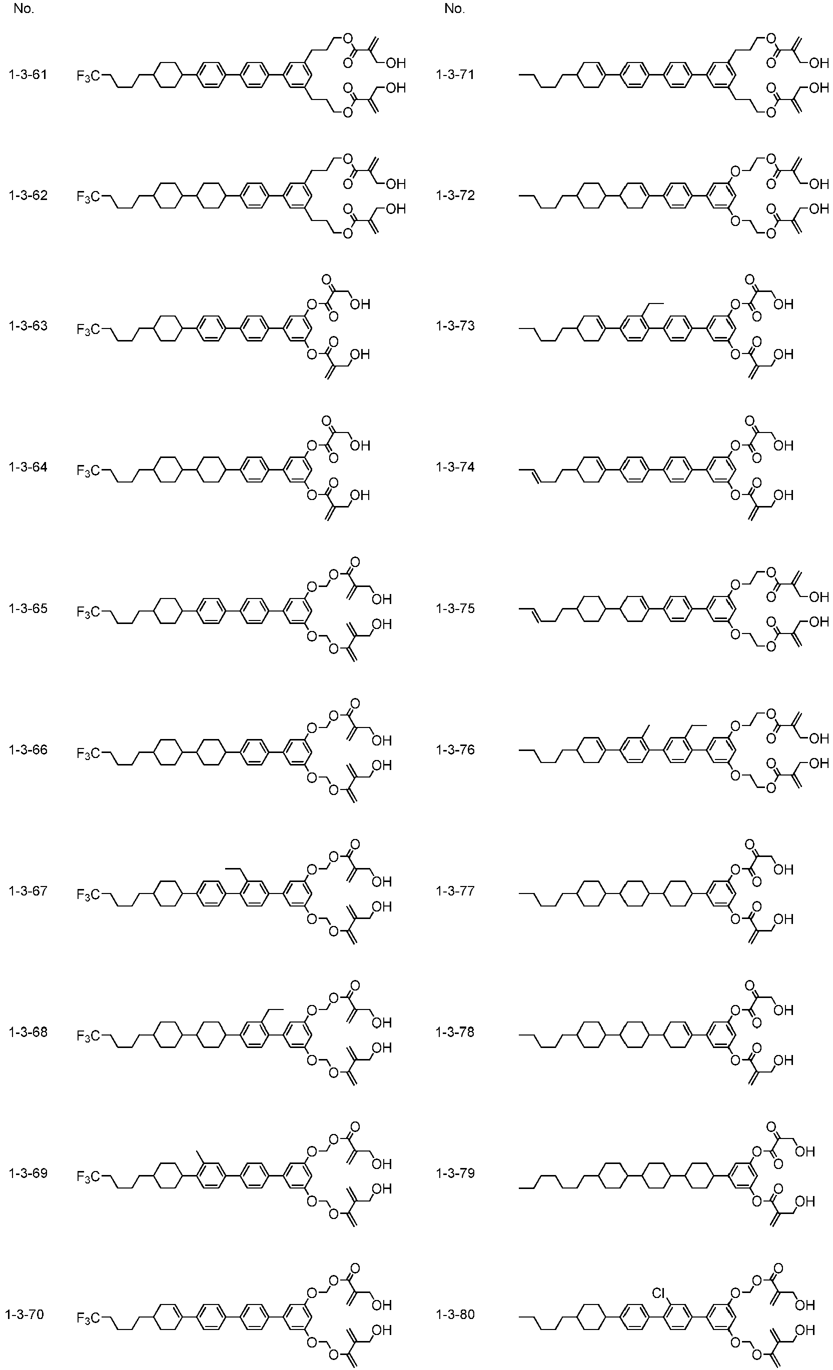

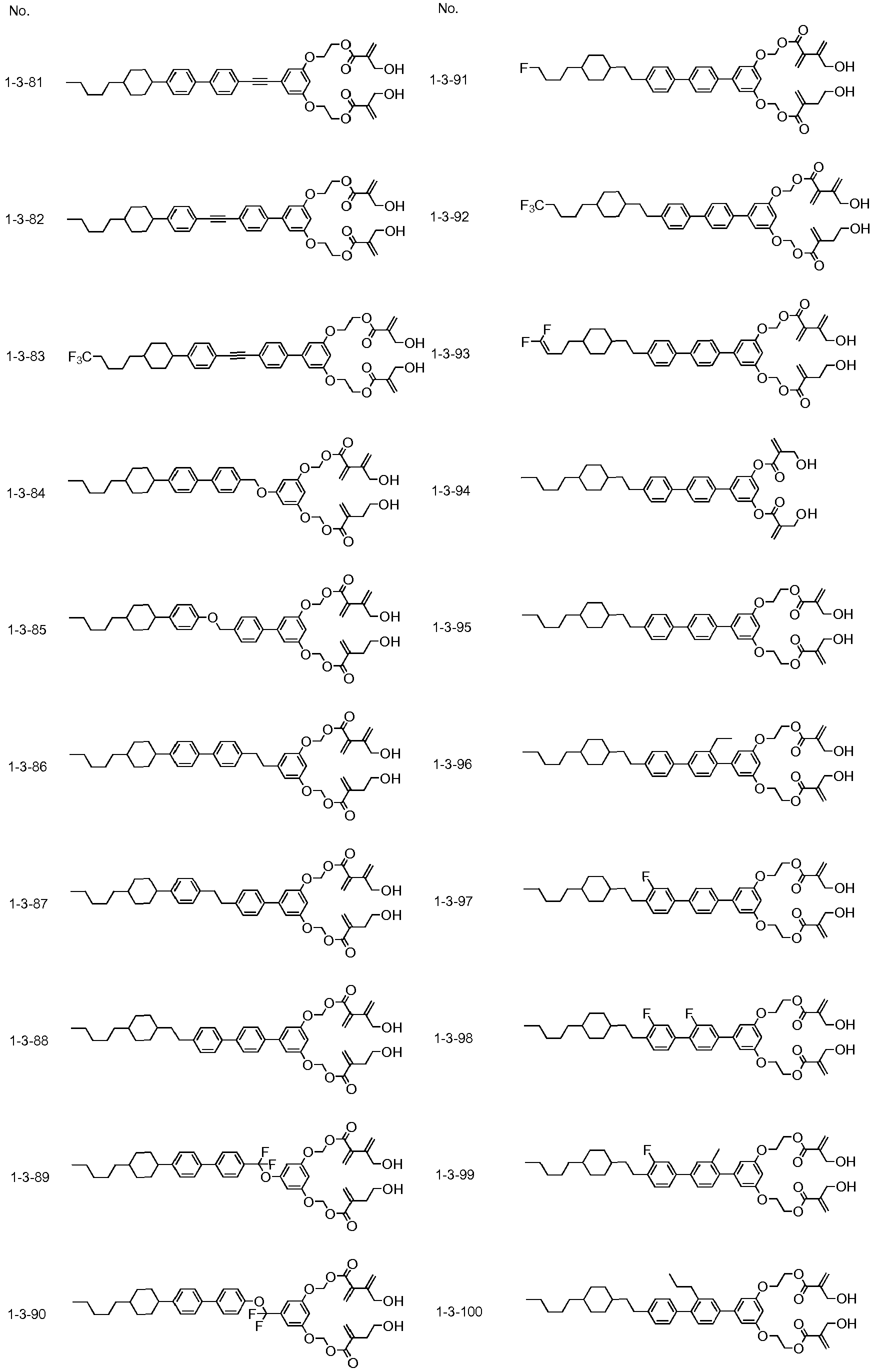

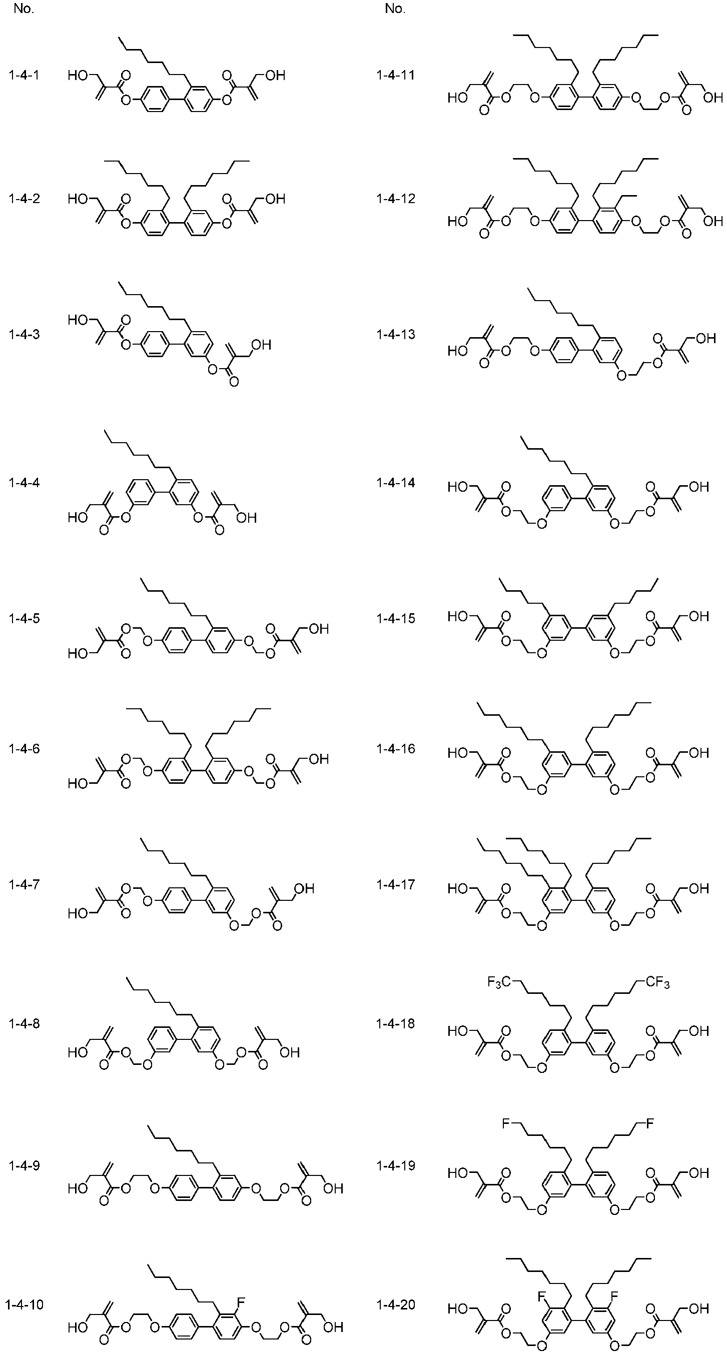

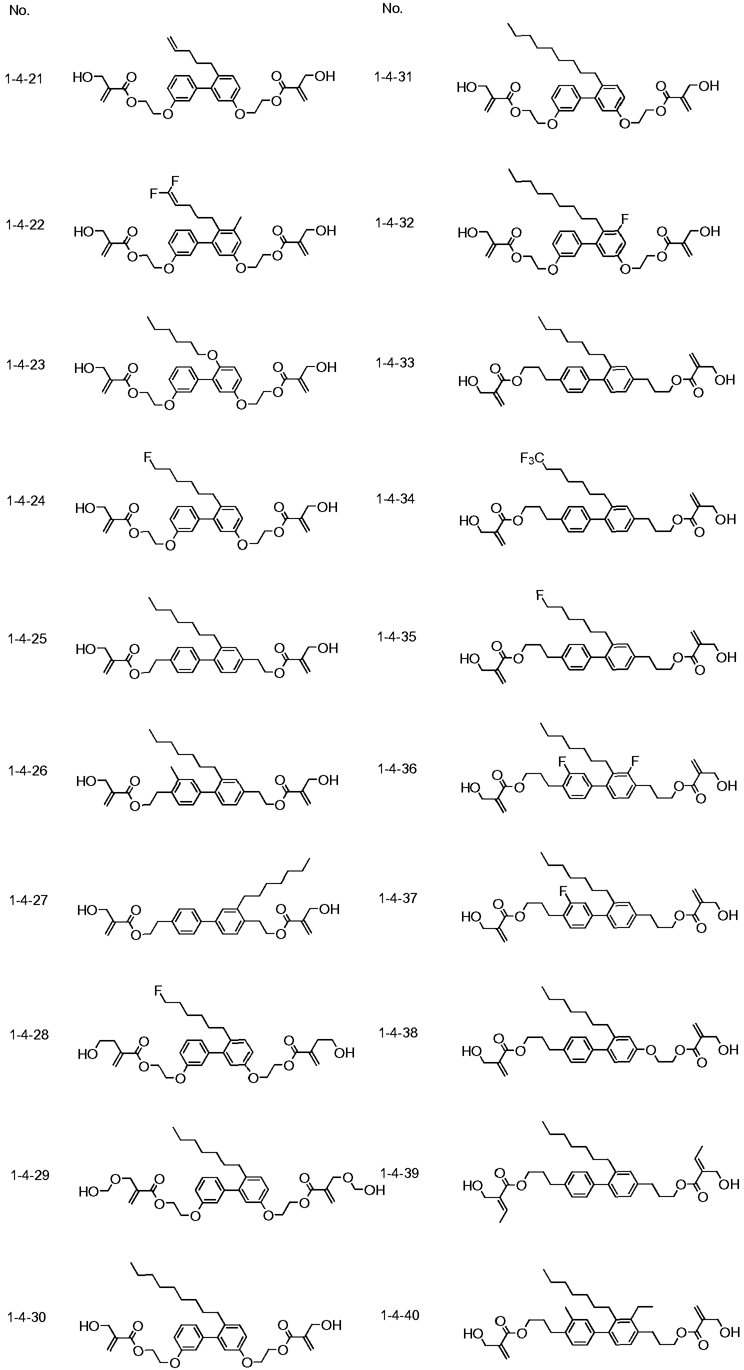

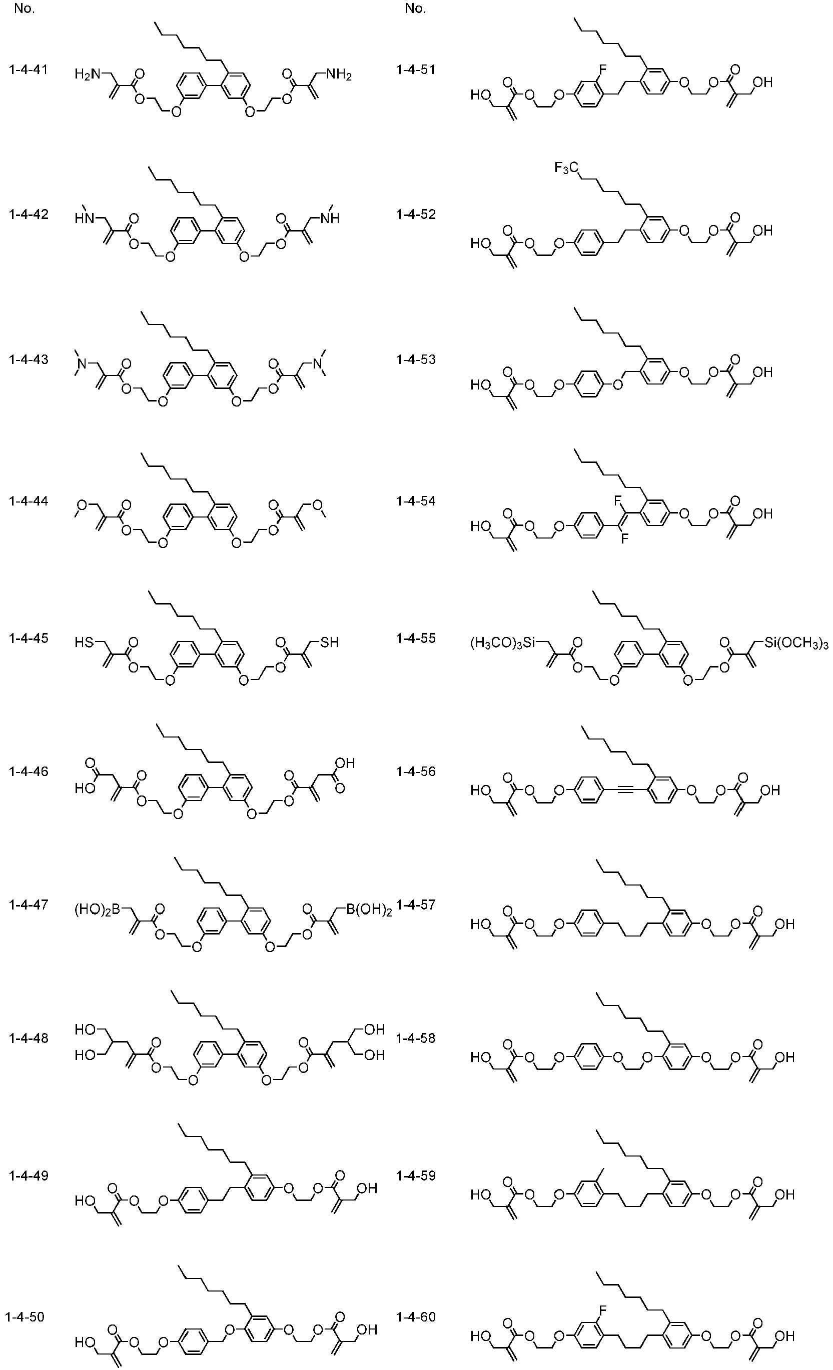

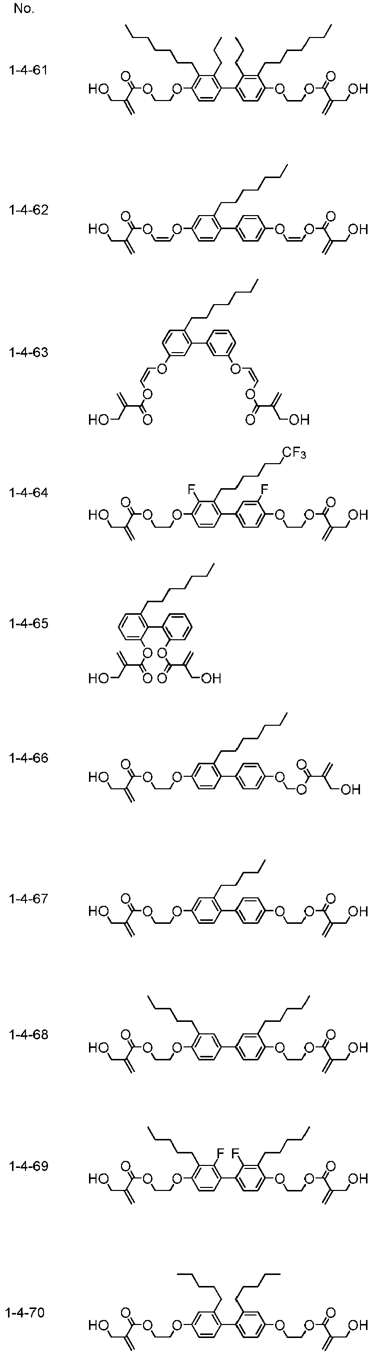

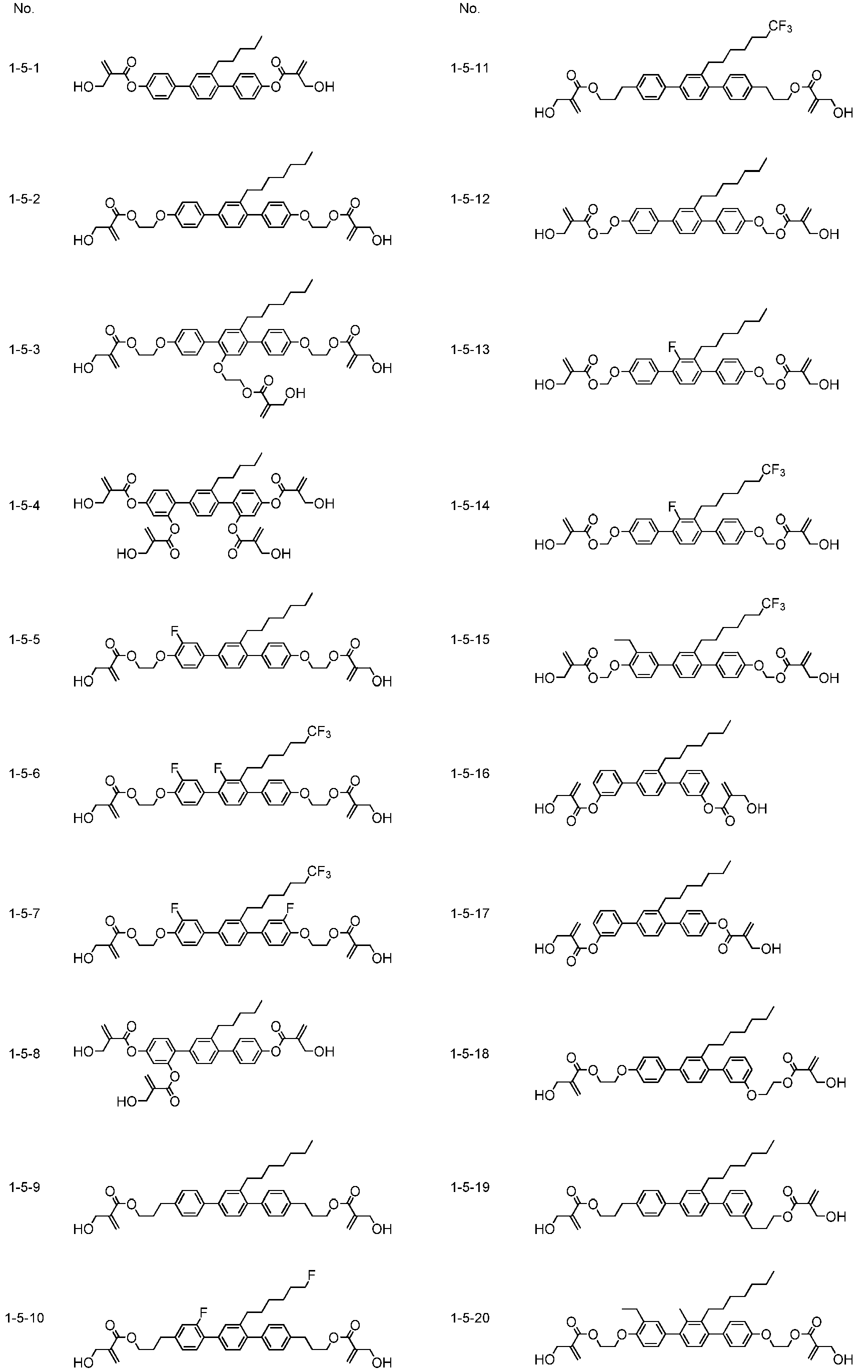

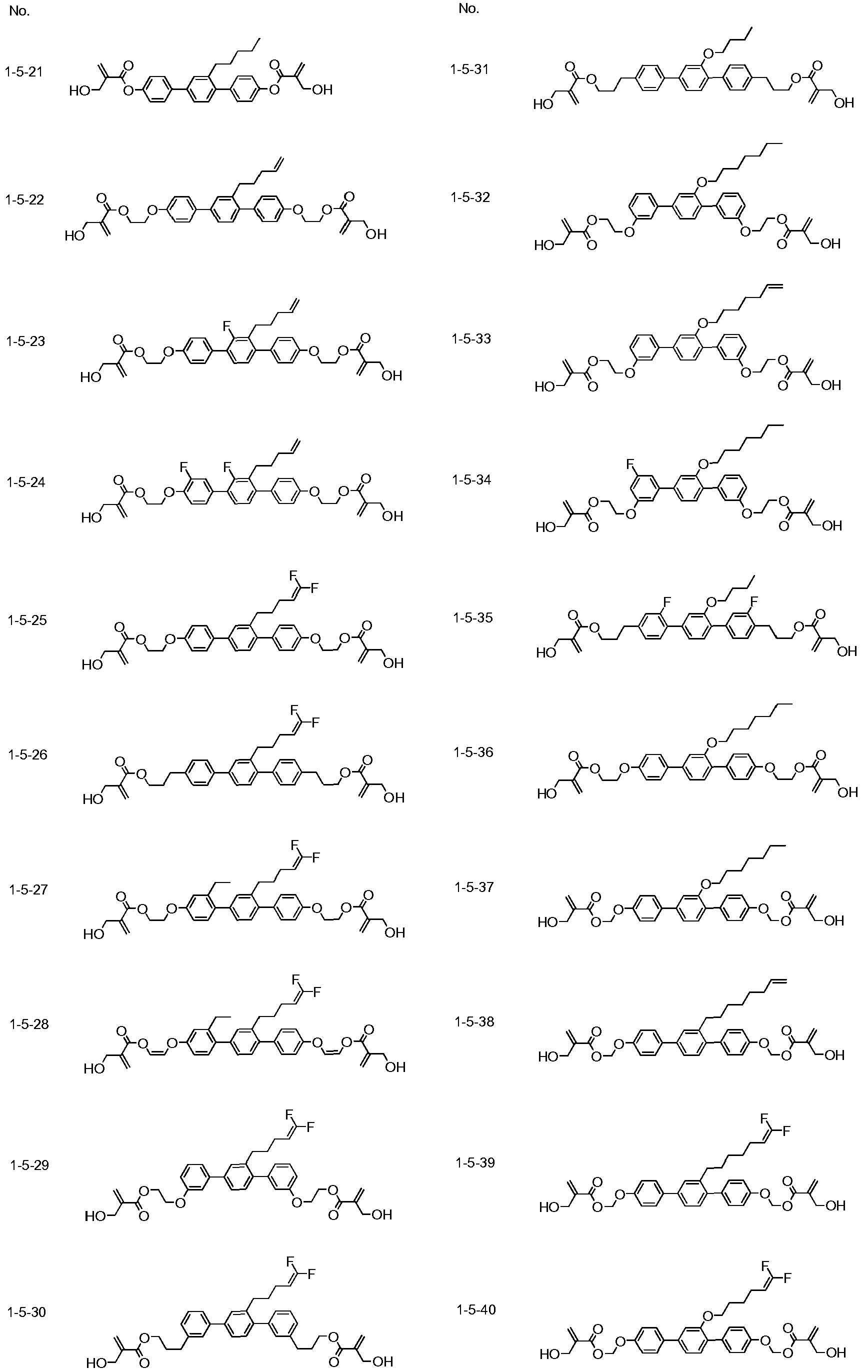

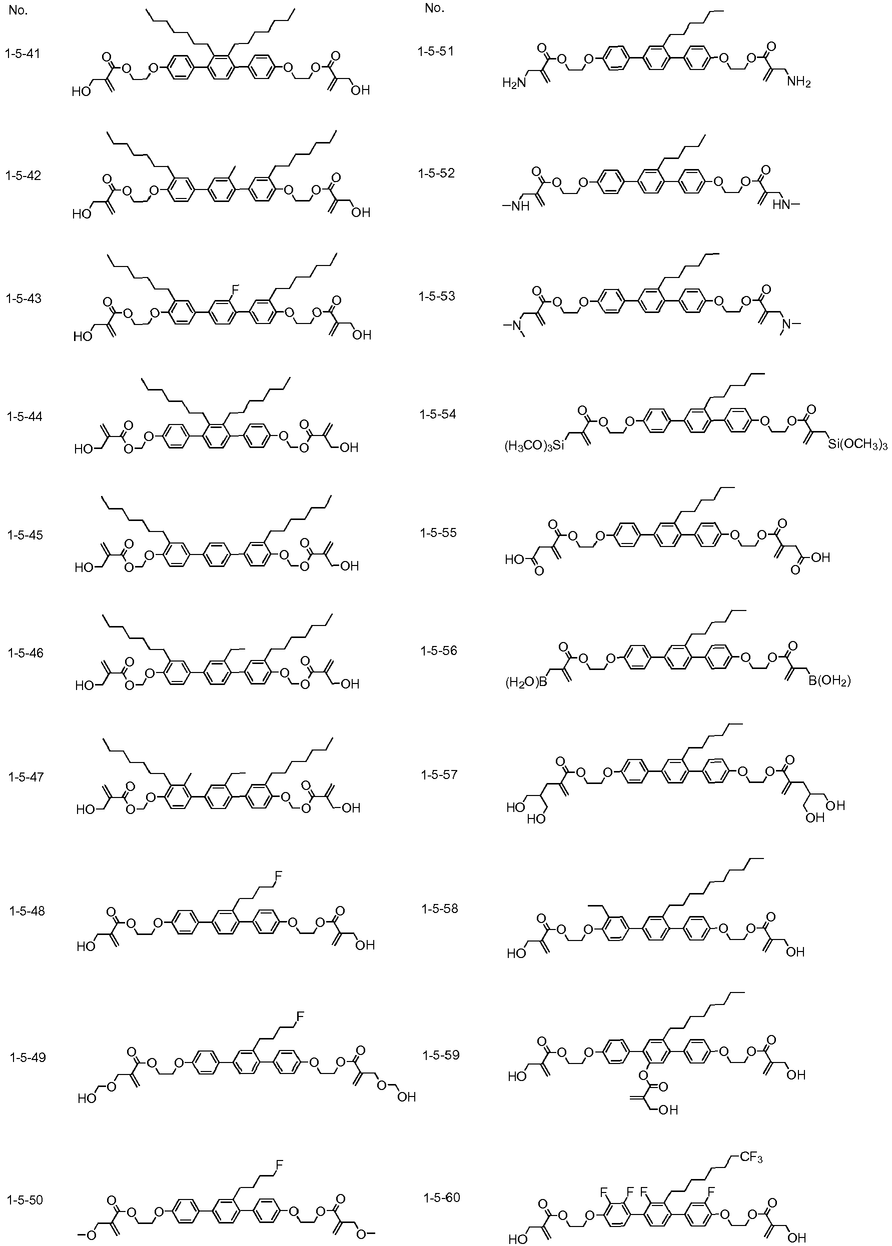

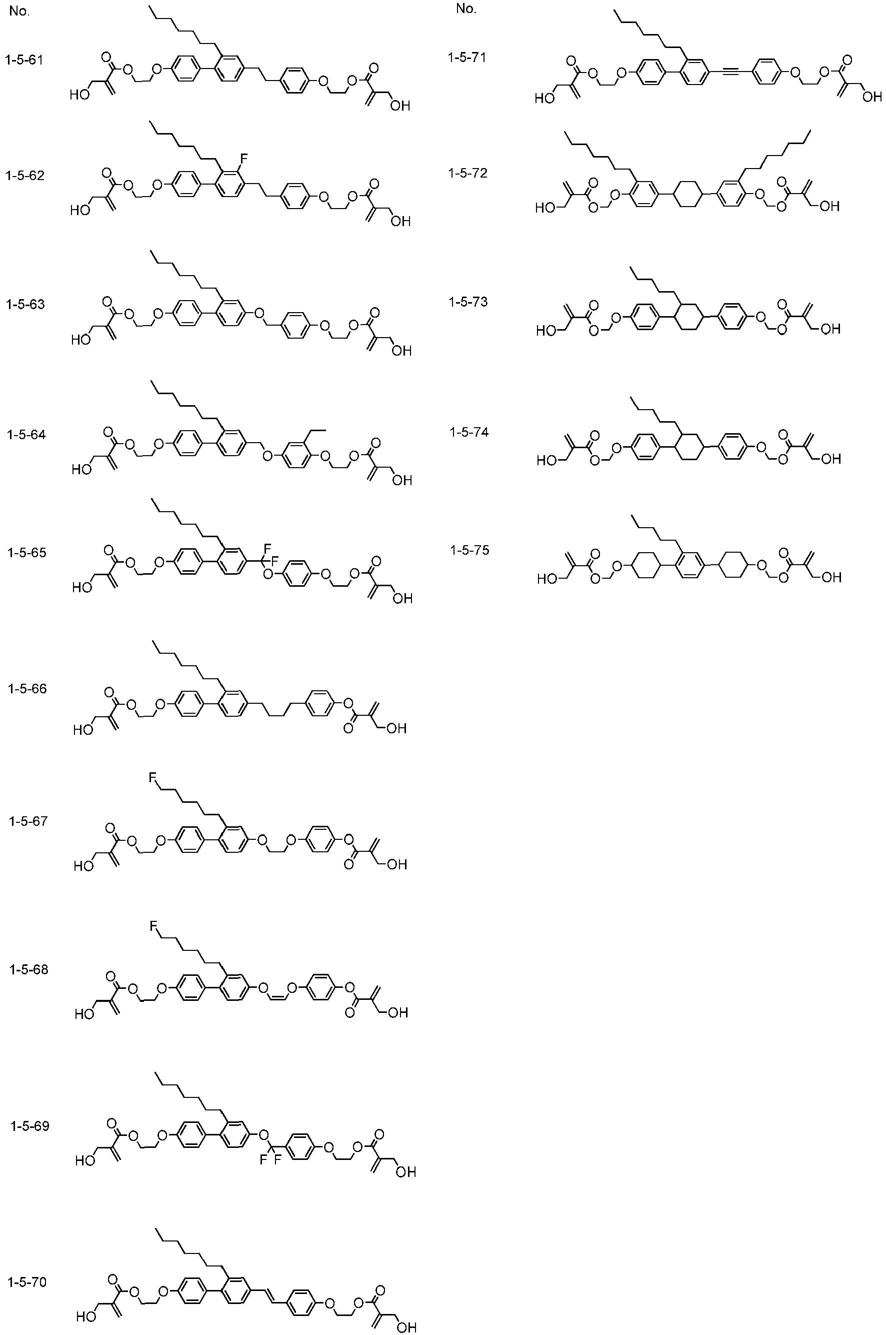

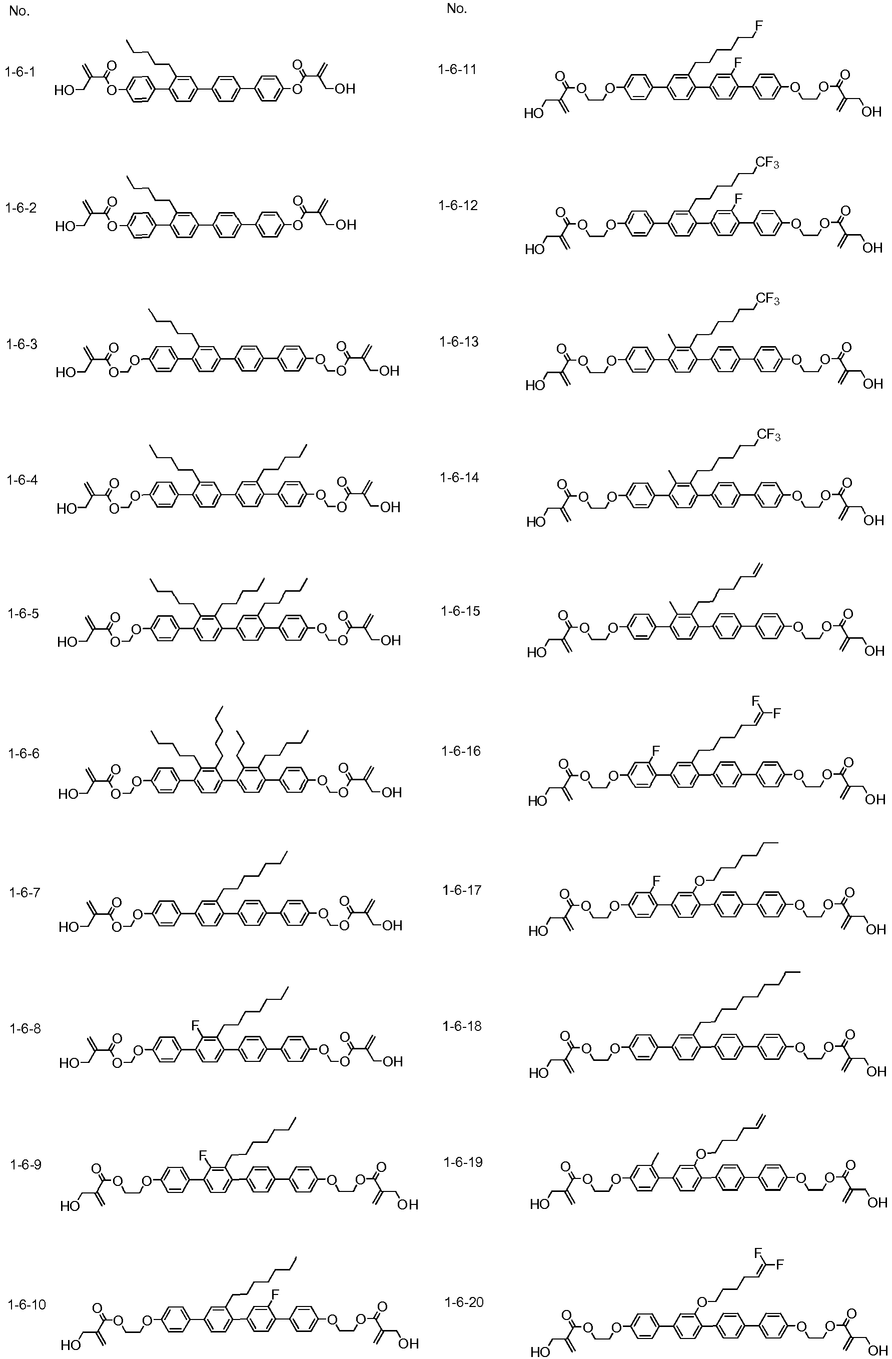

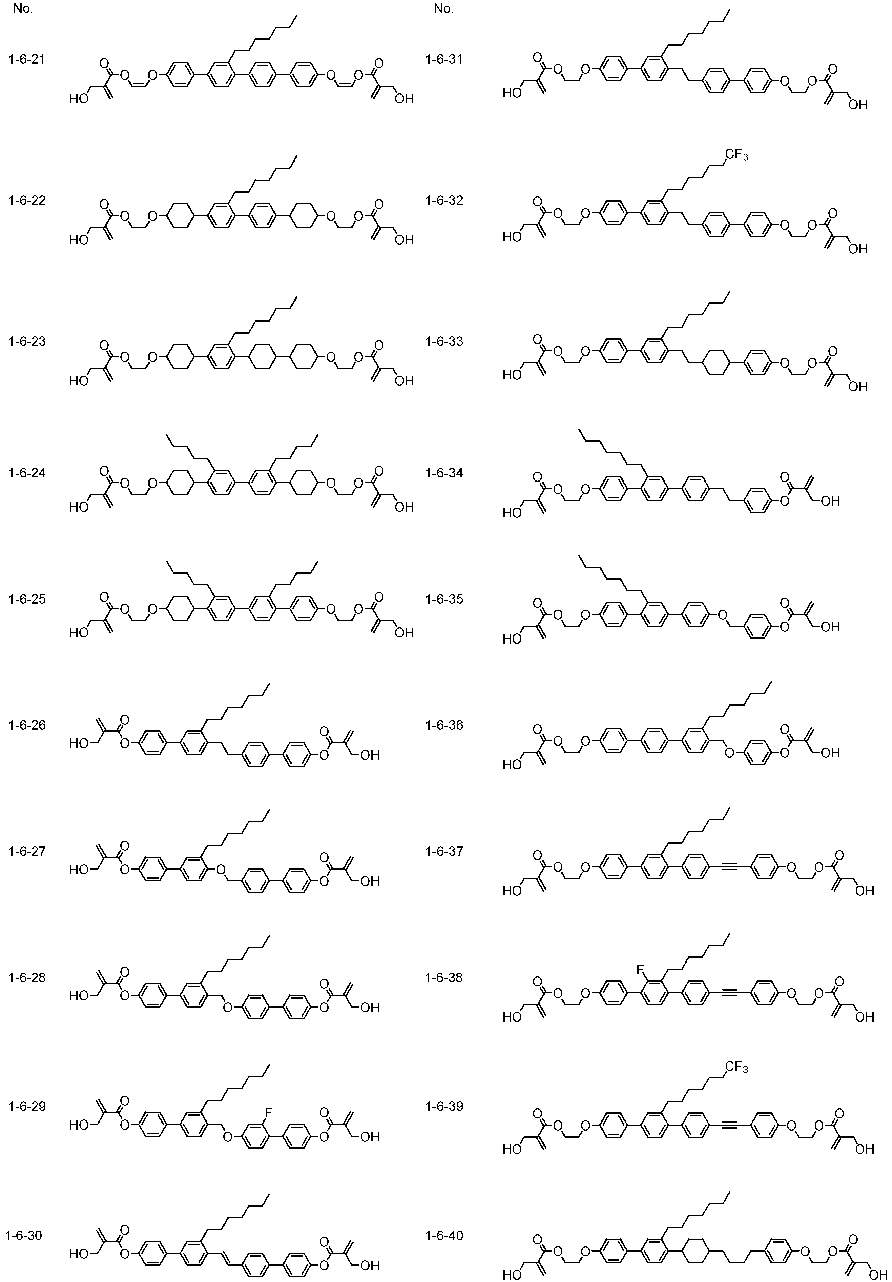

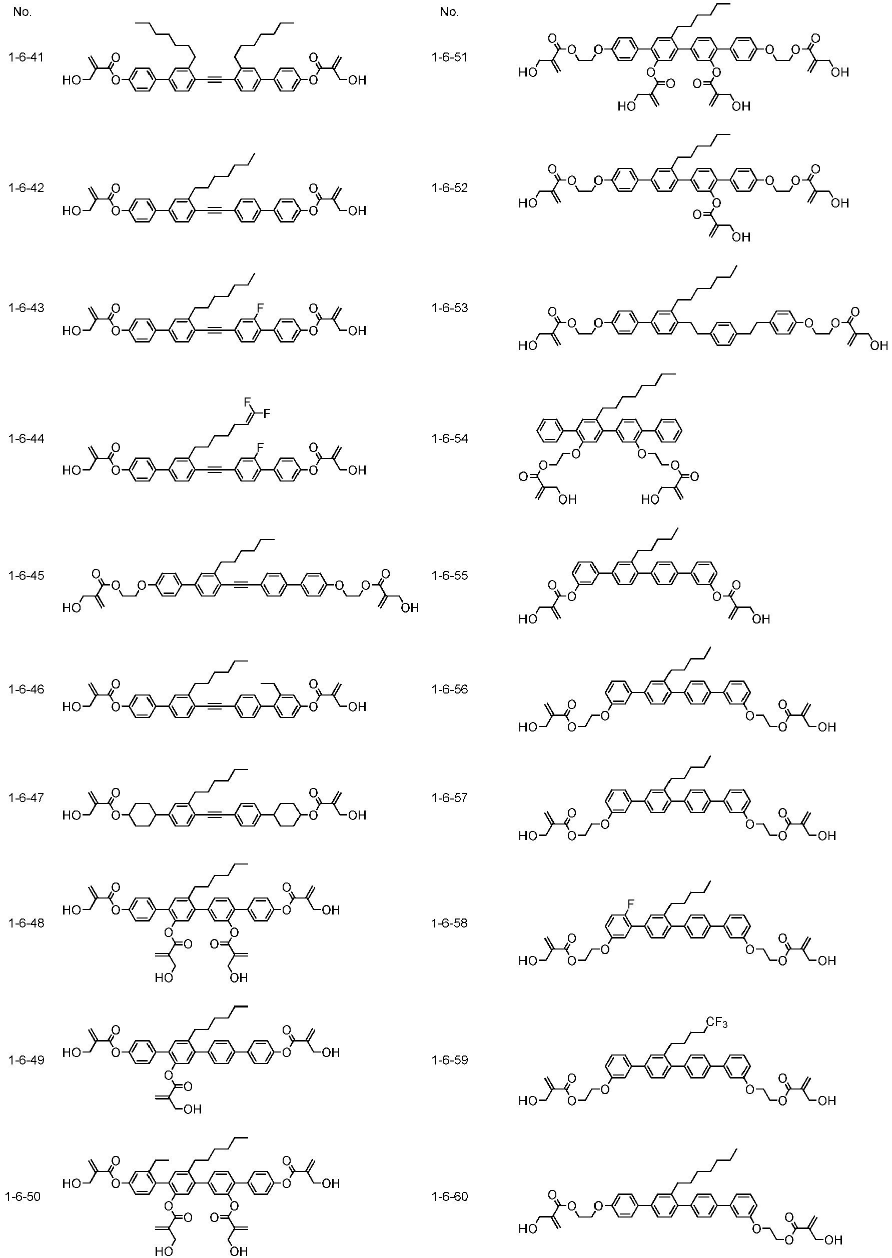

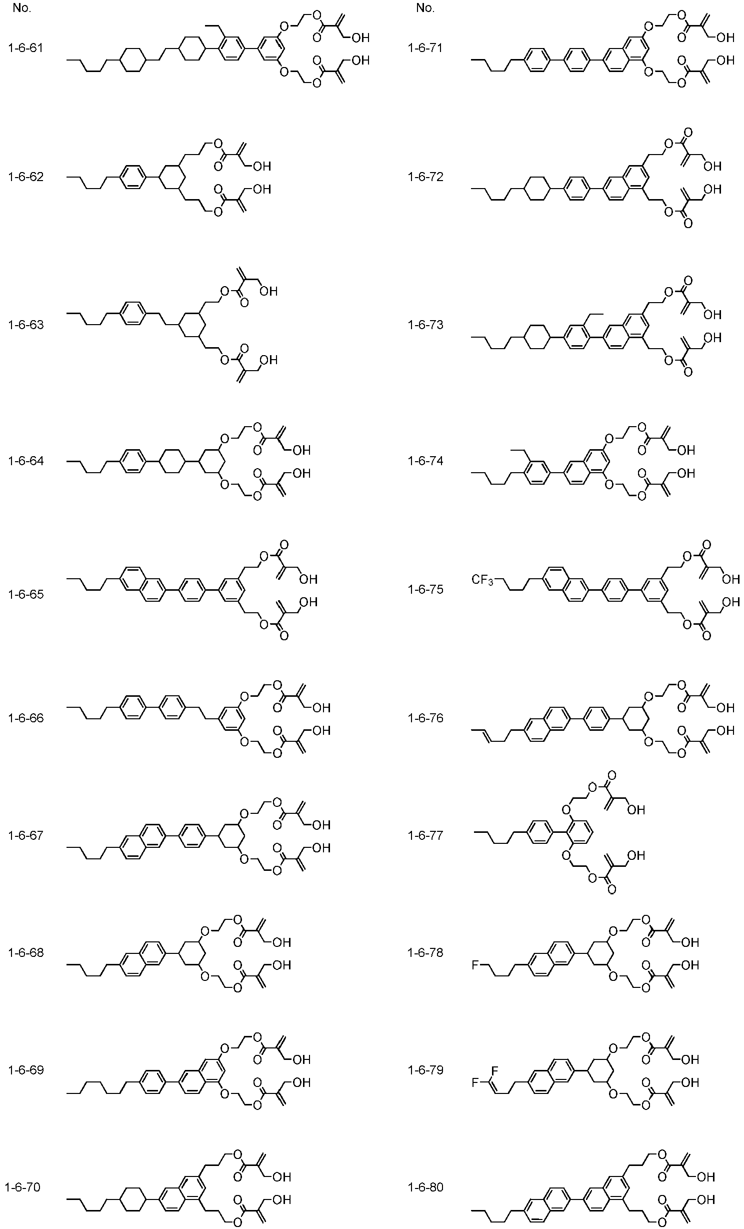

### 2. Examples of compositions

The compounds in Examples were represented by symbols based on the definition in Table 3 described below. In Table 3, a configuration of 1,4-cyclohexylene is trans. A parenthesized number next to a symbolized compound corresponds to the number of the compound. A symbol (-) means any other liquid crystal compound. A proportion (percentage) of the liquid crystal compound is expressed in terms of weight percent (% by weight) based on the weight of the liquid crystal composition. Values of the characteristics of the liquid crystal composition are summarized in a last part. Characteristics were measured according to the methods described above, and measured values were directly described (without extrapolation).

**Table 3 Method for description of compounds using symbols**

| R-(A₁)-Z₁-·····-Zₙ-(Aₙ)-R' | | | |
|---|---|---|---|
| 1) Left-terminal group R- | Symbol | 4) Ring structure -Aₙ- | Symbol |
| CₙH₂ₙ₊₁- | n- | 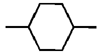 | H |
| CₙH₂ₙ₊₁O- | nO- | | |
| CₘH₂ₘ₊₁OCₙH₂ₙ- | mOn- | | |
| CH₂=CH- | V- | 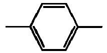 | B |
| CₙH₂ₙ₊₁-CH=CH- | nV- | | |
| CH₂=CH-CₙH₂ₙ- | Vn- | 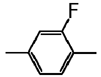 | B(F) |
| CₘH₂ₘ₊₁-CH=CH-CₙH₂ₙ- | mVn- | | |
| CF₂=CH- | VFF- | 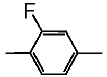 | B(2F) |
| CF₂=CH-CₙH₂ₙ- | VFFn- | | |
| 2) Right-terminal group -R' | Symbol | | |
| -CₙH₂ₙ₊₁ | -n | | |
| -OCₙH₂ₙ₊₁ | -On | 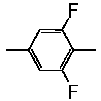 | B(F,F) |
| -COOCH₃ | -EMe | | |
| -CH=CH₂ | -V | | |
| -CH=CH-CₙH₂ₙ₊₁ | -Vn | 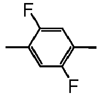 | B(2F,5F) |
| -CₙH₂ₙ-CH=CH₂ | -nV | | |
| -CₘH₂ₘ-CH=CH-CₙH₂ₙ₊₁ | -mVn | | |
| -CH=CF₂ | -VFF | 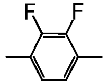 | B(2F,3F) |
| -F | -F | | |
| -Cl | -CL | | |
| -OCF₃ | -OCF3 | 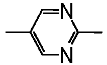 | Py |
| -OCF₂H | -OCF2H | | |
| -CF₃ | -CF3 | | |
| -OCH=CH-CF₃ | -OVCF3 | 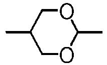 | |
| -C≡N | -C | | G |
| 3) Bonding group -Zₙ- | Symbol | | |
| -CₙH₂ₙ- | n | 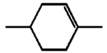 | ch |
| -COO- | E | | |
| -CH=CH- | V | | |
| -CH₂O- | 1O | | |
| -OCH₂- | O1 | | |
| -CF₂O- | X | | |
| -C≡C- | T | | |
| 5) Examples of description | | | |
| Example 1 3-HB-CL | | Example 2 5-HHBB(F,F)-F | |
| 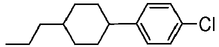 | | 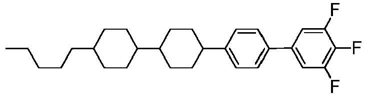 | |
| Example 3 3-HB-O2 | | Example 4 3-HBB(F,F)-F | |
| 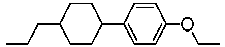 | | 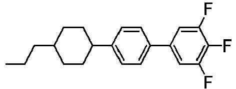 | |

### Use Example 1

| | |
|---|---|
| 5-HB-CL | 16% |
| 3-HH-4 | 11% |
| 3-HH-5 | 4% |
| 3-HHB-F | 3% |
| 3-HHB-CL | 3% |
| 4-HHB-CL | 4% |
| 3-HHB(F)-F | 9% |
| 4-HHB(F)-F | 10% |
| 5-HHB(F)-F | 9% |
| 7-HHB(F)-F | 8% |
| 5-HBB(F)-F | 4% |
| 1O1-HBBH-5 | 3% |
| 3-HHBB(F,F)-F | 3% |
| 4-HHBB(F,F)-F | 3% |
| 5-HHBB(F,F)-F | 3% |
| 3-HH2BB(F,F)-F | 4% |
| 4-HH2BB(F,F)-F | 3% |

To the composition described above, compound (1-2-7) described below was added in a proportion of 4% by weight. 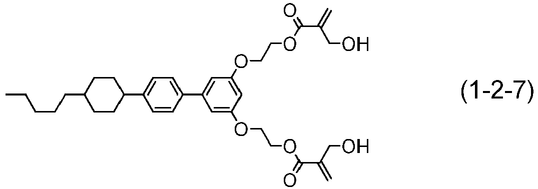 NI = 116.7°C; η = 20.6 mPa·s; Δn = 0.093; Δε = 4.0.

### Use Example 2

| | |
|---|---|
| 7-HB(F,F)-F | 3% |
| 3-HB-O2 | 7% |
| 2-HHB(F)-F | 10% |
| 3-HHB(F)-F | 10% |
| 5-HHB(F)-F | 9% |
| 2-HBB(F)-F | 9% |
| 3-HBB(F)-F | 10% |
| 5-HBB(F)-F | 15% |
| 2-HBB-F | 4% |
| 3-HBB-F | 5% |
| 5-HBB-F | 3% |
| 3-HBB(F,F)-F | 5% |
| 5-HBB(F,F)-F | 10% |

To the composition described above, compound (1-5-2) described below was added in a proportion of 3% by weight.

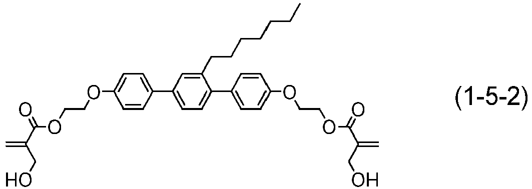

In addition, compound (RM-1) described below was added thereto in a proportion of 0.3% by weight. 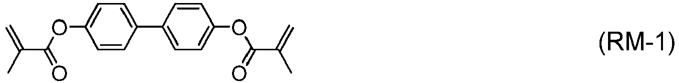 NI = 85.3°C; η = 24.9 mPa·s; Δn = 0.116; Δε = 5.8.

### Use example 3

| | |
|---|---|
| 3-HHB(F,F)-F | 9% |
| 3-H2HB(F,F)-F | 9% |
| 4-H2HB(F,F)-F | 8% |
| 5-H2HB(F,F)-F | 7% |
| 3-HBB(F,F)-F | 21% |
| 5-HBB(F,F)-F | 18% |
| 3-H2BB(F,F)-F | 12% |
| 5-HHBB(F,F)-F | 3% |
| 5-HHEBB-F | 2% |
| 3-HH2BB(F,F)-F | 3% |
| 1O1-HBBH-4 | 5% |
| 1O1-HBBH-5 | 3% |

To the composition described above, compound (1-2-8) described below was added in a proportion of 1% by weight. 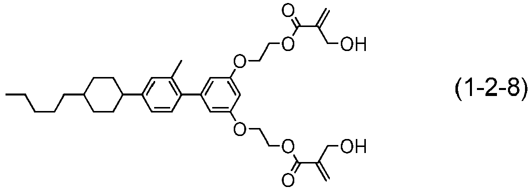 NI = 97.2°C; η = 34.9 mPa·s; Δn = 0.116; Δε = 9.1.

### Use example 4

| | |
|---|---|
| 5-HB-CL | 16% |
| 7-HB(F,F)-F | 4% |
| 3-HH-4 | 11% |
| 3-HH-5 | 5% |
| 3-HB-O2 | 14% |
| 3-HHB-1 | 7% |
| 3-HHB-O1 | 6% |
| 2-HHB(F)-F | 7% |
| 3-HHB(F)-F | 7% |
| 5-HHB(F)-F | 7% |
| 3-HHB(F,F)-F | 6% |
| 3-H2HB(F,F)-F | 5% |
| 4-H2HB(F,F)-F | 5% |

To the composition described above, compound (1-2-48) described below was added in a proportion of 3% by weight. 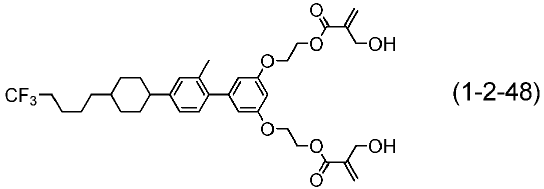 NI = 71.0°C; η = 13.7 mPa·s; Δn = 0.073; Δε = 2.8.

### Use example 6

| | |
|---|---|
| 5-HB-CL | 3% |
| 7-HB(F)-F | 7% |
| 3-HH-4 | 9% |
| 3-HH-EMe | 22% |
| 3-HHEB-F | 8% |
| 5-HHEB-F | 7% |
| 3-HHEB(F,F)-F | 10% |
| 4-HHEB(F,F)-F | 6% |
| 4-HGB(F,F)-F | 6% |
| 5-HGB(F,F)-F | 6% |
| 2-H2GB(F,F)-F | 4% |
| 3-H2GB(F,F)-F | 6% |
| 5-GHB(F,F)-F | 6% |

To the composition described above, compound (1-2-127) described below was added in a proportion of 0.5% by weight. 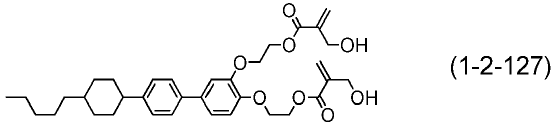 NI = 78.7°C; η = 19.9 mPa·s; Δn = 0.064; Δε = 5.8.

### Use example 7

| | |
|---|---|
| 1V2-BEB(F,F)-C | 7% |
| 3-HB-C | 18% |
| 2-BTB-1 | 10% |
| 5-HH-VFF | 30% |
| 3-HHB-1 | 5% |
| VFF-HHB-1 | 6% |
| VFF2-HHB-1 | 11% |
| 3-H2BTB-2 | 5% |
| 3-H2BTB-3 | 5% |
| 3-H2BTB-4 | 3% |

To the composition described above, compound (1-6-77) described below was added in a proportion of 5% by weight. 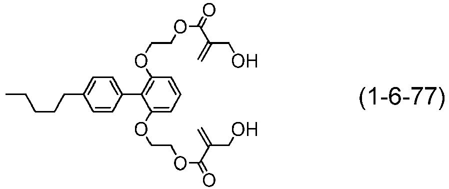 NI = 80.3°C; η = 12.1 mPa·s; Δn = 0.130; Δε = 7.2.

### Use example 8

| | |
|---|---|
| 5-HB-F | 12% |
| 6-HB-F | 9% |
| 7-HB-F | 7% |
| 2-HHB-OCF3 | 5% |
| 3-HHB-OCF3 | 7% |
| 4-HHB-OCF3 | 7% |
| 5-HHB-OCF3 | 5% |
| 3-HH2B-OCF3 | 7% |
| 5-HH2B-OCF3 | 4% |
| 3-HHB(F,F)-OCF2H | 4% |
| 3-HHB(F,F)-OCF3 | 5% |
| 3-HH2B(F)-F | 3% |
| 3-HBB(F)-F | 11% |
| 5-HBB(F)-F | 8% |
| 5-HBBH-3 | 3% |
| 3-HB(F)BH-3 | 3% |

To the composition described above, compound (1-3-38) described below was added in a proportion of 2% by weight. 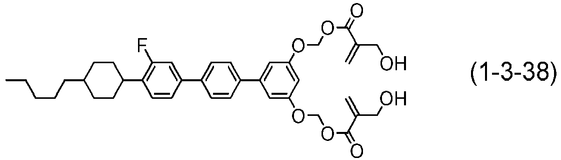 NI = 85.9°C; η = 14.7 mPa·s; Δn = 0.092; Δε = 4.4.

### Use example 9

| | |
|---|---|
| 3-HB-O2 | 10% |
| 5-HB-CL | 13% |
| 3-HBB(F,F)-F | 7% |
| 3-PyB(F)-F | 10% |
| 5-PyB(F)-F | 10% |
| 3-PyBB-F | 11% |
| 4-PyBB-F | 10% |
| 5-PyBB-F | 10% |
| 5-HBB(F)B-2 | 9% |
| 5-HBB(F)B-3 | 10% |

To the composition described above, compound (1-2-7) described below was added in a proportion of 4% by weight. 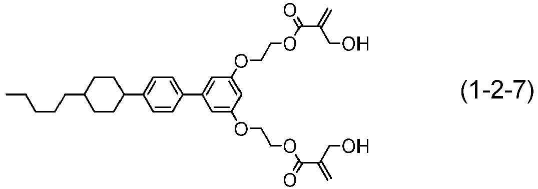 NI = 98.5°C; η = 39.6 mPa·s; Δn = 0.190; Δε = 8.1.

### Use example 10

| | |
|---|---|
| 3-HB-CL | 6% |
| 5-HB-CL | 4% |
| 3-HHB-OCF3 | 5% |
| 3-H2HB-OCF3 | 5% |
| 5-H4HB-OCF3 | 15% |
| V-HHB(F)-F | 5% |
| 3-HHB(F)-F | 6% |
| 5-HHB(F)-F | 5% |
| 3-H4HB(F,F)-CF3 | 9% |
| 5-H4HB(F,F)-CF3 | 9% |
| 5-H2HB(F,F)-F | 5% |
| 5-H4HB(F,F)-F | 7% |
| 2-H2BB(F)-F | 5% |
| 3-H2BB(F)-F | 9% |
| 3-HBEB(F,F)-F | 5% |

To the composition described above, compound (1-5-2) described below was added in a proportion of 3% by weight. 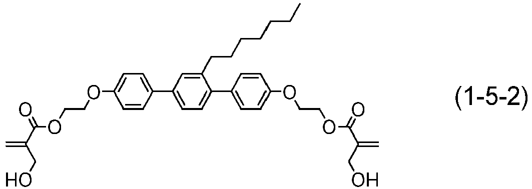 NI = 70.1°C; η = 25.3 mPa·s; Δn = 0.097; Δε = 8.3.

### Use example 11

| | |
|---|---|
| 5-HB-CL | 9% |
| 3-HH-4 | 9% |
| 3-HHB-1 | 4% |
| 3-HHB(F,F)-F | 8% |
| 3-HBB(F,F)-F | 19% |
| 5-HBB(F,F)-F | 13% |
| 3-HHEB(F,F)-F | 9% |
| 4-HHEB(F,F)-F | 5% |
| 5-HHEB(F,F)-F | 4% |
| 2-HBEB(F,F)-F | 5% |
| 3-HBEB(F,F)-F | 4% |
| 5-HBEB(F,F)-F | 5% |
| 3-HHBB(F,F)-F | 6% |

To the composition described above, compound (1-2-8) described below was added in a proportion of 1% by weight.

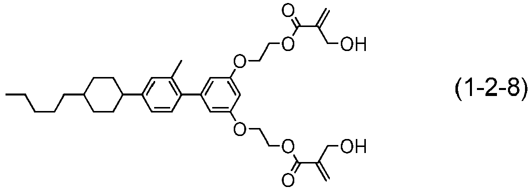

In addition, compound (RM-2) described below was added thereto in a proportion of 0.3% by weight. 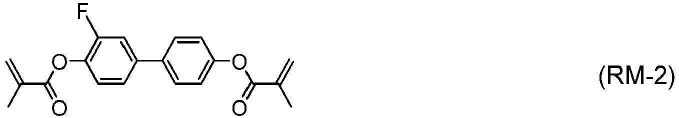 NI = 81.5°C; η = 23.6 mPa·s; Δn = 0.102; Δε = 9.1.

### Use example 12

| | |
|---|---|
| 2-HB-C | 5% |
| 3-HB-C | 15% |
| 3-HB-O2 | 12% |
| 2-BTB-1 | 3% |
| 3-HHB-F | 4% |
| 3-HHB-1 | 4% |
| 3-HHB-O1 | 6% |
| 3-HHB-3 | 14% |
| 3-HHEB-F | 5% |
| 5-HHEB-F | 5% |
| 2-HHB(F)-F | 7% |
| 3-HHB(F)-F | 7% |
| 5-HHB(F)-F | 7% |
| 3-HHB(F,F)-F | 6% |

To the composition described above, compound (1-1-15) described below was added in a proportion of 2% by weight. 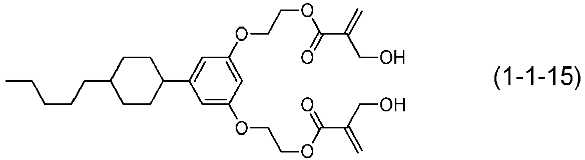 NI = 102.1°C; η = 20.2 mPa·s; Δn = 0.102; Δε = 5.1.

### Use example 13

| | |
|---|---|
| 5-HB-CL | 16% |
| 3-HH-4 | 12% |
| 3-HH-5 | 4% |
| 3-HHB-F | 3% |
| 3-HHB-CL | 3% |
| 4-HHB-CL | 4% |
| 3-HHB(F)-F | 9% |
| 4-HHB(F)-F | 9% |
| 5-HHB(F)-F | 10% |
| 7-HHB(F)-F | 10% |
| 5-HBB(F)-F | 3% |
| 1O1-HBBH-5 | 3% |
| 3-HHBB(F,F)-F | 2% |
| 4-HHBB(F,F)-F | 3% |
| 5-HHBB(F,F)-F | 3% |
| 3-HH2BB(F,F)-F | 3% |
| 4-HH2BB(F,F)-F | 3% |

To the composition described above, compound (1-6-61) described below was added in a proportion of 0.5% by weight. 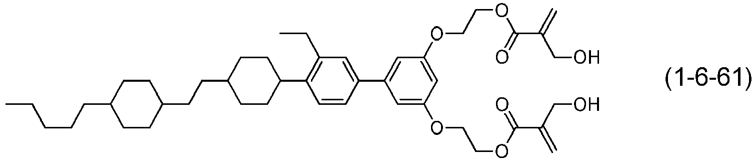 NI = 114.0°C; η = 19.0 mPa·s; Δn = 0.090; Δε = 3.8.

### Use example 14

| | |
|---|---|
| 5-HB-CL | 17% |
| 7-HB(F,F)-F | 3% |
| 3-HH-4 | 10% |
| 3-HH-5 | 8% |
| 3-HB-O2 | 15% |
| 3-HHB-1 | 8% |
| 3-HHB-O1 | 5% |
| 2-HHB(F)-F | 5% |
| 3-HHB(F)-F | 7% |
| 5-HHB(F)-F | 5% |
| 3-HHB(F,F)-F | 6% |
| 3-H2HB(F,F)-F | 4% |
| 4-H2HB(F,F)-F | 7% |

To the composition described above, compound (1-6-62) described below was added in a proportion of 3% by weight. 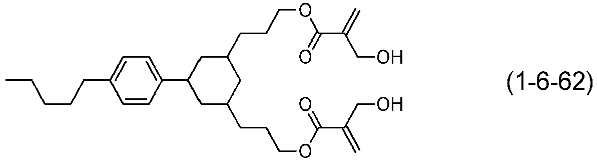 NI = 70.2°C; η = 12.6 mPa·s; Δn = 0.073; Δε = 2.6.

### Use example 15

| | |
|---|---|
| 5-HB-F | 12% |
| 6-HB-F | 9% |
| 7-HB-F | 7 % |
| 2-HHB-OCF3 | 4% |
| 3-HHB-OCF3 | 8% |
| 4-HHB-OCF3 | 7% |
| 5-HHB-OCF3 | 6% |
| 3-HH2B-OCF3 | 3% |
| 5-HH2B-OCF3 | 4% |
| 3-HHB(F,F)-OCF2H | 6% |
| 3-HHB(F,F)-OCF3 | 5% |
| 3-HH2B(F)-F | 3% |
| 3-HBB(F)-F | 10% |
| 5-HBB(F)-F | 10% |
| 5-HBBH-3 | 3% |
| 3-HB(F)BH-3 | 3% |

To the composition described above, compound (1-6-63) described below was added in a proportion of 5% by weight. 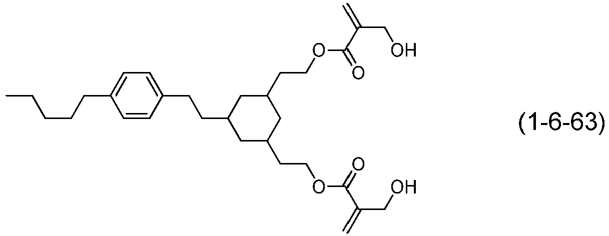 NI = 85.5°C; η = 15.5 mPa·s; Δn = 0.092; Δε = 4.6.

### Use example 16

| | |
|---|---|
| 7-HB(F,F)-F | 3% |
| 3-HB-O2 | 7% |
| 2-HHB(F)-F | 10% |
| 3-HHB(F)-F | 10% |
| 5-HHB(F)-F | 10% |
| 2-HBB(F)-F | 10% |
| 3-HBB(F)-F | 10% |
| 5-HBB(F)-F | 14% |
| 2-HBB-F | 4% |
| 3-HBB-F | 4% |
| 5-HBB-F | 4% |
| 3-HBB(F,F)-F | 4% |
| 5-HBB(F,F)-F | 10% |

To the composition described above, compound (1-6-64) described below was added in a proportion of 1% by weight. 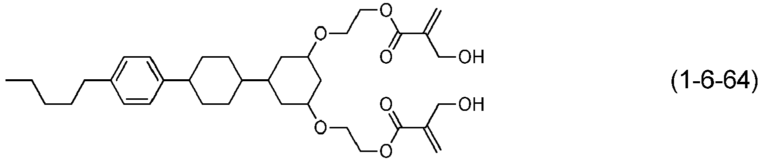 NI = 85.6°C; η = 24.9 mPa·s; Δn = 0.115; Δε = 5.7.

### Use example 17

| | |
|---|---|
| 3-HB-CL | 4% |
| 5-HB-CL | 6% |
| 3-HHB-OCF3 | 5% |
| 3-H2HB-OCF3 | 5% |
| 5-H4HB-OCF3 | 15% |
| V-HHB(F)-F | 5% |
| 3-HHB(F)-F | 3% |
| 5-HHB(F)-F | 6% |
| 3-H4HB(F,F)-CF3 | 8% |
| 5-H4HB(F,F)-CF3 | 10% |
| 5-H2HB(F,F)-F | 5% |
| 5-H4HB(F,F)-F | 7% |
| 2-H2BB(F)-F | 5% |
| 3-H2BB(F)-F | 11% |
| 3-HBEB(F,F)-F | 5% |

To the composition described above, compound (1-6-66) described below was added in a proportion of 3% by weight. 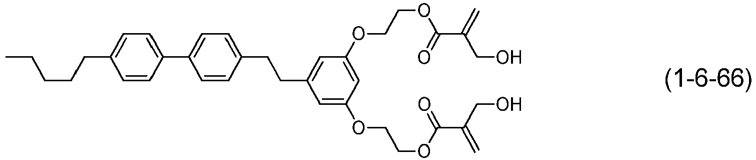 NI = 70.0°C; η = 25.4 mPa·s; Δn = 0.097; Δε = 8.3.

### Use example 18

| | |
|---|---|
| 5-HB-CL | 4% |
| 7-HB(F)-F | 5% |
| 3-HH-4 | 10% |
| 3-HH-5 | 10% |
| 3-HB-O2 | 12% |
| 3-HHEB-F | 9% |
| 5-HHEB-F | 9% |
| 3-HHEB(F,F)-F | 9% |
| 4-HHEB(F,F)-F | 5% |
| 3-GHB(F,F)-F | 6% |
| 4-GHB(F,F)-F | 6% |
| 5-GHB(F,F)-F | 5% |
| 2-HHB(F,F)-F | 5% |
| 3-HHB(F,F)-F | 5% |

To the composition described above, compound (1-6-65) described below was added in a proportion of 4% by weight. 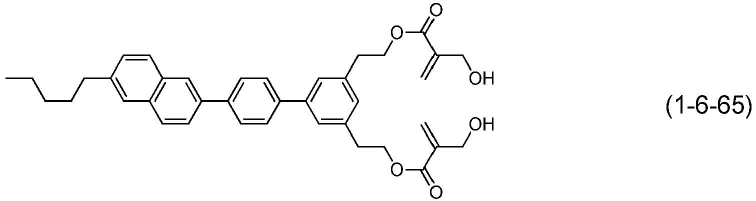 NI = 74.4°C; η = 18.6 mPa·s; Δn = 0.068; Δε = 5.8.

### Industrial Applicability

A liquid crystal composition containing compound (1) can be used in a display device such as a liquid crystal projector and a liquid crystal television.

## Claims

1. A compound, represented by any one of formula (1-7) to formula (1-34): 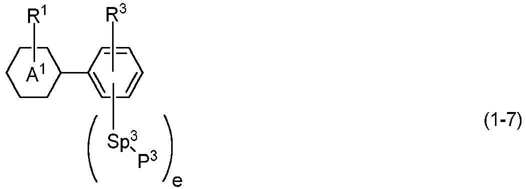 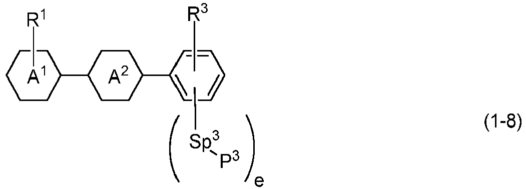 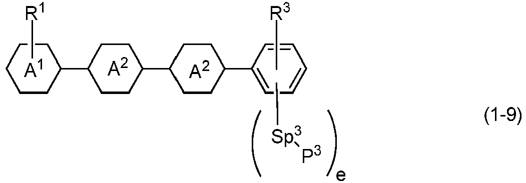 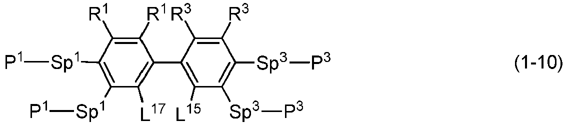 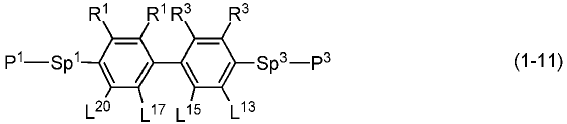 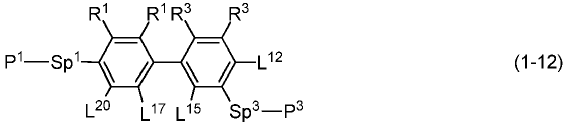 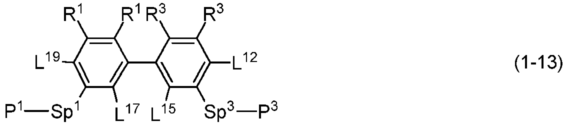 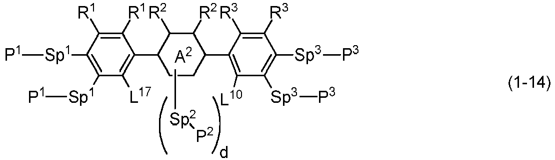 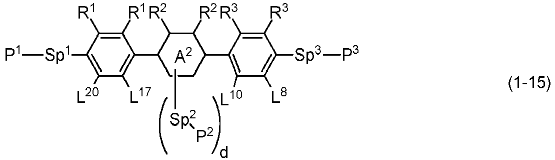 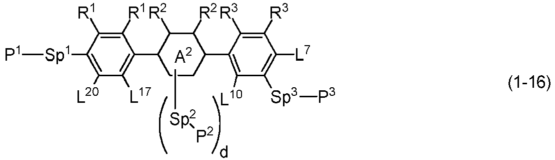 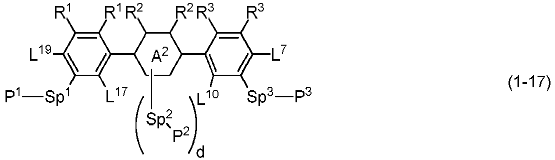 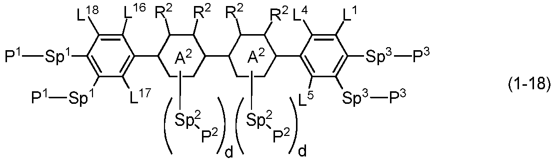 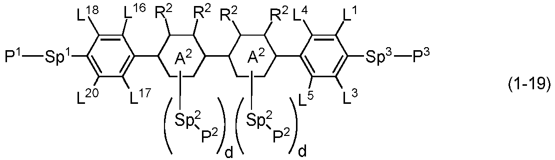 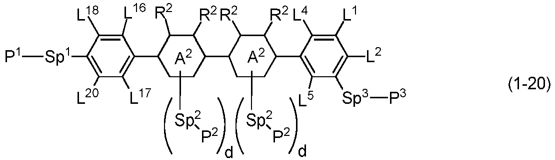 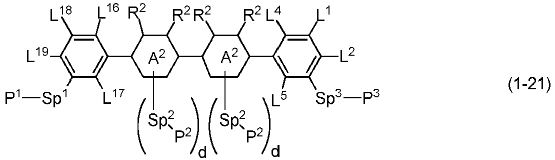
wherein, in formula (1-7) to formula (1-21),
R¹, R² and R³ are independently hydrogen, alkyl having 1 to 8 carbons, alkenyl having 2 to 8 carbons, alkoxy having 1 to 7 carbons or alkenyloxy having 2 to 7 carbons;
ring A¹ is cyclohexyl, cyclohexenyl or phenyl, and ring A² is 1,4-cyclohexylene, 1,4-cyclohexenylene or 1,4-phenylene, and in the rings, at least one hydrogen may be replaced by fluorine;
L¹, L², L³, L⁴, L⁵, L⁷, L⁸, L¹⁰, L¹², L¹³, L¹⁵, L¹⁶, L¹⁷, L¹⁸, L¹⁹ and L²⁰ are independently hydrogen, fluorine, methyl or ethyl;
Sp¹, Sp² and Sp³ are independently a single bond or alkylene having 1 to 5 carbons, and in the alkylene, at least one piece of -CH₂- may be replaced by -O-;
c, d and e are independently 0, 1 or 2, and a sum of c, d and e is 2, 3 or 4; and
P¹, P² and P³ are independently a polymerizable group represented by formula (P-11); 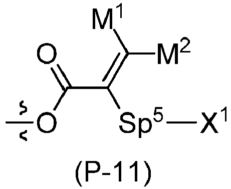
wherein, in formula (P-11), M¹ and M² are independently hydrogen, fluorine, methyl, ethyl or trifluoromethyl; Sp⁵ is a single bond or alkylene having 1 to 5 carbons, and in the alkylene, at least one piece of -CH₂- may be replaced by -O-; and X¹ is -OH or -NH₂; 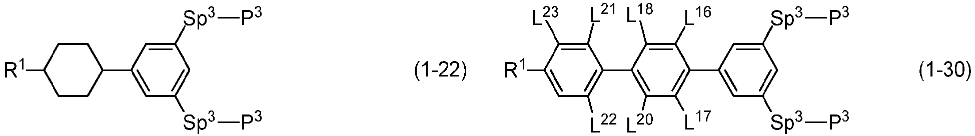 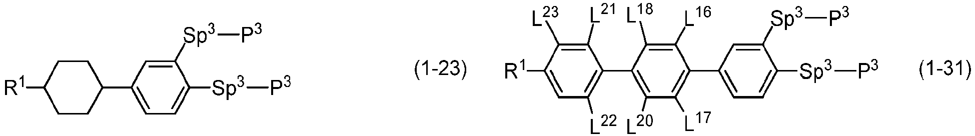 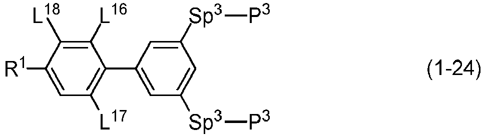 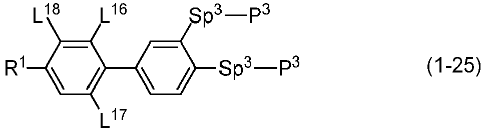 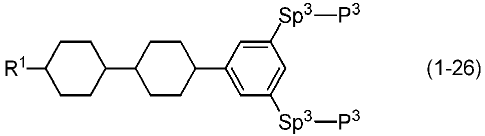 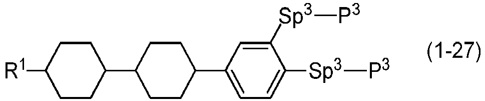 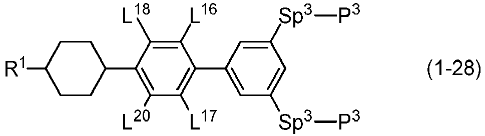 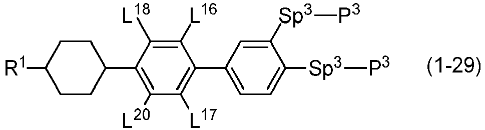 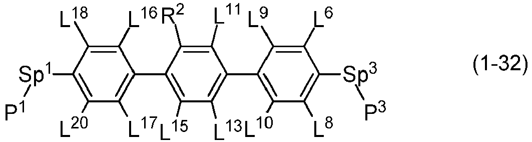 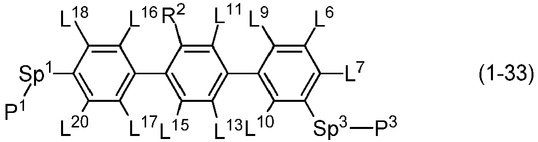 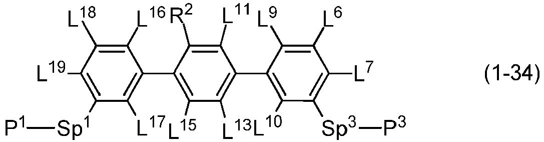
wherein, in formula (1-22) to formula (1-34),
R¹ and R² are independently alkyl having 1 to 7 carbons, alkenyl having 2 to 7 carbons, alkoxy having 1 to 6 carbons or alkenyloxy having 2 to 6 carbons;
L⁶, L⁷, L⁸, L⁹, L¹⁰, L¹¹, L¹³, L¹⁵, L¹⁶, L¹⁷, L¹⁸, L¹⁹, L²⁰, L²¹, L²² and L²³ are independently hydrogen, fluorine, methyl or ethyl;
Sp¹ and Sp³ are independently a single bond or alkylene having 1 to 3 carbons, and in the alkylene, at least one piece of -CH₂- may be replaced by -O-; and
P¹ and P³ are independently a polymerizable group represented by formula (P-111); 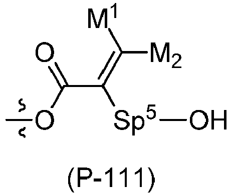
wherein, in formula (P-111), M¹ and M² are independently hydrogen, fluorine or methyl; and Sp⁵ is a single bond or alkylene having 1 to 3 carbons, and in the alkylene, at least one piece of -CH₂- may be replaced by -O-.

2. The compound according to claim 1, which is represented by any one of formula (1-7) to formula (1-21).

3. The compound according to claim 2, wherein, in formula (1-7) to formula (1-21), R¹, R² and R³ are independently hydrogen, alkyl having 1 to 8 carbons, alkenyl having 2 to 8 carbons, alkoxy having 1 to 7 carbons or alkenyloxy having 2 to 7 carbons;
ring A¹ is cyclohexyl, cyclohexenyl or phenyl, and ring A² is 1,4-cyclohexylene, 1,4-cyclohexenylene or 1,4-phenylene, and in the rings, at least one hydrogen may be replaced by fluorine;
L¹, L², L³, L⁴, L⁵, L⁷, L⁸, L¹⁰, L¹², L¹³, L¹⁵, L¹⁶, L¹⁷, L¹⁸, L¹⁹ and L²⁰ are independently hydrogen, fluorine, methyl or ethyl;
Sp¹, Sp² and Sp³ are independently a single bond or alkylene having 1 to 5 carbons, and in the alkylene, at least one piece of -CH₂- may be replaced by -O-;
c, d and e are independently 0, 1 or 2, and a sum of c, d and e is 2, 3 or 4; and
P¹, P² and P³ are independently a polymerizable group represented by formula (P-11); 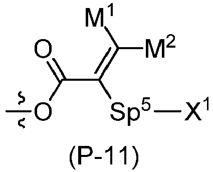 wherein, in formula (P-11), M¹ and M² are independently hydrogen, fluorine, methyl or ethyl; Sp⁵ is a single bond or alkylene having 1 to 5 carbons, and in the alkylene, at least one piece of -CH₂- may be replaced by -O-; and X¹ is -OH or -NH₂.

4. The compound according to claim 1 or 2, wherein, in formula (1-7) to formula (1-21), X¹ is -OH.

5. The compound according to claim 1, which is represented by any one of formula (1-22) to formula (1-34).

6. A liquid crystal composition, containing at least one compound according to any one of claims 1 to 5 as component A and at least one compound selected from the group of compounds represented by formula (2) to formula (7) as component B: 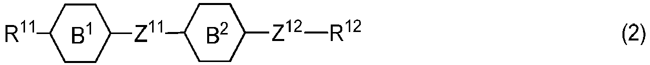 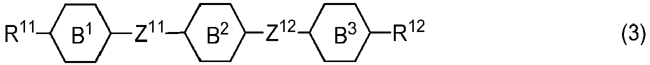 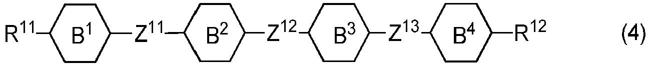 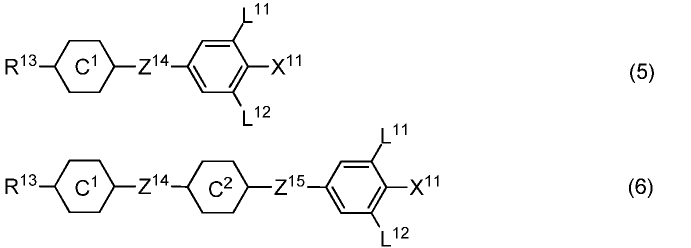 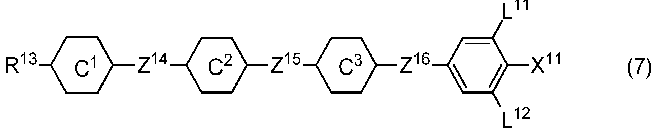
wherein, in formula (2) to formula (4),
R¹¹ and R¹² are independently, alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹ ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -CH₂CH₂-, -CH=CH-, -C≡C- or -COO-;
wherein, in formula (5) to formula (7),
R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C², and ring C³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁴, Z¹⁵ and Z¹⁶ are independently a single bond, -CH₂CH₂-, -CH=CH-, -C≡C-, -COO-, -CF₂O-, -OCF₂- -CH₂O- or -(CH₂)₄-; and
L¹¹ and L¹² are independently hydrogen or fluorine.

7. The liquid crystal composition according to claim 6, further containing at least one compound selected from the group of compounds represented by formula (9) to formula (15) as component E: 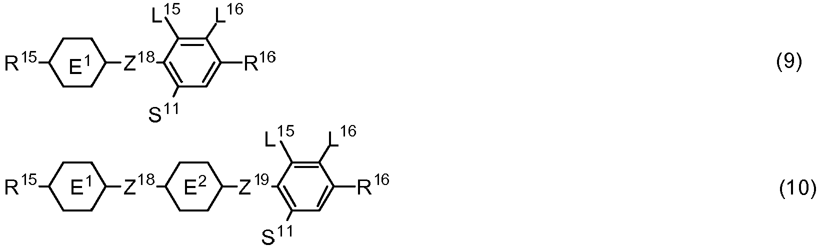 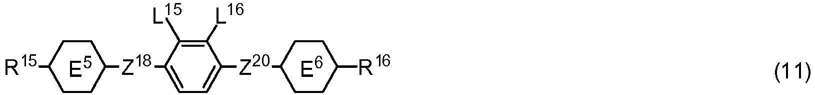 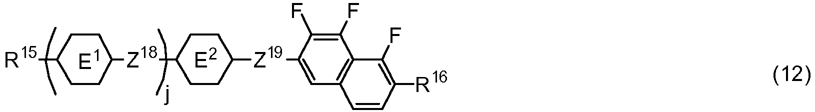 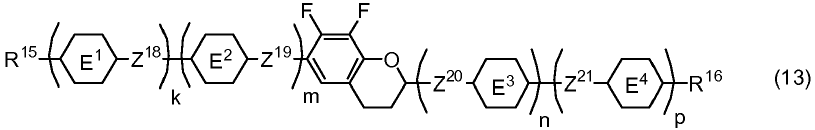 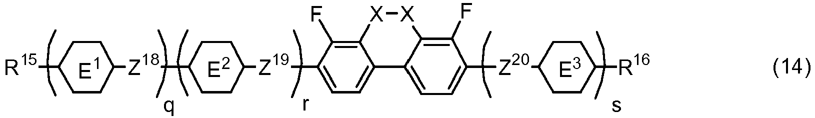 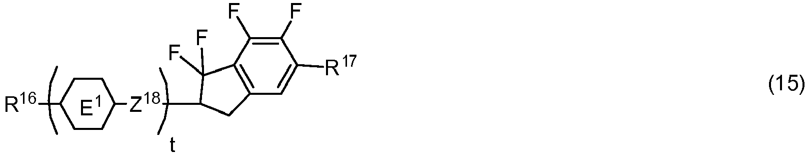 wherein, in formula (9) to formula (15),
R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine;
ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
Z¹⁸, Z¹⁹, Z²⁰ and Z²¹ are independently a single bond, -CH₂CH₂-, -COO-, -CH₂O-, -OCF₂- or -OCF₂CH₂CH₂-;
L¹⁵ and L¹⁶ are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

8. The liquid crystal composition according to claim 6 or 7, further containing at least one compound selected from the group of polymerizable compounds represented by formula (16) as component F: 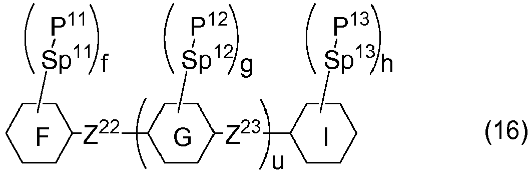 wherein, in formula (16),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
rings G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C(CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

9. The liquid crystal composition according to claim 8, wherein component F is at least one compound selected from the group of polymerizable compounds represented by formula (16-1) to formula (16-7): 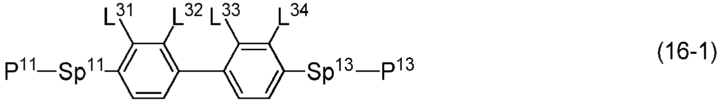 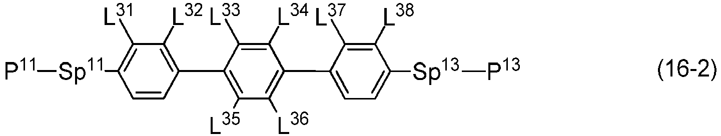 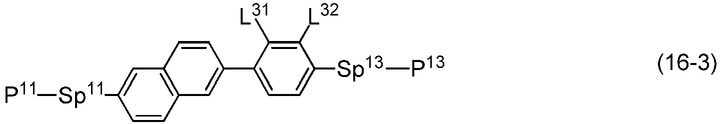 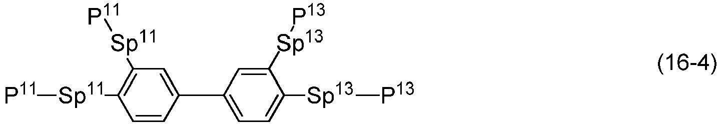 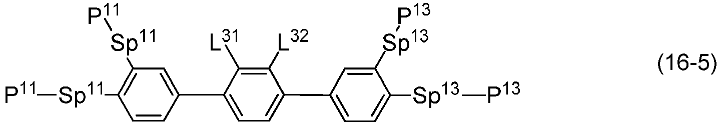 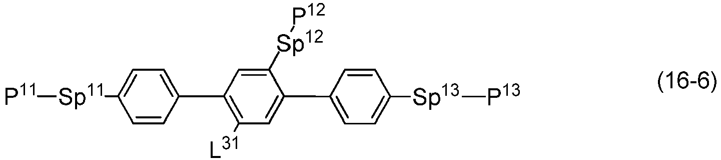 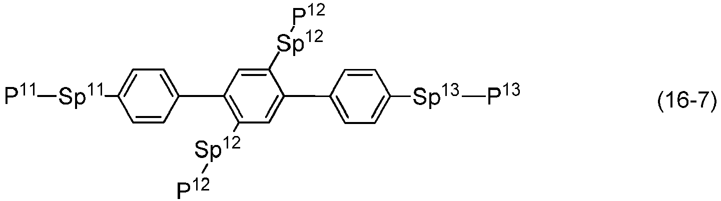 wherein, in formula (16-1) to formula (16-7), P¹¹, P¹² and P¹³ are independently a group selected from the group of polymerizable groups represented by formula (P-2) to formula (P-4), in which M¹¹, M¹² and M¹³ are independently hydrogen, fluorine, alkyl having 1 to 5 carbons, or alkyl having 1 to 5 carbons in which at least one hydrogen is replaced by halogen; 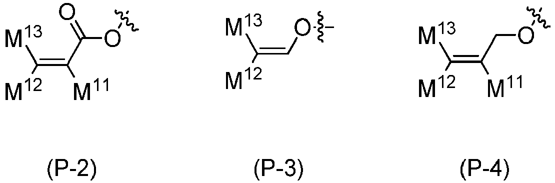 wherein, L³¹, L³², L³³, L³⁴, L³⁵, L³⁶, L³⁷ and L³⁸ are independently hydrogen, fluorine or methyl; and Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine.

10. The liquid crystal composition according to any one of claims 6 to 9, further containing at least one of a polymerizable compound different from the compounds represented by formula (1) and formula (16), a polymerization initiator, a polymerization inhibitor, an optically active compound, an antioxidant, an ultraviolet light absorber, a light stabilizer, a heat stabilizer and an antifoaming agent.

11. A liquid crystal display device, including at least one liquid crystal composition according to any one of claims 6 to 10.

## Patentansprüche

1. Verbindung, die dargestellt ist durch irgendeine aus Formel (1-7) bis Formel (1-34): 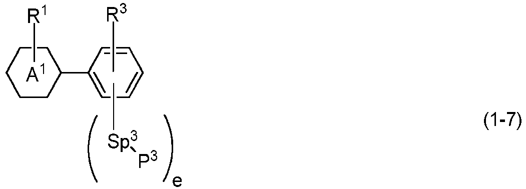 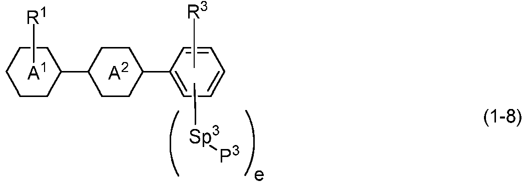 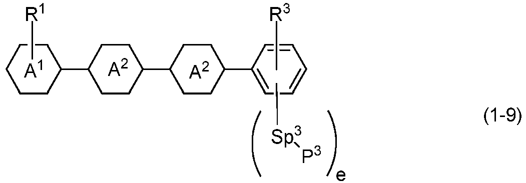 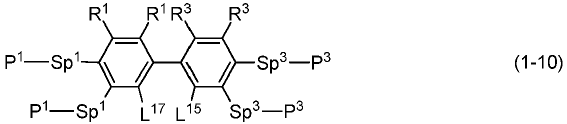 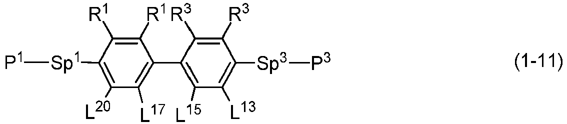 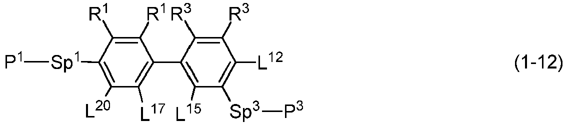 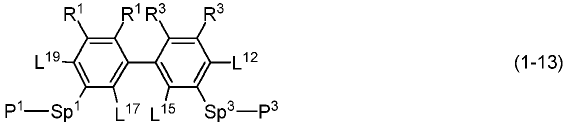 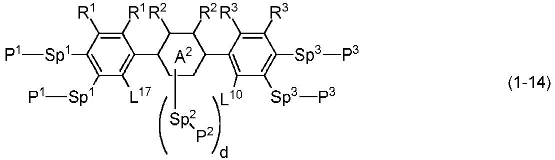 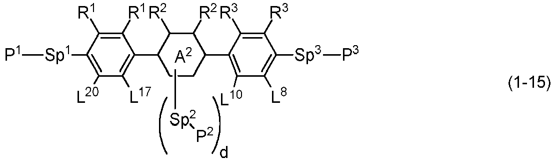 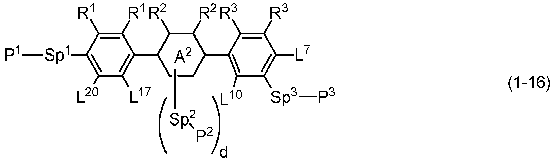 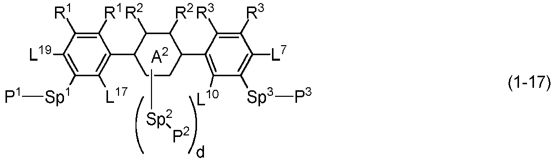 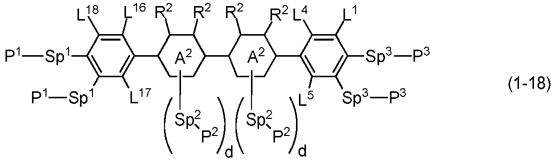 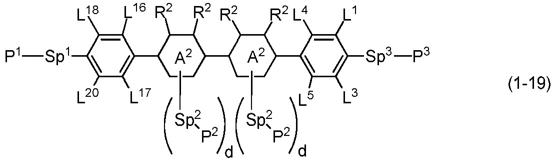 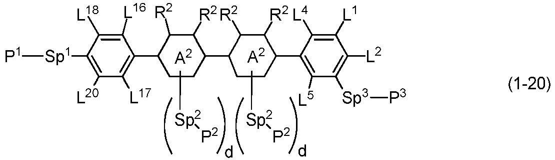 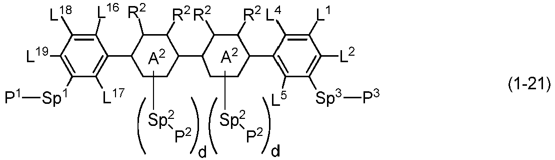
wobei in Formel (1-7) bis Formel (1-21)
R¹, R² und R³ sind unabhängig Wasserstoff, Alkyl mit 1 bis 8 Kohlenstoffen, Alkenyl mit 2 bis 8 Kohlenstoffen, Alkoxy mit 1 bis 7 Kohlenstoffen oder Alkenyloxy mit 2 bis 7 Kohlenstoffen;
Ring A¹ ist Cyclohexyl, Cyclohexenyl oder Phenyl, und Ring A² ist 1,4-Cyclohexylen, 1,4-Cyclohexenylen oder 1,4-Phenylen, und in den Ringen kann mindestens ein Wasserstoff durch Fluor ersetzt sein;
L¹, L², L³, L⁴, L⁵, L⁷, L⁸, L¹⁰, L¹², L¹³, L¹⁵, L¹⁶, L¹⁷, L¹⁸, L¹⁹ und L²⁰ sind unabhängig Wasserstoff, Fluor, Methyl oder Ethyl;
Sp¹, Sp² und Sp³ sind unabhängig eine Einfachbindung oder Alkylen mit 1 bis 5 Kohlenstoffen und in dem Alkylen kann mindestens ein Abschnitt -CH₂- durch -O- ersetzt sein;
c, d und e sind unabhängig 0, 1 oder 2 und eine Summe aus c, d und e ist 2, 3 oder 4; und
P¹, P² und P³ sind unabhängig eine durch die Formel (P-11) dargestellte polymerisierbare Gruppe; 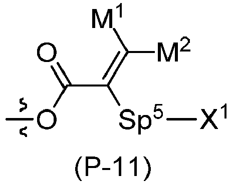
wobei in Formel (P-11) M¹ und M² unabhängig Wasserstoff, Fluor, Methyl, Ethyl oder Trifluormethyl sind; Sp⁵ eine Einfachbindung oder ein Alkylen mit 1 bis 5 Kohlenstoffen ist und in dem Alkylen kann mindestens ein Abschnitt -CH₂- durch -O- ersetzt sein; und X¹ ist -OH oder -NH₂; 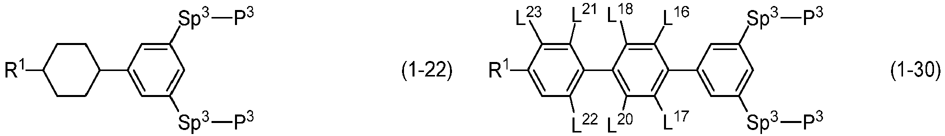 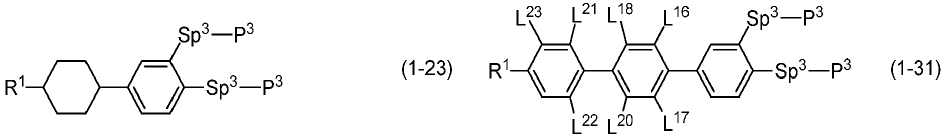 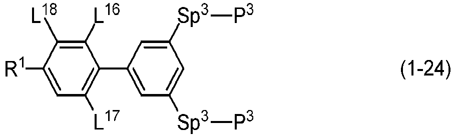 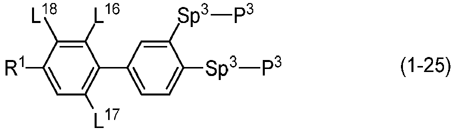 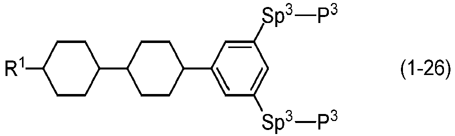 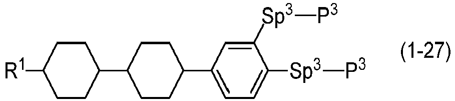 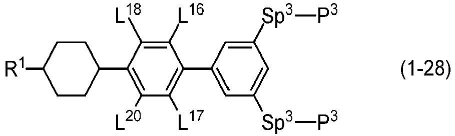 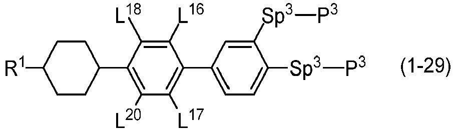 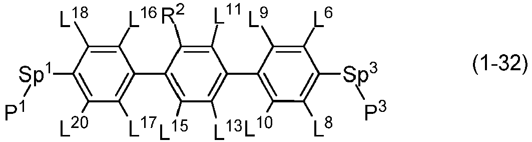 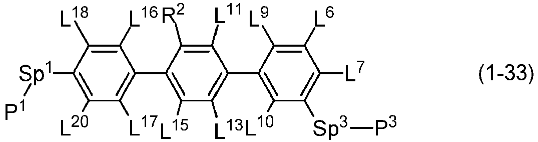 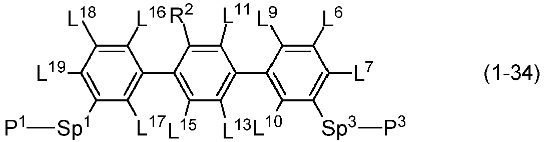
wobei in Formel (1-22) bis Formel (1-34)
R¹ und R² sind unabhängig Alkyl mit 1 bis 7 Kohlenstoffen, Alkenyl mit 2 bis 7 Kohlenstoffen, Alkoxy mit 1 bis 6 Kohlenstoffen oder Alkenyloxy mit 2 bis 6 Kohlenstoffen;
L⁶, L⁷, L⁸, L⁹, L¹⁰, L¹¹, L¹³, L¹⁵, L¹⁶, L¹⁷, L¹⁸, L¹⁹, L²⁰, L²¹, L²² und L²³ sind unabhängig Wasserstoff, Fluor, Methyl oder Ethyl;
Sp¹ und Sp³ sind unabhängig eine Einfachbindung oder Alkylen mit 1 bis 3 Kohlenstoffen und in dem Alkylen kann mindestens ein Abschnitt -CH₂- durch -O- ersetzt sein; und
P¹ und P³ sind unabhängig eine durch die Formel (P-111) dargestellte polymerisierbare Gruppe; 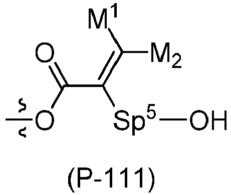
wobei in Formel (P-111) M¹ und M² unabhängig Wasserstoff, Fluor oder Methyl sind; und Sp⁵ eine Einfachbindung oder Alkylen mit 1 bis 3 Kohlenstoffen ist und in dem Alkylen mindestens ein Abschnitt -CH₂- durch -O- ersetzt sein kann.

2. Verbindung nach Anspruch 1, die durch eine der Formeln (1-7) bis (1-21) dargestellt ist.

3. Verbindung nach Anspruch 2, wobei in Formel (1-7) bis Formel (1-21) R¹, R² und R³ unabhängig Wasserstoff, Alkyl mit 1 bis 8 Kohlenstoffen, Alkenyl mit 2 bis 8 Kohlenstoffen, Alkoxy mit 1 bis 7 Kohlenstoffen oder Alkenyloxy mit 2 bis 7 Kohlenstoffen sind;
Ring A¹ ist Cyclohexyl, Cyclohexenyl oder Phenyl, und Ring A² ist 1,4-Cyclohexylen, 1,4-Cyclohexenylen oder 1,4-Phenylen, und in den Ringen kann mindestens ein Wasserstoff durch Fluor ersetzt sein;
L¹, L², L³, L⁴, L⁵, L⁷, L⁸, L¹⁰, L¹², L¹³, L¹⁵, L¹⁶, L¹⁷, L¹⁸, L¹⁹ und L²⁰ sind unabhängig Wasserstoff, Fluor, Methyl oder Ethyl;
Sp¹, Sp² und Sp³ sind unabhängig eine Einfachbindung oder Alkylen mit 1 bis 5 Kohlenstoffen und in dem Alkylen kann mindestens ein Abschnitt -CH₂- durch -O- ersetzt sein;
c, d und e sind unabhängig 0, 1 oder 2 und eine Summe aus c, d und e ist 2, 3 oder 4; und
P¹, P² und P³ sind unabhängig eine durch die Formel (P-11) dargestellte polymerisierbare Gruppe; 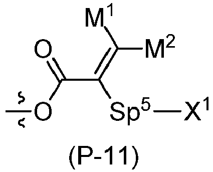 wobei in Formel (P-11) M¹ und M² unabhängig Wasserstoff, Fluor, Methyl oder Ethyl sind; Sp⁵ eine Einfachbindung oder Alkylen mit 1 bis 5 Kohlenstoffen ist und in dem Alkylen mindestens ein Abschnitt -CH₂- durch -O- ersetzt sein kann; und X¹ ist -OH oder -NH₂.

4. Verbindung nach Anspruch 1 oder 2, wobei in Formel (1-7) bis Formel (1-21) X¹ -OH ist.

5. Verbindung nach Anspruch 1, die durch eine der Formeln (1-22) bis (1-34) dargestellt ist.

6. Flüssigkristallzusammensetzung, enthaltend mindestens eine Verbindung nach einem der Ansprüche 1 bis 5 als Komponente A und mindestens eine Verbindung ausgewählt aus der Gruppe von Verbindungen, die durch Formel (2) bis Formel (7) dargestellt sind, als Komponente B: 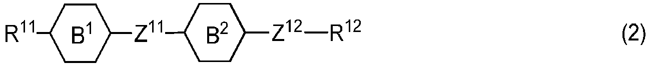 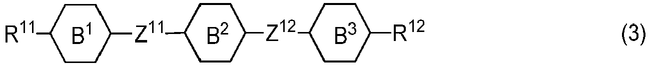 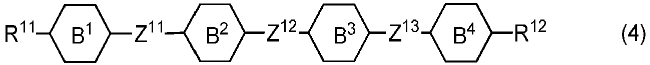 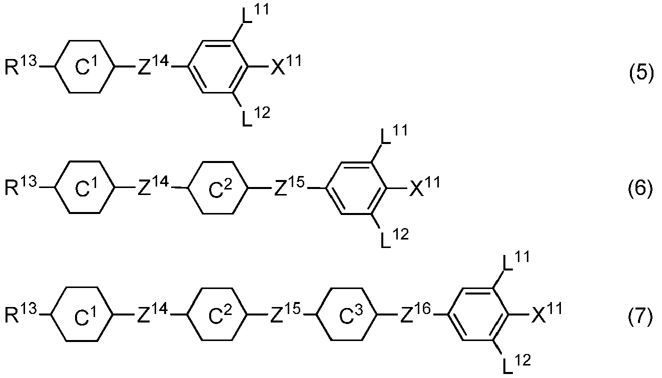
wobei in Formel (2) bis Formel (4)
R¹¹ und R¹² sind unabhängig Alkyl mit 1 bis 10 Kohlenstoffen oder Alkenyl mit 2 bis 10 Kohlenstoffen und in dem Alkyl und dem Alkenyl kann mindestens ein Abschnitt -CH₂- durch -O- ersetzt sein, und in den Gruppen kann mindestens ein Wasserstoff durch Fluor ersetzt sein;
Ring B¹, Ring B², Ring B³ und Ring B⁴ sind unabhängig 1,4-Cyclohexylen, 1,4-Phenylen, 2-Fluor-1,4-phenylen, 2,5-Difluor-1,4-phenylen oder Pyrimidin-2,5-diyl; und
Z¹¹, Z¹² und Z¹³ sind unabhängig eine Einfachbindung, -CH₂CH₂-, -CH=CH-, -C≡C- oder -COO-;
wobei in Formel (5) bis Formel (7)
R¹³ ist Alkyl mit 1 bis 10 Kohlenstoffen oder Alkenyl mit 2 bis 10 Kohlenstoffen und in dem Alkyl und dem Alkenyl kann mindestens ein Abschnitt -CH₂- durch -O- ersetzt sein, und in den Gruppen kann mindestens ein Wasserstoff durch Fluor ersetzt sein;
X¹¹ ist Fluor, Chlor, -OCF₃, -OCHF₂, -CF₃, -CHF₂, -CH₂F, -OCF₂CHF₂ oder -OCF₂CHFCF₃;
Ring C¹, Ring C² und Ring C³ sind unabhängig 1,4-Cyclohexylen, 1,4-Phenylen, 1,4-Phenylen, bei dem mindestens ein Wasserstoffatom durch Fluor ersetzt ist, Tetrahydropyran-2,5-diyl, 1,3-Dioxan-2,5-diyl oder Pyrimidin-2,5-diyl;
Z¹⁴, Z¹⁵ und Z¹⁶ sind unabhängig eine Einfachbindung, -CH₂CH₂-, -CH=CH-, -C≡C-, -COO-, -CF₂O-, -OCF₂- -CH₂O- oder - (CH₂)₄-; und
L¹¹ und L¹² sind unabhängig Wasserstoff oder Fluor.

7. Flüssigkristallzusammensetzung nach Anspruch 6, ferner enthaltend mindestens eine Verbindung ausgewählt aus der Gruppe von Verbindungen, die durch Formel (9) bis Formel (15) dargestellt sind, als Komponente E: 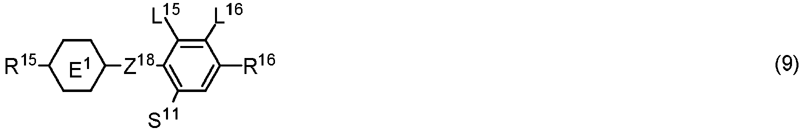 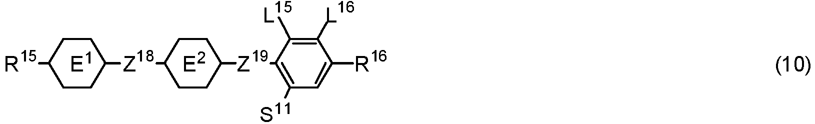 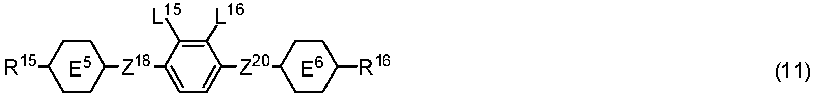 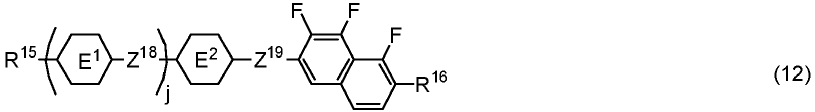 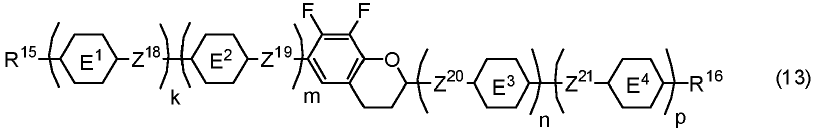 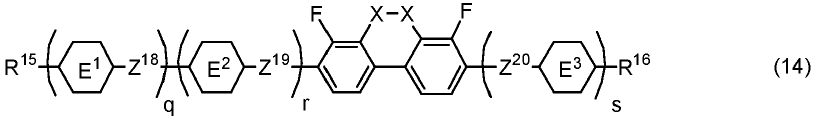 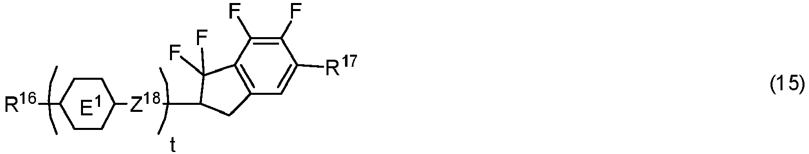 wobei in Formel (9) bis Formel (15),
R¹⁵, R¹⁶ und R¹⁷ sind unabhängig Alkyl mit 1 bis 10 Kohlenstoffen oder Alkenyl mit 2 bis 10 Kohlenstoffen und in dem Alkyl und dem Alkenyl kann mindestens ein Abschnitt -CH₂- durch -O- ersetzt sein, und in den Gruppen kann mindestens ein Wasserstoff durch Fluor ersetzt sein und R¹⁷ kann Wasserstoff oder Fluor sein;
Ring E¹, Ring E², Ring E³ und Ring E⁴ sind unabhängig 1,4-Cyclohexylen, 1,4-Cyclohexenylen, 1,4-Phenylen, 1,4-Phenylen, bei dem mindestens ein Wasserstoff durch Fluor ersetzt ist, Tetrahydropyran-2,5-diyl oder Decahydronaphthalen-2,6-diyl;
Ring E⁵ und Ring E⁶ sind unabhängig 1,4-Cyclohexylen, 1,4-Cyclohexenylen, 1,4-Phenylen, Tetrahydropyran-2,5-diyl oder Decahydronaphthalen-2,6-diyl;
Z¹⁸, Z¹⁹, Z²⁰ und Z²¹ sind unabhängig eine Einfachbindung, -CH₂CH₂-, -COO-, -CH₂O-, -OCF₂- oder -OCF₂CH₂CH₂-;
L¹⁵ und L¹⁶ sind unabhängig Fluor oder Chlor;
S¹¹ ist Wasserstoff oder Methyl;
X ist -CHF- oder -CF₂-; und
j, k, m, n, p, q, r und s sind unabhängig 0 oder 1, eine Summe aus k, m, n und p ist 1 oder 2, eine Summe aus q, r und s ist 0, 1, 2 oder 3 und t ist gleich 1, 2 oder 3.

8. Flüssigkristallzusammensetzung nach Anspruch 6 oder 7, ferner umfassend mindestens eine Verbindung ausgewählt aus der Gruppe polymerisierbarer Verbindungen, die durch Formel (16) dargestellt sind, als Komponente F: 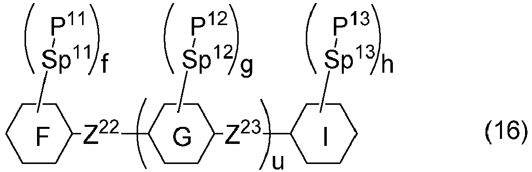 wobei in Formel (16)
Ring F und Ring I sind unabhängig Cyclohexyl, Cyclohexenyl, Phenyl, 1-Naphthyl, 2-Naphthyl, Tetrahydropyran-2-yl, 1,3-Dioxan-2-yl, Pyrimidin-2-yl oder Pyridin-2-yl, und in den Ringen kann mindestens ein Wasserstoff durch Halogen, Alkyl mit 1 bis 12 Kohlenstoffen, Alkoxy mit 1 bis 12 Kohlenstoffen oder Alkyl mit 1 bis 12 Kohlenstoffen, bei dem mindestens ein Wasserstoffatom durch Halogen ersetzt ist, ersetzt sein;
Ringe G sind 1,4-Cyclohexylen, 1,4-Cyclohexenylen, 1,4-Phenylen, Naphthalen-1,2-diyl, Naphthalen-1,3-diyl, Naphthalen-1,4-diyl, Naphthalen-1,5-diyl, Naphthalen-1,6-diyl, Naphthalen-1,7-diyl, Naphthalen-1,8-diyl, Naphthalen-2,3-diyl, Naphthalen-2,6-diyl, Naphthalen-2,7-diyl, Phenanthren-2,7-diyl, Tetrahydropyran-2,5-diyl, 1,3-Dioxan-2,5-diyl, Pyrimidin-2,5-diyl oder Pyridin-2,5-diyl, und in den Ringen kann mindestens ein Wasserstoff durch Halogen, Alkyl mit 1 bis 12 Kohlenstoffen, Alkoxy mit 1 bis 12 Kohlenstoffen oder Alkyl mit 1 bis 12 Kohlenstoffen, bei dem mindestens ein Wasserstoff durch Halogen ersetzt ist, ersetzt sein.
Z²² und Z²³ sind unabhängig eine Einfachbindung oder Alkylen mit 1 bis 10 Kohlenstoffen, und in dem Alkylen kann mindestens ein Abschnitt-CH₂- durch -O-, -CO-, -COO- oder -OCO- ersetzt sein, und mindestens ein Abschnitt -CH₂CH₂- kann durch -CH=CH-, -C(CH₃)=CH-, -CH=C(CH₃)- oder -C(CH₃)=C(CH₃)- ersetzt sein, und in den Gruppen kann mindestens ein Wasserstoff durch Fluor oder Chlor ersetzt sein;
P¹¹, P¹² und P¹³ sind unabhängig eine polymerisierbare Gruppe;
Sp¹¹, Sp¹² und Sp¹³ sind unabhängig eine Einfachbindung oder Alkylen mit 1 bis 10 Kohlenstoffen, und in dem Alkylen kann mindestens ein Abschnitt -CH₂- durch -O-, -COO-, -OCO- oder -OCOO- ersetzt sein, und mindestens ein Abschnitt -CH₂CH₂- kann durch -CH=CH- oder -C≡C- ersetzt sein, und in den Gruppen kann mindestens ein Wasserstoff durch Fluor oder Chlor ersetzt sein;
u ist 0, 1 oder 2; und
f, g und h sind unabhängig 0, 1, 2, 3 oder 4 und eine Summe aus f, g und h ist 1 oder mehr.

9. Flüssigkristallzusammensetzung nach Anspruch 8, wobei Komponente F mindestens eine Verbindung ist, die aus der Gruppe der polymerisierbaren Verbindungen, die durch Formeln (16-1) bis (16-7) dargestellt sind, ausgewählt ist: 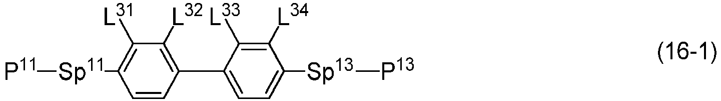 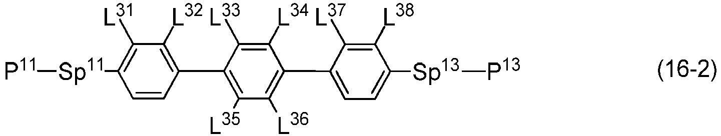 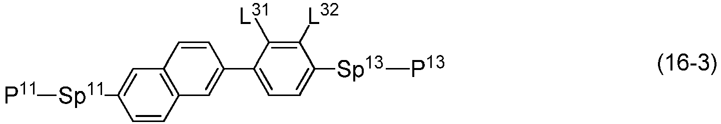 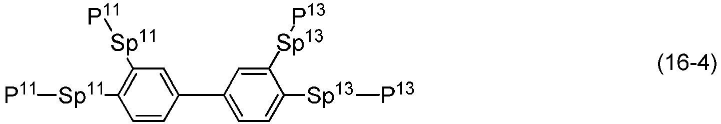 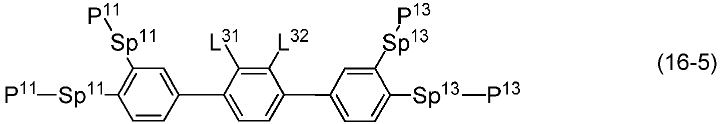 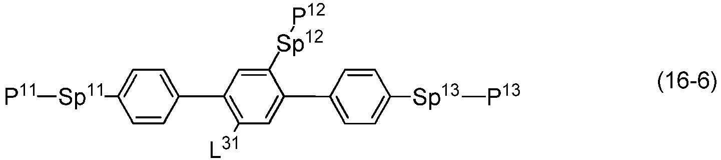 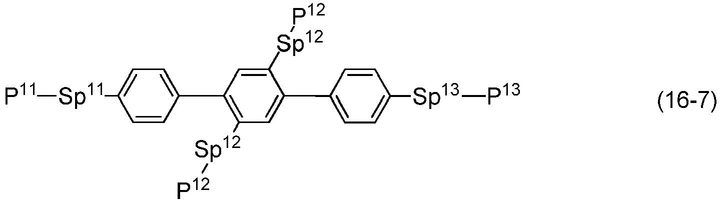 wobei in Formel (16-1) bis Formel (16-7) P¹¹, P¹² und P¹³ unabhängig eine Gruppe sind, ausgewählt aus der Gruppe polymerisierbarer Gruppen, die durch Formel (P-2) bis Formel (P-4) dargestellt sind, wobei M¹¹, M¹² und M¹³ unabhängig Wasserstoff, Fluor, Alkyl mit 1 bis 5 Kohlenstoffen oder Alkyl mit 1 bis 5 Kohlenstoffen, bei dem mindestens ein Wasserstoff durch Halogen ersetzt ist, sind; 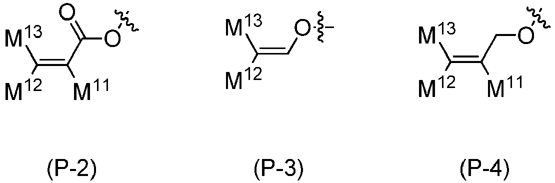 wobei L³¹, L³², L³³, L³⁴, L³⁵, L³⁶, L³⁷ und L³⁸ unabhängig Wasserstoff, Fluor oder Methyl sind; und Sp¹¹, Sp¹² und Sp¹³ sind unabhängig eine Einfachbindung oder Alkylen mit 1 bis 10 Kohlenstoffen, und in dem Alkylen kann mindestens ein Abschnitt -CH₂- durch -O-, -COO-, -OCO- oder -OCOOersetzt sein, und mindestens ein Abschnitt -CH₂CH₂- kann durch -CH=CH- oder -C≡C- ersetzt sein, und in den Gruppen kann mindestens ein Wasserstoff durch Fluor oder Chlor ersetzt sein.

10. Flüssigkristallzusammensetzung nach einem der Ansprüche 6 bis 9, ferner enthaltend mindestens eine von den durch Formel (1) und Formel (16) dargestellten Verbindungen verschiedene polymerisierbare Verbindung, einen Polymerisationsinitiator, einen Polymerisationsinhibitor, eine optisch aktive Verbindung, ein Antioxidans, ein UV-Absorber, ein Lichtstabilisator, ein Wärmestabilisator und ein Antischaummittel.

11. Flüssigkristallanzeigevorrichtung, umfassend mindestens eine Flüssigkristallzusammensetzung nach einem der Ansprüche 6 bis 10.

## Revendications

1. Composé, représenté par l'une quelconque des formules (1-7) à (1-34) : 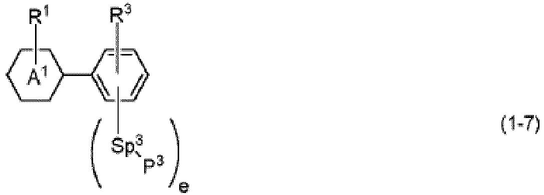 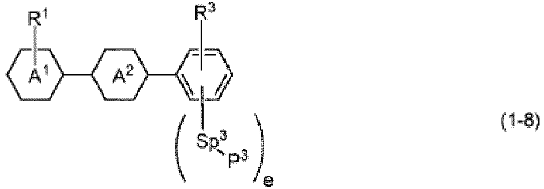 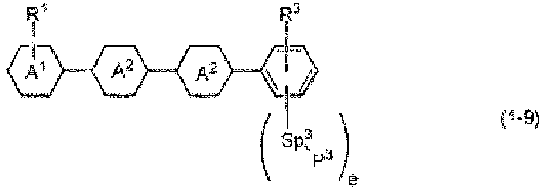 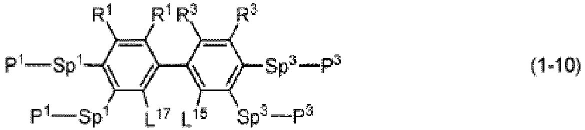 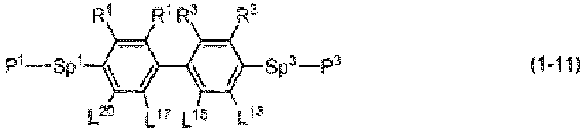 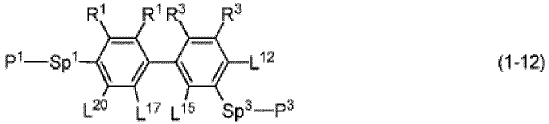 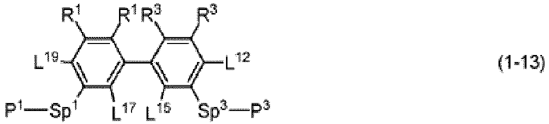 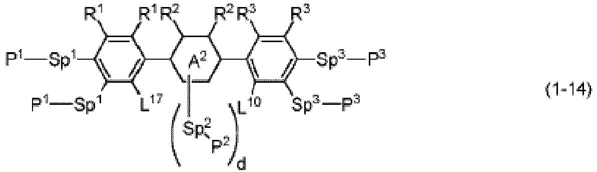 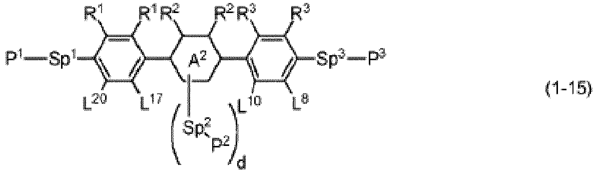 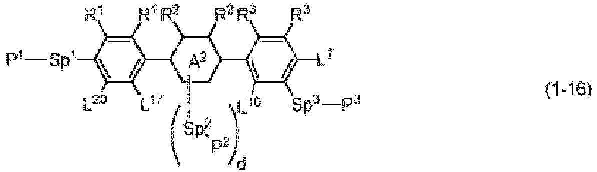 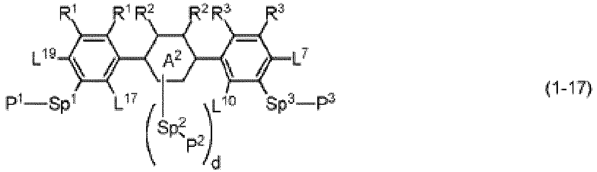 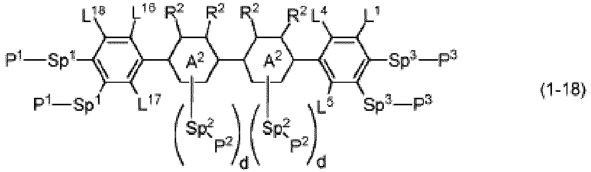 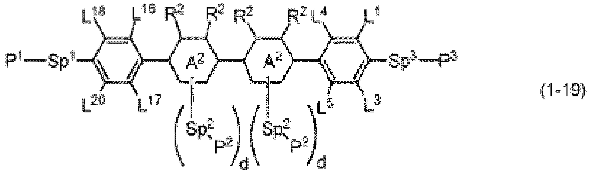 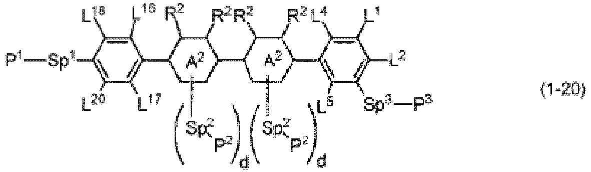 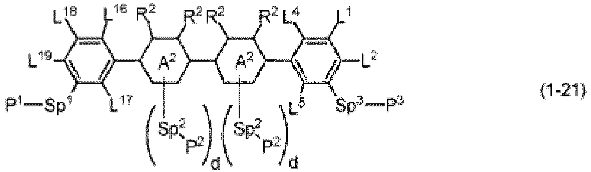
dans lesquelles, dans les formules (1-7) à (1-21),
R¹, R² et R³ sont indépendamment un hydrogène, un alkyle ayant 1 à 8 carbones, un alcényle ayant 2 à 8 carbones, un alcoxy ayant 1 à 7 carbones ou un alcényloxy ayant 2 à 7 carbones ;
le cycle A¹ est un cyclohexyle, un cyclohexènyle ou un phényle, et le cycle A² est un 1,4-cyclohexylène, un 1,4-cyclohexènylène ou un 1,4-phénylène, et dans les cycles, au moins un hydrogène peut être remplacé par un fluor ;
L¹, L², L³, L⁴, L⁵, L⁷, L⁸, L¹⁰, L¹², L¹³, L¹⁵, L¹⁶, L¹⁷, L¹⁸, L¹⁹ et L²⁰ sont indépendamment un hydrogène, un fluor, un méthyle ou un éthyle ;
Sp¹, Sp² et Sp³ sont indépendamment une liaison simple ou un alkylène ayant 1 à 5 carbones, et dans l'alkylène, au moins un fragment de -CH₂- peut être remplacé par -O- ;
c, d et e sont indépendamment 0, 1 ou 2, et une somme de c, d et e est 2, 3 ou 4 ; et
P¹, P² et P³ sont indépendamment un groupe polymérisable représenté par la formule (P-11) ; 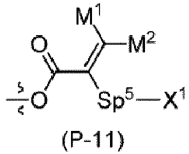
dans laquelle, dans la formule (P-11), M¹ et M² sont indépendamment un hydrogène, un fluor, un méthyle, un éthyle ou un trifluorométhyle ; Sp⁵ est une liaison simple ou un alkylène ayant 1 à 5 carbones, et dans l'alkylène, au moins un fragment de -CH₂- peut être remplacé par -O- ; et X¹ est -OH ou -NH₂ ; 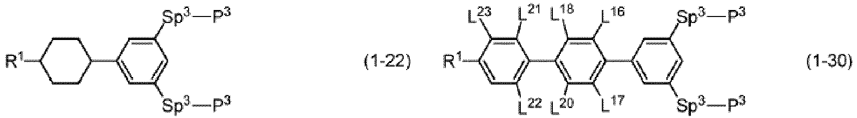 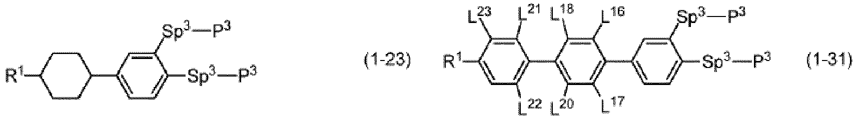 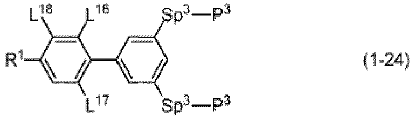 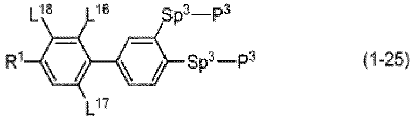 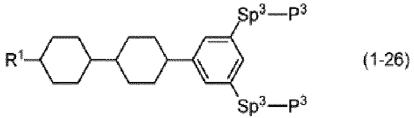 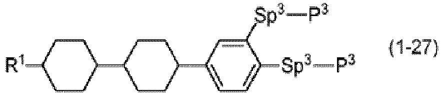 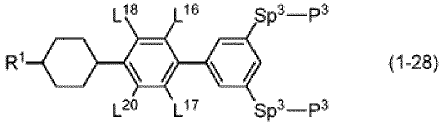 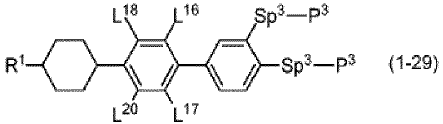 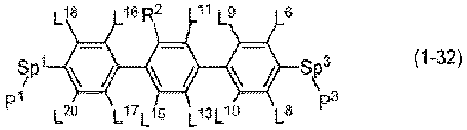 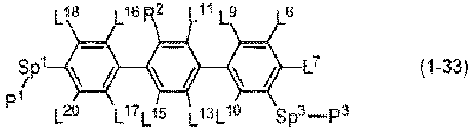 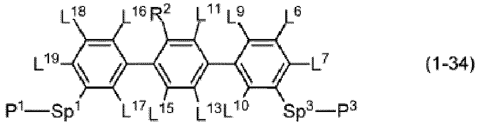
dans lesquelles, dans les formules (1-22) à (1-34),
R¹ et R² sont indépendamment un alkyle ayant 1 à 7 carbones, un alcényle ayant 2 à 7 carbones, un alcoxy ayant 1 à 6 carbones ou un alcényloxy ayant 2 à 6 carbones ;
L⁶, L⁷, L⁸, L⁹, L¹⁰, L¹¹, L¹³, L¹⁵, L¹⁶, L¹⁷, L¹⁸, L¹⁹, L²⁰, L²¹, L²² et L²³ sont indépendamment un hydrogène, un fluor, un méthyle ou un éthyle ;
Sp¹ et Sp³ sont indépendamment une liaison simple ou un alkylène ayant 1 à 3 carbones, et dans l'alkylène, au moins un fragment de -CH₂- peut être remplacé par -O- ; et
P¹ et P³ sont indépendamment un groupe polymérisable représenté par la formule (P-111) ; 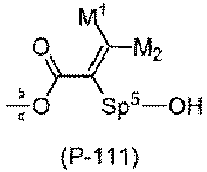
dans laquelle, dans la formule (P-111), M¹ et M² sont indépendamment un hydrogène, un fluor ou un méthyle ; et Sp⁵ est une liaison simple ou un alkylène ayant 1 à 3 carbones, et dans l'alkylène, au moins un fragment de -CH₂- peut être remplacé par -O-.

2. Composé selon la revendication 1, qui est représenté par l'une quelconque des formules (1-7) à (1-21).

3. Composé selon la revendication 2, dans lequel, dans les formules (1-7) à (1-21), R¹, R² et R³ sont indépendamment un hydrogène, un alkyle ayant 1 à 8 carbones, un alcényle ayant 2 à 8 carbones, un alcoxy ayant 1 à 7 carbones ou un alcényloxy ayant 2 à 7 carbones ;
le cycle A¹ est un cyclohexyle, un cyclohexènyle ou un phényle, et le cycle A² est un 1,4-cyclohexylène, un 1,4-cyclohexènylène ou un 1,4-phénylène, et dans les cycles, au moins un hydrogène peut être remplacé par un fluor ;
L¹, L², L³, L⁴, L⁵, L⁷, L⁸, L¹⁰, L¹², L¹³, L¹⁵, L¹⁶, L¹⁷, L¹⁸, L¹⁹ et L²⁰ sont indépendamment un hydrogène, un fluor, un méthyle ou un éthyle ;
Sp¹, Sp² et Sp³ sont indépendamment une liaison simple ou un alkylène ayant 1 à 5 carbones, et dans l'alkylène, au moins un fragment de -CH₂- peut être remplacé par -O- ;
c, d et e sont indépendamment 0, 1 ou 2, et une somme de c, d et e est 2, 3 ou 4 ; et
P¹, P² et P³ sont indépendamment un groupe polymérisable représenté par la formule (P-11) ; 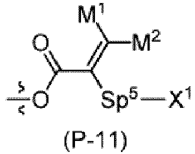 dans laquelle, dans la formule (P-11), M¹ et M² sont indépendamment un hydrogène, un fluor, un méthyle ou un éthyle ; Sp⁵ est une liaison simple ou un alkylène ayant 1 à 5 carbones, et dans l'alkylène, au moins un fragment de -CH₂- peut être remplacé par -O- ; et X¹ est -OH ou -NH₂.

4. Composé selon la revendication 1 ou 2, dans lequel, dans les formules (1-7) à (1-21), X¹ est -OH.

5. Composé selon la revendication 1, qui est représenté par l'une quelconque des formules (1-22) à (1-34).

6. Composition de cristaux liquides, contenant au moins un composé selon l'une quelconque des revendications 1 à 5 en tant que composant A et au moins un composé sélectionné dans le groupe de composés représentés par les formules (2) à (7) en tant que composant B : 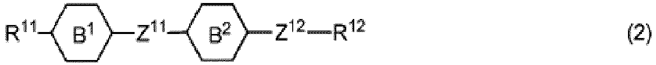 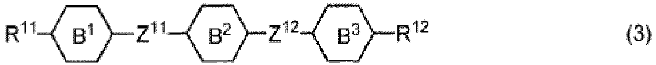 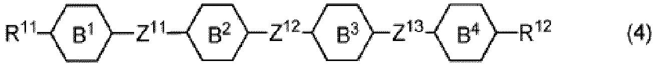 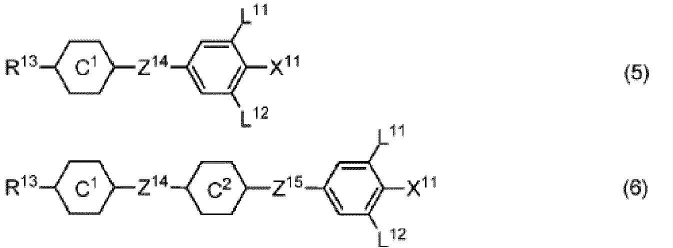 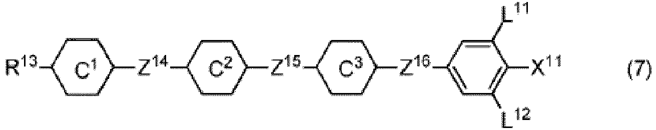
dans lesquelles, dans les formules (2) à (4),
R¹¹ et R¹² sont indépendamment un alkyle ayant 1 à 10 carbones ou un alcényle ayant 2 à 10 carbones, et dans l'alkyle et l'alcényle, au moins un fragment de -CH₂-peut être remplacé par -O-, et dans les groupes, au moins un hydrogène peut être remplacé par un fluor ;
le cycle B¹, le cycle B², le cycle B³ et le cycle B⁴ sont indépendamment un 1,4-cyclohexylène, un 1,4-phénylène, un 2-fluoro-1,4-phénylène, un 2,5-difluoro-1,4-phénylène ou un pyrimidine-2,5-diyle ; et
Z¹¹, Z¹² et Z¹³ sont indépendamment une liaison simple, -CH₂CH₂-, -CH=CH-, C≡C- ou -COO-.
dans lesquelles, dans les formules (5) à (7),
R¹³ est un alkyle ayant 1 à 10 carbones ou un alcényle ayant 2 à 10 carbones, et dans l'alkyle et l'alcényle, au moins un fragment de -CH₂- peut être remplacé par -O-, et dans les groupes, au moins un hydrogène peut être remplacé par un fluor ;
X¹¹ est un fluor, un chlore, -OCF₃, -OCHF₂, -CF₃, -CHF₂, -CH₂F, -OCF₂CHF₂ ou -OCF₂CHFCF₃ ;
le cycle C¹, le cycle C² et le cycle C³ sont indépendamment un 1,4-cyclohexylène, un 1,4-phénylène, un 1,4-phénylène dans lequel au moins un hydrogène est remplacé par un fluor, un tétrahydropyrane-2,5-diyle, un 1,3-dioxane-2,5-diyle ou un pyrimidine-2,5-diyle ;
Z¹⁴, Z¹⁵ et Z¹⁶ sont indépendamment une liaison simple, -CH₂CH₂-, -CH=CH-, C≡C-, -COO-, -CF₂O-, -OCF₂- -CH₂O- ou -(CH₂)₄- ; et
L¹¹ et L¹² sont indépendamment un hydrogène ou un fluor.

7. Composition de cristaux liquides selon la revendication 6, contenant en outre au moins un composé sélectionné dans le groupe de composés représentés par les formules (9) à (15) en tant que composant E : 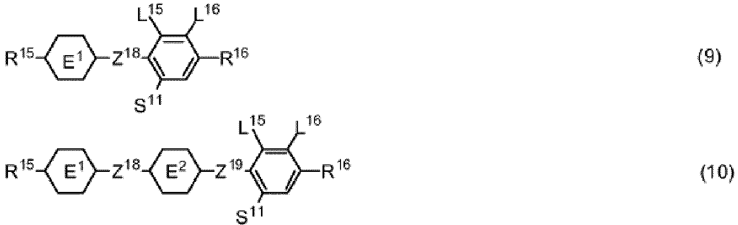 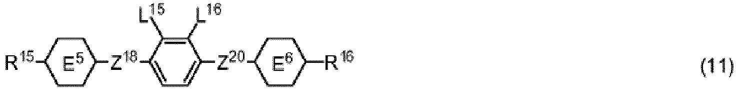 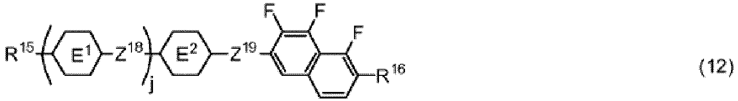 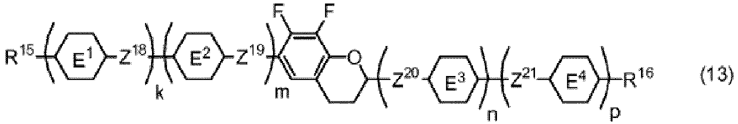 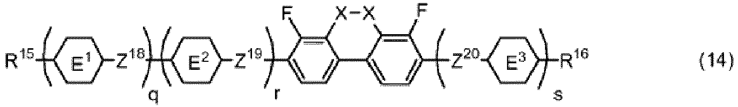 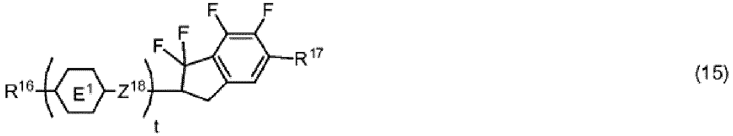 dans lesquelles, dans les formules (9) à (15),
R¹⁵, R¹⁶ et R¹⁷ sont indépendamment un alkyle ayant 1 à 10 carbones ou un alcényle ayant 2 à 10 carbones, et dans l'alkyle et l'alcényle, au moins un fragment de -CH₂-peut être remplacé par -O-, et dans les groupes, au moins un hydrogène peut être remplacé par un fluor, et R¹⁷ peut être un hydrogène ou un fluor ;
le cycle E¹, le cycle E², le cycle E³ et le cycle E⁴ sont indépendamment un 1,4-cyclohexylène, un 1,4-cyclohexènylène, un 1,4-phénylène, un 1,4-phénylène dans lequel au moins un hydrogène est remplacé par un fluor, un tétrahydropyrane-2,5-diyle ou un décahydronaphtalène-2,6-diyle ;
le cycle E⁵ et le cycle E⁶ sont indépendamment un 1,4-cyclohexylène, un 1,4-cyclohexènylène, un 1,4-phénylène, un tétrahydropyrane-2,5-diyle ou un décahydronaphtalène-2,6-diyle ;
Z¹⁸, Z¹⁹, Z²⁰ et Z²¹ sont indépendamment une liaison simple, -CH₂CH₂-, -COO-, - CH₂O-, -OCF₂- ou -OCF₂CH₂CH₂- ;
L¹⁵ et L¹⁶ sont indépendamment un fluor ou un chlore ;
S¹¹ est un hydrogène ou un méthyle ;
X est -CHF- ou -CF₂- ; et
j, k, m, n, p, q, r et s sont indépendamment 0 ou 1, une somme de k, m, n et p est 1 ou 2, une somme de q, r et s est 0, 1, 2 ou 3, et t est 1, 2 ou 3.

8. Composition de cristaux liquides selon la revendication 6 ou 7, contenant en outre au moins un composé sélectionné dans le groupe des composés polymérisables représentés par la formule (16) en tant que composant F : 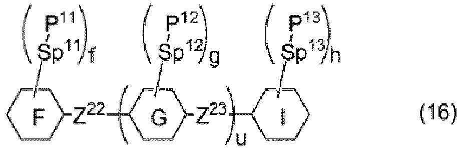 dans laquelle, dans la formule (16),
le cycle F et le cycle I sont indépendamment un cyclohexyle, un cyclohexènyle, un phényle, un 1-naphtyle, un 2-naphtyle, un tétrahydropyran-2-yle, un 1,3-dioxan-2-yle, un pyrimidin-2-yle ou un pyridin-2-yle, et dans les cycles, au moins un hydrogène peut être remplacé par un halogène, un alkyle ayant 1 à 12 carbones, un alcoxy ayant 1 à 12 carbones ou un alkyle ayant 1 à 12 carbones dans lequel au moins un hydrogène est remplacé par un halogène ;
les cycles G sont un 1,4-cyclohexylène, un 1,4-cyclohexènylène, un 1,4-phénylène, un naphtalène-1,2-diyle, un naphtalène-1,3-diyle, un naphtalène-1,4-diyle, un naphtalène-1,5-diyle, un naphtalène-1,6-diyle, un naphtalène-1,7-diyle, un naphtalène-1,8-diyle, un naphtalène-2,3-diyle, un naphtalène-2,6-diyle, un naphtalène-2,7-diyle, un phénanthrène-2,7-diyle, un tétrahydropyrane-2,5-diyle, un 1,3-dioxane-2,5-diyle, un pyrimidine-2,5-diyle ou un pyridine-2,5-diyle, et dans les cycles, au moins un atome d'hydrogène peut être remplacé par un halogène, un alkyle ayant 1 à 12 carbones, un alcoxy ayant 1 à 12 carbones ou un alkyle ayant 1 à 12 carbones dans lequel au moins un hydrogène est remplacé par un halogène ;
Z²² et Z²³ sont indépendamment une liaison simple ou un alkylène ayant 1 à 10 carbones, et dans l'alkylène, au moins un fragment de -CH₂- peut être remplacé par -O-, -CO-, -COO- ou -OCO-, et au moins un fragment de -CH₂CH₂- peut être remplacé par -CH=CH-, -C(CH₃)=CH-, -CH=C(CH₃)- ou -C(CH₃)=C(CH₃)-, et dans les groupes, au moins un hydrogène peut être remplacé par un fluor ou un chlore ;
P¹¹, P¹² et P¹³ sont indépendamment un groupe polymérisable ;
Sp¹¹, Sp¹² et Sp¹³ sont indépendamment une liaison simple ou un alkylène ayant 1 à 10 carbones, et dans l'alkylène, au moins un fragment de -CH₂- peut être remplacé par -O-, -COO-, -OCO- ou -OCOO-, et au moins un fragment de -CH₂CH₂- peut être remplacé par -CH=CH- ou -C≡C-, et dans les groupes, au moins un hydrogène peut être remplacé par un fluor ou un chlore ;
u est 0, 1 ou 2 ; et
f, g et h sont indépendamment 0, 1, 2, 3 ou 4, et une somme de f, g et h est 1 ou plus.

9. Composition de cristaux liquides selon la revendication 8, dans laquelle le composant F est au moins un composé sélectionné dans le groupe des composés polymérisables représentés par les formules (16-1) à (16-7) : 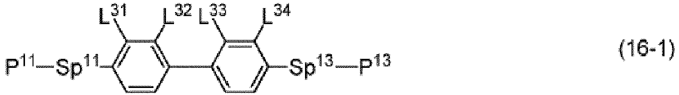 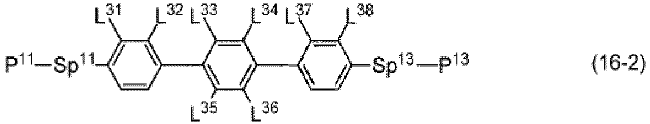 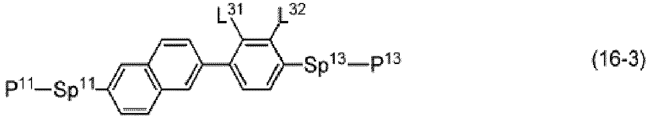 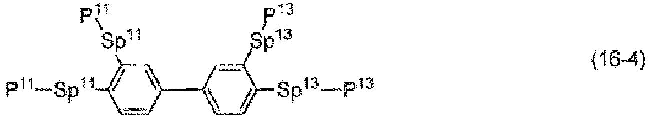 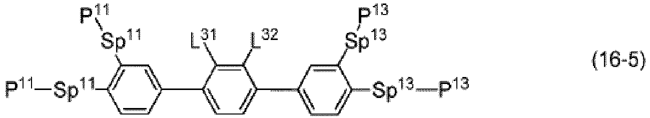 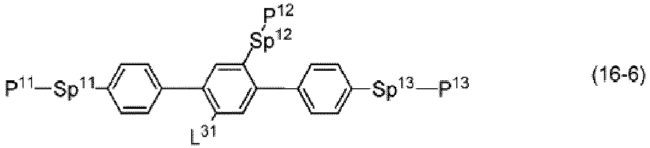 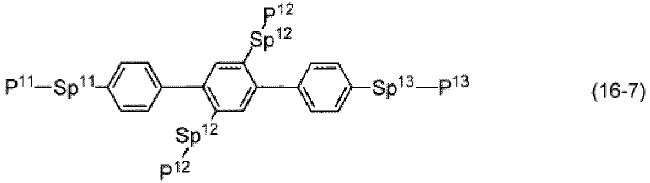 dans lesquelles, dans les formules (16-1) à (16-7), P¹¹, P¹² et P¹³ sont indépendamment un groupe sélectionné dans le groupe des groupes polymérisables représentés par les formules (P-2) à (P-4), dans lesquelles M¹¹, M¹² et M¹³ sont indépendamment un hydrogène, un fluor, un alkyle ayant 1 à 5 carbones ou un alkyle ayant 1 à 5 carbones dans lequel au moins un hydrogène est remplacé par un halogène ; 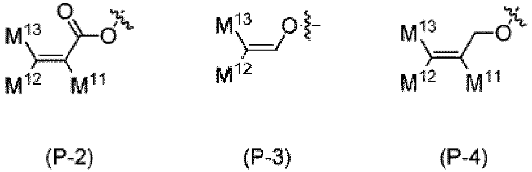 dans lesquelles, L³¹, L³², L³³, L³⁴, L³⁵, L³⁶, L³⁷ et L³⁸ sont indépendamment un hydrogène, un fluor ou un méthyle ; et Sp¹¹, Sp¹² et Sp¹³ sont indépendamment une liaison simple ou un alkylène ayant 1 à 10 carbones, et dans l'alkylène, au moins un fragment de -CH₂- peut être remplacé par -O-, -COO-, -OCO- ou -OCOO-, et au moins un fragment de -CH₂CH₂-peut être remplacé par -CH=CH- ou -C≡C-, et dans les groupes, au moins un hydrogène peut être remplacé par un fluor ou un chlore.

10. Composition de cristaux liquides selon l'une quelconque des revendications 6 à 9, contenant en outre au moins l'un d'un composé polymérisable différent des composés représentés par la formule (1) et la formule (16), un initiateur de polymérisation, un inhibiteur de polymérisation, un composé optiquement actif, un antioxydant, un absorbeur de lumière ultraviolette, un photostabilisant, un thermostabilisant et un agent antimousse.

11. Dispositif d'affichage à cristaux liquides, comprenant au moins une composition de cristaux liquides selon l'une quelconque des revendications 6 à 10.
